# EUROPEAN PATENT SPECIFICATION

(11) **EP 2 872 636 B1**
(45) Date of publication and mention of the grant of the patent: **11.06.2025**
(21) Application number: 13816444.7
(22) Date of filing: 15.07.2013
(51) Int. Cl.: C12N 5/00, C12N 5/02, C12N 5/0735, C12N 5/074, A61K 38/45, A61K 35/545, C12N 5/073

(54) **METHOD FOR INDUCING CELLS TO LESS MATURE STATE**
VERFAHREN ZUR INDUZIERUNG EINES MINDEREN ZELLREIFEZUSTANDS
PROCÉDÉ D'INDUCTION DE CELLULES À UN ÉTAT MOINS MATURE

(30) Priority: 13.07.2012 US 201261671588 P; 13.07.2012 US 201261671558 P; 19.07.2012 US 201261673617 P; 24.07.2012 US 201261675264 P; 24.07.2012 US 201261675292 P; 30.07.2012 US 201261677442 P; 02.08.2012 US 201261679021 P; 14.08.2012 US 201261683155 P; 17.08.2012 US 201261684654 P; 27.08.2012 US 201261693712 P; 17.10.2012 WO PCT/US2012/060684
(43) Date of publication of application: 20.05.2015
(73) Proprietor: Minerva Biotechnologies Corporation, Waltham, MA 02451 (US)
(72) Inventor: BAMDAD, Cynthia, Waltham, MA 02451 (US)
(74) Representative: Mewburn Ellis LLP
(86) International application number: PCT/US2013/050563
(87) International publication number: WO 2014/012115

(56) References cited:
- WO-A2-2008/070171
- WO-A2-2010/042891
- US-A1- 2009 075 926
- US-A1- 2010 055 678
- US-A1- 2010 093 092
- US-A1- 2010 316 688
- US-A1- 2012 156 246
- HIKITA SHERRY T ET AL: "MUC1* mediates the growth of human pluripotent stem cells", PLOS ONE, PUBLIC LIBRARY OF SCIENCE, US, vol. 3, no. 10, 3 October 2008 (2008-10-03), pages E3312, XP002560322, ISSN: 1932-6203, DOI: 10.1371/JOURNAL.PONE.0003312
- BRUGGER ET AL.: "Expression of MUC-1 Epitopes on Normal Bone Marrow: Implications for the Detection of Micrometastatic Tumor Cells.", J CLIN ONCOL., vol. 17, no. 5, May 1999 (1999-05-01), pages 1535 - 44, XP002466551

## Description

### BACKGROUND OF THE INVENTION

### 1. Field of the Invention:

The present application relates to the field of inducing pluripotency in cells.

### 2. General Background and State of the Art:

It has been demonstrated, in mouse and human, that somatic cells can be reprogrammed by ectopic expression of transcription factors (Lowry et al., 2008; Maherali et al., 2007; Nakagawa et al., 2008; Okita et al., 2007; Park et al., 2008; Takahashi et al., 2006; Takahashi and Yamanaka, 2006; Wernig et al., 2007; Yu et al., 2006) to become pluripotent. The generation of induced pluripotent stem (iPS) cells holds great promise for the realization of truly personalized regenerative medicine (Yamanaka, 2007; Jaenish and Young, 2008) because stem cells derived from a patient's own skin cell can be used to generate cells and tissues to repair damage caused by disease or aging. Forced expression of combinations of the transcription factors, Oct4, Sox2, Klf4 and c-Myc or Oct4, Sox2, Nanog and Lin28 have been shown to cause mature cells to revert to the pluripotent state.

In earlier studies, the transcription factors were expressed using multiple viral vectors (Takahashi and Yamanaka, 2006; Okita et al., 2007; Maherali et al., 2007; Wernig et al., 2007; Takahashi et al., 2006;Yu et al., 2006; Park et al., 2008). The use of multiple vectors presented a problem because of multiple integration events, which could lead to increased risk of oncogenicity (Takahashi and Yamanaka, 2006; Aoi et al., 2008). Researchers have tried to overcome this problem by using single vector systems (Sommer et al., 2009), excisable vectors (Kaji et al., 2009; Soldner et al., 2009; Woltjen et al., 2009), non-integrating vectors (Stadtfeld et al., 2009; Yu et al., 2009) and transient transfections (Okita et al., 2009). However, these methods are extremely inefficient at achieving epigenetic reprogramming.

Methods for inducing pluripotency include transfection of the oncogene c-Myc, which is undesirable because of its potential to cause cancer. iPS cells can be generated without transfecting c-Myc (Nakagawa et al., 2008; Wernig et al., 2008). However, the efficiency of reprogramming was greatly decreased. Similarly, Klf4 can induce dysplasia (Foster et al., 2005). A method for inducing pluripotency is described, for example, in WO 2010/042891, a method of isolating or selecting stem cells is described, for example, in WO 2008/070171, and a method of culturing, expanding or growing stem cells is described, for example, in US 2010/316688.

Because of the problems associated with multiple viral vector integration and undesirable side effects of some of the genes that induce pluripotency, there is a need to replace the use of some or all of the pluripotency-inducing genes with the protein gene products and proteins that regulate their expression or whose expression is regulated by the pluripotency-inducing genes or small molecules that regulate the expression of genes or proteins that induce pluripotency. To this end, it has been reported that the introduction of the gene products, rather than the genes, also induced pluripotency (Zhou et al., 2009). Recombinant Oct4, Sox2, Klf4 and c-Myc, tagged with poly-arginine to facilitate entry into the cell, reprogrammed mouse somatic cells. Others have used small molecules to replace the need for one of the genes of the core set. A small molecule that upregulated Nanog eliminated the need for the Klf4 gene, which also upregulates Nanog (Lyssiotis et al., 2009). In another study, a small molecule HDAC inhibitor removed the requirement for both Klf4 and c-Myc (Huangfu et al., 2008, a&b). These studies show that: 1) the protein gene products can replace the need for the genes; 2) small molecules that upregulate genes can replace the need for the genes; and 3) genes (or gene products) in the same regulatory pathway can substitute for one another.

Despite these achievements, a major problem that remains is that these methods suffer from low efficiency of reprogramming. Current rates of inducing pluripotency in somatic cells are so low that they make therapeutic uses of iPS cells impractical. Therefore, what is needed is to identify proteins and small molecules that either alone or in addition to those already identified, induce pluripotency or improve the efficiency of the induction of pluripotency in cells.

### SUMMARY OF THE INVENTION

The invention is defined in the claims Generally, the disclosed method is for generating less mature cells from starting cells comprising inducing the starting cells to revert to a less mature state, comprising contacting the starting cells with an NME1 dimer or NME7 protein gene encoding the NME7 protein.

The method may further include transfecting the starting cell with a nucleic acid that directly or indirectly causes an increase in the amount, expression or activity of Oct4, Sox2, Klf4, c-Myc, Lin28, or Nanog.

The method may further include contacting the starting cells with the peptide or protein that directly or indirectly causes an increase in the amount, expression or activity of Oct4, Sox2, Klf4, c-Myc, Lin28, or Nanog.

In the method the contacting the starting cells as described above directly or indirectly causes an increase in the amount, expression or activity of Oct4, Sox2, Klf4, c-Myc, Lin28, or Nanog.

In the methods described above, the method may further include contacting the starting cell with a molecule that increases expression of gene products that induce pluripotency. Such gene product may include OCT4, SOX2, NANOG, KLF4 or LIN28. In particular, OCT4, and SOX2.

The cells in the invention may be mammalian, including human.

The starting cells may be pluripotent stem cells, multipotent stem cells or terminally differentiated cells. The pluripotent stem cell may be in the primed state. The multipotent stem cell may be a hematopoietic cell, a bone marrow cell or a neuronal cell. The terminally differentiated cell may be a fibroblast, a dermablast, a blood cell, or a neuronal cell.

The generated cells may be then differentiated, *in vitro* or *in vivo.*

As described herein, the method of generating a less mature cell, including induced pluripotent cell comprises contacting starting cells with NME in the absence of bFGF.

These and other objects of the invention will be more fully understood from the following description of the invention the referenced drawings attached hereto and the claims appended hereto.

### BRIEF DESCRIPTION OF THE DRAWINGS

The present disclosure will become more fully understood from the detailed description given herein below, and the accompanying drawings which are given by way of illustration only, and thus are not limitative of the present disclosure, and wherein;

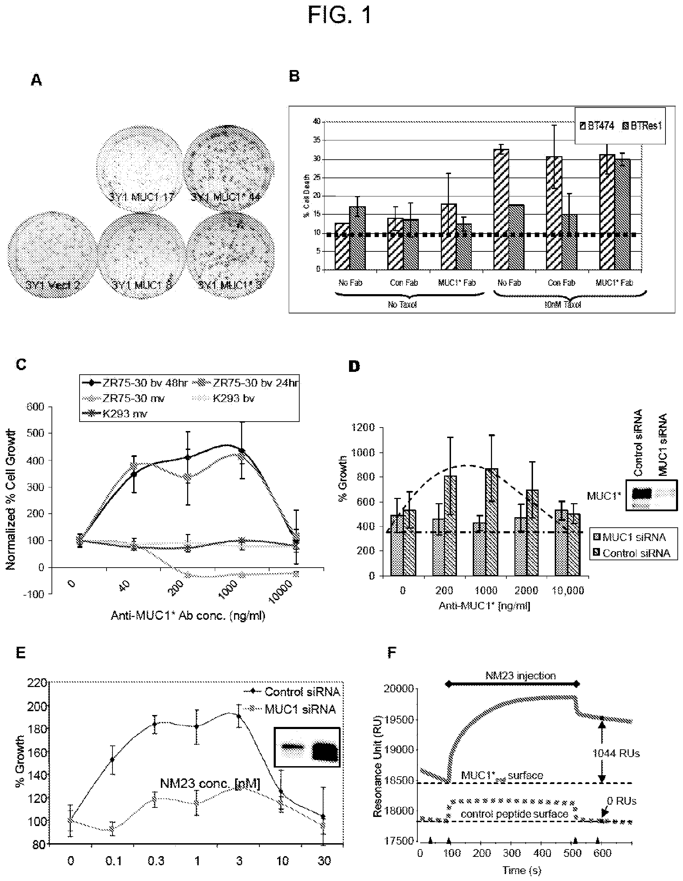

FIGURES 1A-1F show that MUC1* increases growth rate. A. Clonogenic assay shows that transfecting rat fibroblasts (3Y1) with MUC1* increases growth rate but MUC1 (full-length) does not; B. MUC1* activity increases survival. Breast cancer cells that have acquired resistance to TAXOL^{®} do so by increasing MUC1* expression. Treatment with anti-MUC1* Fab reverses acquired resistance to TAXOL^{®}-induced cell death; C. Ligand-induced dimerization of MUC1* extracellular domain stimulates growth. The addition of bivalent anti-MUC1* antibodies stimulates the growth of MUC1*-positive cells. Blocking with the anti-MUC1* (mv) Fab inhibits cell growth. Bell-shaped growth curve is characteristic of receptor dimerization. Growth of control MUC1-negative HEK 293 cells was not affected; D. Suppression of MUC1*, using specific siRNA, abolishes the growth stimulatory effects of adding a MUC1* dimerizing ligand; E. NM23 is the native MUC1* activating ligand. NM23 stimulates growth of MUC1*-positive cancer cells and produces bell-shaped curve indicative of receptor dimerizatio. Effect is abolished by siRNA suppression of MUC1; F. Direct binding of NM23 to the MUC1* peptide is detected by SPR. 15nM NM23 binds to MUC1* extracellular domain peptide but not to irrelevant peptide. Measurements were done using SPR (surface plasmon resonance) and NTA-Ni-SAM coated Au chips.

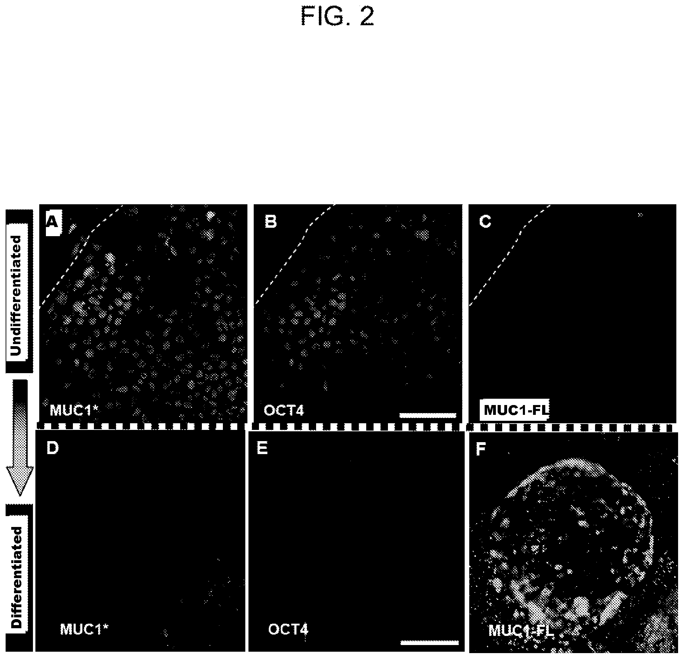

FIGURES 2A-2F show that MUC1 is cleaved on undifferentiated hESCs but MUC1 is not cleaved on differentiated hESCs. Immunocytochemistry shows that undifferentiated (pluripotent) stem cells express MUC1* and not the full-length protein; OCT4 is the gold standard marker for pluripotency. All pluripotent stem cells are MUC1*-positive. However, as soon as differentiation initiates (loss of OCT4 expression), cleavage stops and only full-length MUC1 (MUC1-FL) is detected. Panels A-C are photos of the same undifferentiated stem cell colony stained with: A. anti-MUC1* antibody that recognizes the PSMGFR peptide; B. anti-OCT4; C. anti-MUC1 full-length VU4HS. Panels D-F are photos of the same newly differentiated stem cell colony stained with: D. anti-MUC1* antibody that recognizes the PSMGFR peptide; E. anti-OCT4; F. anti-MUC1 full-length VU4H5.

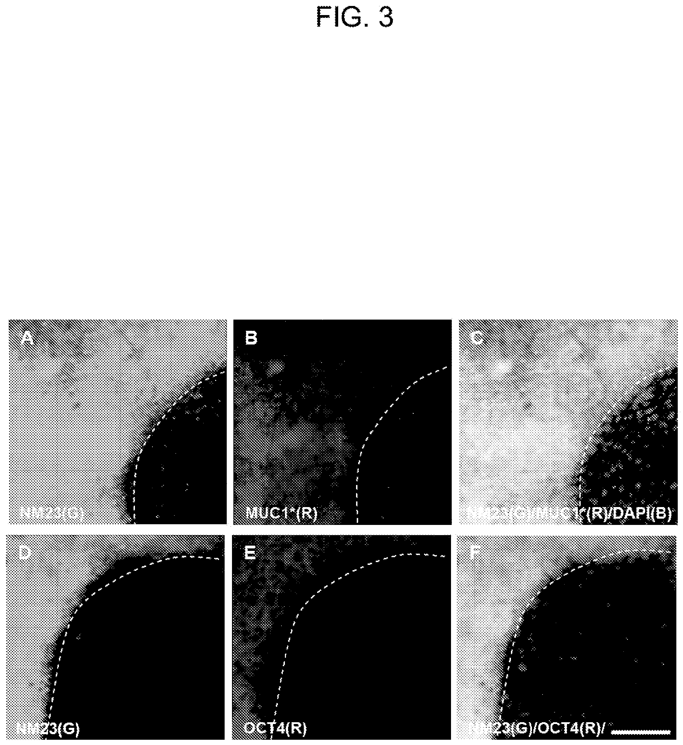

FIGURES 3A-3F show that NM23 (MUC1* ligand) co-localizes with MUC1* and OCT4 on undifferentiated hESCs, but not on differentiated cells. Immunocytochemistry shows that undifferentiated (pluripotent) stem cells express MUC1* and its activating ligand NM23. However, when stem cells begin to differentiate (loss of OCT4 expression), then MUC1 is expressed as the full-length protein and NM23 is no longer secreted. Dotted lines indicate the border between the undifferentiated and the newly differentiating portions. Panels A-C are photos of the same undifferentiated stem cell colony stained with: A. an antibody that recognizes NM23; B. anti-MUC1* antibody that recognizes the PSMGFR peptide; C. an overlay of (A), (B) and the same cells stained with DAPI to stain nuclei. Panels D-F are photos of the same undifferentiated stem cell colony stained with: D. an antibody that recognizes NM23; E. an antibody that recognizes OCT4; F. an overlay of (D), (E) and the same cells stained with DAPI to stain nuclei.

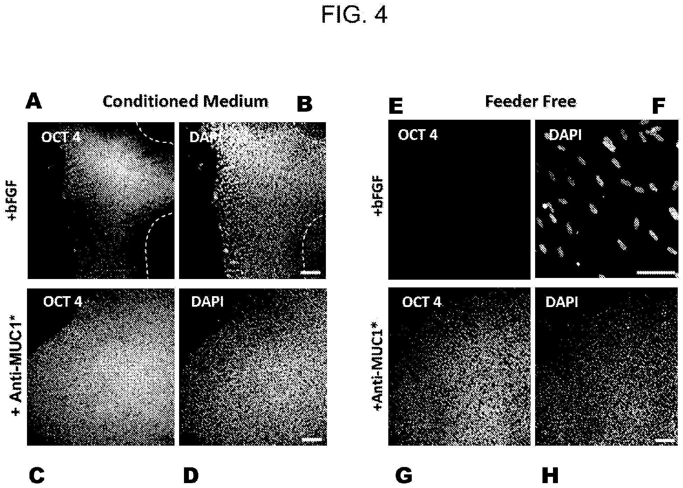

FIGURES 4A-4H show that stimulation of MUC1* via ligand-induced dimerization promotes growth and inhibits differentiation of hESCs in the absence of bFGF and conditioned media. Ligand-induced dimerization of MUC1* extracellular domain produced, using bivalent anti-MUC1* antibody, essentially 100% pluripotent colonies after 5 weeks growth in minimal media without adding bFGF or conditioned media. Colonies were grown on matrigel. The same results were obtained when NM23 or NM23 S120G mutant was used to activate MUC1*. Panels A-D are photos of wells where cell growth medium was supplemented with conditioned medium from fibroblast feeder cells plus either anti-MUC1* or bFGF. Panels E-H are photos of wells where stem cells were cultured in minimal medium plus either anti-MUC1* or bFGF. Images are of cells stained with: A. antibody that recognizes OCT4; B. DAPI staining of cells of (A); C. antibody that recognizes OCT4; D. DAPI staining of cells of (C); E. antibody that recognizes OCT4; F. DAPI staining of cells of (E); G. antibody that recognizes OCT4; H. DAPI staining of cells of (G).
FIGURE 5 shows a bar graph indicating that MUC1* activity is required for pluripotent stem cell growth. Blocking MUC1* with anti-MUC1* Fab caused total stem cell death within 8-12 hours even though bFGF and conditioned media (CM) was present. Bivalent anti-MUC1* stimulated growth. Cells cultured 25 hrs; live cells were measured in a Calcein fluorescent assay.

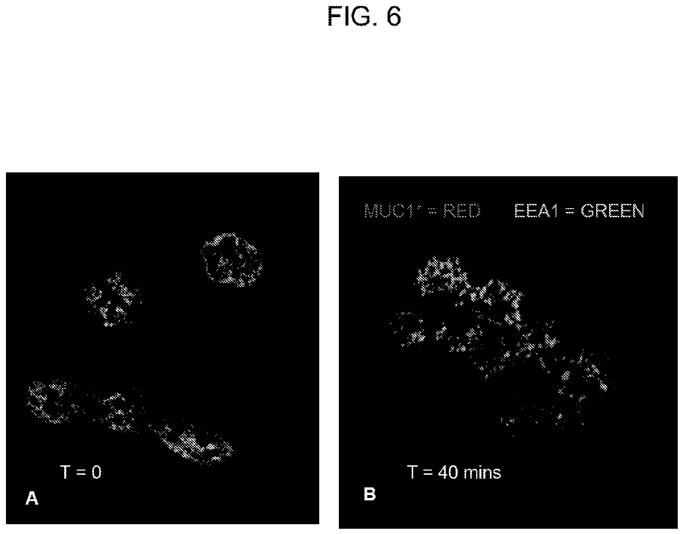

FIGURES 6A-6B show photos evidencing that MUC1* translocates to the nucleus of cells. A MUC1* Fab was fluorescently labeled (red) then incubated with MUC1*-positive cells. The photos show that, initially, MUC1* is uniformly distributed on the cell surface. However, after 40 minutes, MUC1* is concentrated in the nucleus. For comparison, cells were also stained with a fluorescently labeled antibody (green) that recognizes EEA1, which remains uniformly distributed in cytoplasm throughout the experiment. A. photo of cells taken at time zero; B. photo of cells taken 40 minutes after the addition of the Fab of anti-MUC1*.
FIGURES 7A-D are magnified photos Day 4 of an experiment wherein fibroblast cells were not transfected with any pluripotency genes but were cultured in either NM23 media (A,B) or serum containing fibroblast media (C,D).
FIGURES 8A-C show magnified photos Day 4 of an experiment wherein cells were transfected with OSKM and cultured in NM23 media (A,B) or fibroblast media (C).
FIGURES 9A-D show magnified photos Day 11 of an experiment wherein fibroblast cells were not transfected with any pluripotency genes but were cultured in NM23 media and cells were transferred onto different surfaces on Day 5: plastic (A), MEFs (B), anti-MUC1* antibody, C3 (C), or anti-MUC1* antibody, C3 plus a Rho kinase inhibitor (ROCi) (D).
FIGURES 10 A, B shows magnified photos of Day 11 of the experiment wherein the untransfected cells were cultured in fibroblast media until Day7, then cultured in the standard FGF media. Cells were transferred to MEFs on Day 5.
FIGURES 11A-D show magnified photos on Day 11 of the experiment of fibroblasts transfected with OSKM (OCT4, SOX2, KLF4 and c-Myc) and cultured in NM23 media Always (A,B), or in fibroblast media until Day 5, then Replaced with NM23 media (C, D).
FIGURES 12A-B show magnified photos on Day 11 of the experiment of fibroblasts transfected with OSKM (OCT4, SOX2, KLF4 and c-Myc) and cultured in FGF media over a surface of human feeder cells (A) or mouse feeders (B).
FIGURES 13A-D show magnified photos on Day 14 of the experiment of untransfected cells, which have been cultured in NM23-MM-A (always) over anti-MUC1* antibody (A,B) or over fibroblast feeder cells (C,D).
FIGURES 14A-C show magnified photos on Day 14 of the experiment of untransfected cells, cultured in standard FM until Day 5, then FGF media over feeder cells and show no signs of induction of pluripotency.
FIGURES 15A-C show magnified photos of the fibroblasts transfected with OSKM on Day 14 of the experiment, which have been cultured in NM23 media Always over an anti-MUC1* antibody surface (A), over plastic (B) or over MEFs (C).
FIGURES 16A-D show magnified photos Day 14 of the experiment of fibroblasts transfected with OSKM and cultured in NM23 after Day 7 (A,B) or cultured in FGF media (C,D), wherein cells were plated onto mouse feeders (A,C) or human feeders (B,D).
FIGURES 17A-D show magnified photos of the Control, untransfected cells on Day 19 of the experiment, which have been cultured in either NM23-MM-A (always) or NM23-MM-R (replaced). In the absence of transfected genes, NM23-MM induces pluripotent cell morphology.
FIGURES 18A-B show magnified photos of the Control, untransfected cells on Day 19 of the experiment, which have been cultured in FM, then FGF-MM. No induction of pluripotency can be seen.
FIGURES 19A-D show magnified photos of the fibroblasts transfected with OSKM on Day 19 of the experiment, which have been cultured in either NM23-MM-A (A,B) or NM23-MM-R (C,D). The images show that NM23-MM always enhances induction of pluripotency.
FIGURES 20A-B show magnified photos day 19 of fibroblasts transfected with OSKM, wherein cells have been cultured in FM for 7 days then FGF-MM.
FIGURES 21A-B show graphs of RT-PCR experiments on Day 4 (A) or Day 20 (B) post transfection of three or more of the pluripotency genes then assayed for the presence of pluripotency genes.
FIGURES 22A-B show graphs of RT-PCR experiments assaying for the expression of Oct4 on Day 4 (A) and on Day 20 (B) post transfection of 3 or 4 of the pluripotency genes.
FIGURES 23A-C are photos of immunocytochemistry experiments wherein transfected fibroblasts were assayed on Day 10 post transfection for the presence of the pluripotency marker Tra 1-60.
FIGURES 24A-E show photos on Day 10 post transfection of fibroblasts fluorescently stained for the presence of pluripotency marker Tra 1-60, wherein the cells had been cultured in NM23 media.
FIGURES 25A-C show photos on Day 10 post transfection of fibroblasts fluorescently stained for the presence of pluripotency marker Tra 1-60, wherein the cells had been cultured in FGF media.
FIGURES 26A-C show photos on Day 15 of the experiment of untransfected fibroblasts cultured in NM23 always (A), NM23 replacing fibroblast media on Day 7 (B) or FGF media (C).
FIGURES 27A-C show photos on Day 15 post transfection of fibroblasts with OSKM (A), OSK (B), or OSM (C) wherein all were cultured in NM23 media for the duration of the experiment.
FIGURES 28A-C shows photos on Day 15 post transfection of fibroblasts with OSKM (A), OSK (B), or OSM (C) wherein all were cultured in NM23 media from Day 7 onward.
FIGURES 29A-C show photos on Day 15 post transfection of fibroblasts with OSKM (A), OSK (B), or OSM (C) wherein all were cultured in FGF media from Day 7 onward.
FIGURES 30A-C show photos on Day 15 post transfection of fibroblasts with OSKM and cultured in NM23 media always (A), NM23 from Day 7 onward (B), or FGF media from Day 7 onward.
FIGURES 31A-C show photos on Day 15 post transfection of fibroblasts with OSK and cultured in NM23 media always (A), NM23 from Day 7 onward (B), or FGF media from Day 7 onward.
FIGURES 32A-C show photos on Day 15 post transfection of fibroblasts with OSM and cultured in NM23 media always (A), NM23 from Day 7 onward (B), or FGF media from Day 7 onward.
FIGURE 33 shows a graph of a FACS experiment in which mouse embryonic stem cells were assayed for the presence of pluripotency marker SSEA4 after being cultured in standard mouse LIF media or in NM23 media.
FIGURE 34 shows a graph of a FACS experiment in which mouse embryonic stem cells were assayed for the presence of MUC1*, using a panel of antibodies specific for MUC1*, after being cultured in standard mouse LIF media or in NM23 media.
FIGURES 35A-C show Day 19 FACS scans of cells induced to become pluripotent wherein the cells were stained for CD13, a marker of differentiated fibroblasts, and Tra 1-60, a surface marker of pluripotency. A) shows FACS scans of cells that were transfected with Oct4, Sox2, Klf4, and c-Myc (OSKM) according to the standard method and switched from fibroblast media to FGF media on Day 7 whereas B) shows FACS scans of cells also transfected with OSKM but wherein the cells were cultured in NM23 dimer media from the onset and not subjected to serum or FGF. The results are tabulated in C).
FIGURE 36 is a graph of the FACS results of Figure 35.
FIGURE 37 shows Day 18 FACS scans of cells transfected with OSKM, OSK or OSM and cultured in either FGF media, NM23 media always or only after Day7 when switched from fibroblast media. Cells were stained for CD13, a marker of differentiated fibroblasts, SSEA4 a surface marker of pluripotency and Tra 1-60, a surface marker of pluripotency.
FIGURE 38 shows tabulated results of Day 18 FACS scans measuring CD13 and Tra 1-60.
FIGURE 39 shows tabulated results of Day 18 FACS scans measuring CD13 and SSEA4.
FIGURES 40A-F show photos of a human induced pluripotent stem (iPS) cell line cultured in either FGF media over a layer of MEFs (A-C) or cultured in NME7-AB media over a layer of anti-MUC1* antibody, and assayed by immunocytochemistry for the presence of MUC1* (A,D) and pluripotency markers Rex-1 (B,E) and Tra 1-60 (C,F).
FIGURES 41A-F show photos of a human embryonic stem (ES) cell line cultured in either FGF media over a layer of MEFs (A-C) or cultured in NME7-AB media over a layer of anti-MUC1* antibody, and assayed by immunocytochemistry for the presence of MUC1* (A,D) and pluripotency markers Rex-1 (B,E) and Tra 1-60 (C,F).
FIGURES 42A-F show photos of a human iPS cell line cultured in either FGF media over a layer of MEFs (A-C) or cultured in NM23-S 120G dimer media over a layer of anti-MUC1* antibody (D-F), and assayed by immunocytochemistry for the presence of MUC1* (A,D), nuclear stain DAPI (B,E) and merged images (C,F).
FIGURES 43A-L show photos of a human iPS cell line cultured in either FGF media over a layer of MEFs (A-F) or cultured in NM23-S120G dimer media over a layer of anti-MUC1* antibody (G-L), and assayed by immunocytochemistry for the presence of MUC1* (A,G), pluripotency marker Tra 1-60 (D,J), nuclear stain DAPI (B,E,H,K) and merged images (C,F,I,L).
FIGURES 44A-C show photos of a human iPS cell line cultured in NM23-S 120G dimer media over a layer of anti-MUC1* antibody and assayed by immunocytochemistry for the presence of NME7 (A,B,C) and nuclear stain DAPI (C).

TABLE 2 shows how many stem-like colonies resulted by Day 19 from induction of pluripotency of human fibroblasts under a variety of conditions and calculates induction efficiency, which is the number of cells required for generating a single colony, and induction rate, which is the inverse of that number.

### DETAILED DESCRIPTION OF THE PREFERRED EMBODIMENTS

In the present application, "a" and "an" are used to refer to both single and a plurality of objects.

As used herein, "increasing MUC1* activity" refers to directly or indirectly increasing MUC1* signaling, and includes without limitation the dimerization of MUC1* receptor and also increased production of MUC1* by cleavage of the MUC1 receptor. MUC1* activity may be also increased by higher transcriptional expression of MUC1 receptor, which is further cleaved and dimerized. Therefore, in one aspect, MUC1* activity may be increased by a higher activity of the effector molecule that dimerizes MUC1*, or the higher activity of the cleavage molecule that cleaves MUC1 so that MUC1* is formed, or increased expression of the MUC1. Therefore, any chemical or biological species that is able to increase the activity of the MUC1* dimerizing ligand, MUC1 cleavage enzyme to form MUC1*, or any transcriptional activator that enhances expression of MUC1, is encompassed as a species that "increases MUC1* activity".

As used herein, "MUC1 Growth Factor Receptor" (MGFR) is a functional definition meaning that portion of the MUC1 receptor that interacts with an activating ligand, such as a growth factor or a modifying enzyme such as a cleavage enzyme. The MGFR region of MUC1 is that extracellular portion that is closest to the cell surface and is defined by most or all of the PSMGFR, as defined below. The MGFR is inclusive of both unmodified peptides and peptides that have undergone enzyme modifications, such as, for example, phosphorylation, glycosylation and so forth.

As used herein, "Primary Sequence of the MUC1 Growth Factor Receptor" (PSMGFR) refers to peptide sequence that defines most or all of the MGFR in some cases, and functional variants and fragments of the peptide sequence. The PSMGFR is defined as SEQ ID NO:6, and all functional variants and fragments thereof having any integer value of amino acid substitutions up to 20 (i.e. 1, 2, 3, 4, 5, 6, 7, 8, 9, 10, 11, 12, 13, 14, 15, 16, 17, 18, 19, or 20) and/or any integer value of amino acid additions or deletions up to 20 at its N-terminus and/or C-terminus. A "functional variant or fragment" in the above context refers to such variant or fragment having the ability to specifically bind to, or otherways specifically interact with, ligands that specifically bind to, or otherwise specifically interact with, the peptide of SEQ ID NO:6, while not binding strongly to identical regions of other peptide molecules identical to themselves, such that the peptide molecules would have the ability to aggregate (i.e. self-aggregate) with other identical peptide molecules. One example of a PSMGFR that is a functional variant of the PSMGFR peptide of SEQ NO:6 is SEQ ID NO:8, which differs from SEQ ID NO:6 by including an -SPY- sequence instead of the -SRY-.

As used herein, "MUC1*" refers to the MUC1 protein with the N-terminus truncated such that the extracellular domain is essentially comprised of the PSMGFR (SEQ ID NO:5).

As used herein "MUC1* associated factors" refers to agents that modify, activate, modulate the activity of, or modulate the expression of MUC1*. MUC1* associated factors include, without limitation, agents that affect dimerization of MUC1* receptor, increased production of MUC1*, induce cleavage of the MUC1 receptor, agents that increase MUC1* activity by higher transcriptional expression of MUC1 receptor, which is further cleaved and dimerized.

As used herein, "effective amount" is an amount sufficient to effect beneficial or desired clinical or biochemical results. An effective amount can be administered one or more times. For purposes of this disclosure, an effective amount of an inhibitor compound is an amount that is sufficient to induce or maintain pluripotency of a cell or activate MUC1*

As used herein, "fragments" or "functional derivatives" refers to biologically active amino acid sequence variants and fragments of the native ligands or receptors of the present disclosure, as well as covalent modifications, including derivatives obtained by reaction with organic derivatizing agents, post-translational modifications, derivatives with nonproteinaceous polymers, and immunoadhesins.

As used herein, "immature" cells refers to cells that can undergo at least one more step of differentiation and expresses markers of a particular cell type that is known to be able to undergo at least one more step of differentiation.

As used herein, "cell having less mature state than starting cell" refers to a cell that has de-differentiated so that it has an increased ability to differentiate into a different cell type than the starting cell or has an increased ability to differentiate into more cell types than the starting cell. A cell in a less mature state can be identified by measuring an increase in the expression of pluripotency markers, by a determination that the expression levels of pluripotency markers are closer to those of pluripotent stem cells or by measuring markers of a less mature state than the starting cells. For example, hematopoietic stem cells that can differentiate into any blood cell type are characterized by the expression of CD34 and the absence of CD38. As these cells differentiate, they go from CD34+/CD38- to CD34+/CD38-then to CD34-/CD38+. If one were to induce the CD34-/CD38+ cells to revert to a less mature state, the cells would regain expression of CD34. The technique of transdifferentiation involves reverting starting cells to a less mature state wherein the cells become unstable and can be directed to differentiate into a differentiate cell type than the starting cell, even if the starting cell was at the same relative level of differentiation as the resultant cell (Iede et al 2010; Efe et al 2011). For example, cardio fibroblasts have been reverted to a less mature state by brief ectopic expression of OCT4, SOX2, KLF4 and c-MYC, then from this unstable state, differentiated into cardiomyocytes.

As used herein, "ligand" refers to any molecule or agent, or compound that specifically binds covalently or transiently to a molecule such as a polypeptide. When used in certain context, ligand may include antibody. In other context, "ligand" may refer to a molecule sought to be bound by another molecule with high affinity, such as but not limited to a natural or unnatural ligand for MUC1* or a cleaving enzyme binding to MUC1 or MUC1* or a dimerizing ligand for MUC1*.

As used herein, "Naive stem cells" are those that resemble and share quantifiable characteristics with cells of the inner mass of a blastocyst. Naive stem cells have quantifiable differences in expression of certain genes compared to primed stem cells, which resemble and share traits and characteristics of cells from the epiblast portion of a blastocyst. Notably, naive stem cells of a female source have two active X chromosomes, referred to as XaXa, whereas the later primed stem cells of a female source have one of the X chromosomes inactivated.

As used herein, "NME" family proteins is a family of ten (10) proteins, some of which have been recently discovered, wherein they are categorized by their shared sequence homology to nucleoside diphosphate kinase (NDPK) domains, even though many of the NME family members are incapable of kinase activity. NME proteins were previously known as NM23-H1 and NM23-H2 then NM23-H3 through NM23-10 as they were being discovered. The different NME proteins function differently. Herein, NME1 and NME6 bind to and dimerize the MUC1* receptor (wherein its extra cellular domain is comprised essentially of the PSMGFR sequence) when they are in dimer form; NME7 has two (2) binding sites for MUC1* receptor extra cellular domain and also dimerizes the receptor. NME1 dimers, NME6 dimers and NME7 are the preferred NME family members for use as MUC1* ligands to induce or maintain cells in a less mature state than the starting cells. Other NME family members that are able to bind to and dimerize the MUC1* receptor are also contemplated for use as MUC1* ligands to induce or maintain cells in a less mature state than the starting cells.

As used herein, "pluripotency markers" are those genes and proteins whose expression is increased when cells revert to a less mature state than the starting cells. Pluripotency markers include OCT4, SOX2, NANOG, KLF4, KLF2, Tra 1-60, Tra 1-81, SSEA4, and REX-1 as well as others previously described and those currently being discovered. For example, fibroblast cells express no detectable or low levels of these pluripotency markers, but express a fibroblast differentiation marker called CD13. To determine if a cell is becoming less mature than the starting cells, one could measure a difference in the expression levels of the pluripotency markers between the starting cells and the resultant cells.

As used herein, "primed stem cells" are cells that resemble and share traits and characteristics of cells from the epiblast portion of a blastocyst.

As used herein, the term "specifically binds" refers to a non-random binding reaction between two molecules, for example between an antibody molecule immunoreacting with an antigen, or a non-antibody ligand reacting with another polypeptide, such as NM23 specifically binding with MUC1* or an antibody binding to MUC1* or a cleaving enzyme binding to MUC1 or MUC1*.

As used herein, "pluripotent" stem cell refers to stem cells that can differentiate to all three germlines, endoderm, ectoderm and mesoderm, to differentiate into any cell type in the body, but cannot give rise to a complete organism. Typical markers of pluripotency are OCT4, KLF4, NANOG, Tra 1-60, Tra 1-81 and SSEA4.

As used herein, "multipotent" stem cells refer to stem cells that can differentiate into other cell types wherein the number of different cell types is limited.

As used herein, "semi-pluripotent" or "pre-iPS state" refers to a cell that has some or all of the morphological characteristics of a pluripotent stem cell, but its level of expression of the pluripotency markers or its ability to differentiate to all three germlines is less than that of a pluripotent stem cell.

As used herein, "stem-like" morphology refers to a morphology that resembles that of a stem cell, a level of expression of one or more of the pluripotency genes, or an ability to differentiate into multiple cell types. Stem-like morphology is when the cells have a rounded shape, and are rather small compared to the size of their nucleus, which is often has a large nucleus to cytoplasm ratio, which is characteristic of pluripotent stem cells. By contrast, fibroblast morphology is when cells have a long, spindly shape and do not have a large nucleus to cytoplasm ratio. Additionally, pluripotent stem cells are non-adherent, whereas other cell types, such as fibroblasts, are adherent.

As used herein, "vector", "polynucleotide vector", "construct" and "polynucleotide construct" are used interchangeably herein. A polynucleotide vector of this disclosure may be in any of several forms, including, but not limited to, RNA, DNA, RNA encapsulated in a retroviral coat, DNA encapsulated in an adenovirus coat, DNA packaged in another viral or viral-like form (such as herpes simplex, and adeno-associated virus (AAV)), DNA encapsulated in liposomes, DNA complexed with polylysine, complexed with synthetic polycationic molecules, complexed with compounds such as polyethylene glycol (PEG) to immunologically "mask" the molecule and/or increase half-life, or conjugated to a non-viral protein. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides.

### Sequence Listing Free Text

As regards the use of nucleotide symbols other than a, g, c, t, they follow the convention set forth in WIPO Standard ST.25, Appendix 2, Table 1, wherein k represents t or g; n represents a, c, t or g; m represents a or c; r represents a or g; s represents c or g; w represents a or t and y represents c or t. 
MTPGTQSPFF LLLLLTVLTV VTGSGHASST PGGEKETSAT QRSSVPSSTE
KNAVSMTSSV LSSHSPGSGS STTQGQDVTL APATEPASGS AATWGQDVTS
VPVTRPALGS TTPPAHDVTS APDNKPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDNRPALGS
TAPPVHNVTS ASGSASGSAS TLVHNGTSAR ATTTPASKST PFSIPSHHSD
TPTTLASHST KTDASSTHHS SVPPLTSSNH STSPQLSTGV SFFFLSFHIS
NLQFNSSLED PSTDYYQELQ RDISEMFLQI YKQGGFLGLS NIKFRPGSVV
VQLTLAFREG TINVHDVETQ FNQYKTEAAS RYNLTISDVS VSDVPFPFSA
QSGAGVPGWG IALLVLVCVL VALAIVYLIA LAVCQCRRKN YGQLDIFPAR
DTYHPMSEYP TYHTHGRYVP PSSTDRSPYE KVSAGNGGSS LSYTNPAVAA
ASANL (SEQ ID NO: 1) describes full-length MUC1 Receptor (Mucin 1 precursor, Genbank
Accession number: P15941).
MTPGTQSPFFLLLLLTVLT (SEQ ID NO: 2)
MTPGTQSPFFLLLLLTVLT VVTA (SEQ ID NO: 3)
MTPGTQSPFFLLLLLTVLT VVTG (SEQ ID NO: 4)

SEQ ID NOS:2, 3 and 4 describe N-terminal MUC-1 signaling sequence for directing MUC1 receptor and truncated isoforms to cell membrane surface. Up to 3 amino acid residues may be absent at C-terminal end as indicated by variants in SEQ ID NOS:2, 3 and 4.

GTINVHDVETQFNQYKTEAASRYNLTISDVSVSDVPFPFSAQSGAGVPGWGIALLVLVCVLV ALAIVYLIALAVCQCRRKNYGQLDIFPARDTYHPMSEYPTYHTHGRYVPPSSTDRSPYEKVS AGNGGSSLSYTNPAVAAASANL (SEQ ID NO:5) describes a truncated MUC1 receptor isoform having nat-PSMGFR at its N-terminus and including the transmembrane and cytoplasmic sequences of a full-length MUC1 receptor.

GTINVHDVETQFNQYKTEAASRYNLTISDVSVSDVPFPFSAQSGA (SEQ ID NO:6) describes Native Primary Sequence of the MUC1 Growth Factor Receptor (nat-PSMGFR - an example of "PSMGFR"):
TINVHDVETQFNQYKTEAASRYNLTISDVSVSDVPFPFSAQSGA (SEQ ID NO:7) describes Native Primary Sequence of the MUC1 Growth Factor Receptor (nat-PSMGFR - An example of "PSMGFR"), having a single amino acid deletion at the N-terminus of SEQ ID NO:6).

GTINVHDVETQFNQYKTEAASPYNLTISDVSVSDVPFPFSAQSGA (SEQ ID NO:8) describes "SPY" functional variant of the native Primary Sequence of the MUC1 Growth Factor Receptor having enhanced stability (var-PSMGFR - An example of "PSMGFR").

TINVHDVETQFNQYKTEAASPYNLTISDVSVSDVPFPFSAQSGA (SEQ ID NO: 9) describes "SPY" functional variant of the native Primary Sequence of the MUC1 Growth Factor Receptor having enhanced stability (var-PSMGFR - An example of "PSMGFR"), having a single amino acid deletion at the C-terminus of SEQ ID NO:8). 
tgtcagtgccgccgaaagaactacgggcagctggacatctttccagcccgggatacctacca
tcctatgagcgagtaccccacctaccacacccatgggcgctatgtgccccctagcagtaccg
atcgtagcccctatgagaaggtttctgcaggtaacggtggcagcagcctctcttacacaaac
ccagcagtggcagccgcttctgccaacttg (SEQ ID NO:10) describes MUC1 cytoplasmic
domain nucleotide sequence.
 CQCRRKNYGQLDIFPARDTYHPMSEYPTYHTHGRYVPPSSTDRSPYEKVSAGNGGSSLSYTN PAVAAASANL (SEQ ID NO:11) describes MUC1 cytoplasmic domain amino acid sequence.

gagatcctgagacaatgaatcatagtgaaagattcgttttcattgcagagtggtatgatcca
aatgcttcacttcttcgacgttatgagcttttattttacccaggggatggatctgttgaaat
gcatgatgtaaagaatcatcgcacctttttaaagcggaccaaatatgataacctgcacttgg
aagatttatttataggcaacaaagtgaatgtcttttctcgacaactggtattaattgactat
ggggatcaatatacagctcgccagctgggcagtaggaaagaaaaaacgctagccctaattaa
accagatgcaatatcaaaggctggagaaataattgaaataataaacaaagctggatttacta
taaccaaactcaaaatgatgatgctttcaaggaaagaagcattggattttcatgtagatcac
cagtcaagaccctttttcaatgagctgatccagtttattacaactggtcctattattgccat
ggagattttaagagatgatgctatatgtgaatggaaaagactgctgggacctgcaaactctg
gagtggcacgcacagatgcttctgaaagcattagagccctctttggaacagatggcataaga
aatgcagcgcatggccctgattcttttgcttctgcggccagagaaatggagttgttttttcc

ttcaagtggaggttgtgggccggcaaacactgctaaatttactaattgtacctgttgcattg
ttaaaccccatgctgtcagtgaaggtatgttgaatacactatattcagtacattttgttaat
aggagagcaatgtttattttcttgatgtactttatgtatagaaaataa (SEQ ID NO:12)
describes NME7 nucleotide sequence (NME7: GENBANK ACCESSION AB209049).

DPETMNHSERFVFIAEWYDPNASLLRRYELLFYPGDGSVEMHDVKNHRTFLKRTKYDNLHLE
DLFIGNKVNVFSRQLVLIDYGDQYTARQLGSRKEKTLALIKPDAISKAGEIIEIINKAGFTI
TKLKMMMLSRKEALDFHVDHQSRPFFNELIQFITTGPIIAMEILRDDAICEWKRLLGPANSG
VARTDASESIRALFGTDGIRNAAHGPDSFASAAREMELFFPSSGGCGPANTAKFTNCTCCIV
KPHAVSEGMLNTLYSVHFVNRRAMFIFLMYFMYRK (SEQ ID NO:13) describes NME7
amino acid sequence (NME7: GENBANK ACCESSION AB209049).

atggtgctactgtctactttagggatcgtctttcaaggcgaggggcctcctatctcaagctg
tgatacaggaaccatggccaactgtgagcgtaccttcattgcgatcaaaccagatggggtcc
agcggggtcttgtgggagagattatcaagcgttttgagcagaaaggattccgccttgttggt
ctgaaattcatgcaagcttccgaagatcttctcaaggaacactacgttgacctgaaggaccg
tccattctttgccggcctggtgaaatacatgcactcagggccggtagttgccatggtctggg
aggggctgaatgtggtgaagacgggccgagtcatgctcggggagaccaaccctgcagactcc
aagcctgggaccatccgtggagacttctgcatacaagttggcaggaacattatacatggcag
tgattctgtggagagtgcagagaaggagatcggcttgtggtttcaccctgaggaactggtag
attacacgagctgtgctcagaactggatctatgaatga (SEQ ID NO:14) describes NM23-
H1 nucleotide sequence (NM23-H1: GENBANK ACCESSION AF487339).

MVLLSTLGIVFQGEGPPISSCDTGTMANCERTFIAIKPDGVQRGLVGEIIKRFEQKGFRLVG
LKFMQASEDLLKEHYVDLKDRPFFAGLVKYMHSGPVVAMVWEGLNVVKTGRVMLGETNPADS
KPGTIRGDFCIQVGRNIIHGSDSVESAEKEIGLWFHPEELVDYTSCAQNWIYE (SEQ ID
NO:15) NM23-H1 describes amino acid sequence (NM23-H1: GENBANK ACCESSION
AF487339).

atggtgctactgtctactttagggatcgtctttcaaggcgaggggcctcctatctcaagctg
tgatacaggaaccatggccaactgtgagcgtaccttcattgcgatcaaaccagatggggtcc
agcggggtcttgtgggagagattatcaagcgttttgagcagaaaggattccgccttgttggt
ctgaaattcatgcaagcttccgaagatcttctcaaggaacactacgttgacctgaaggaccg
tccattctttgccggcctggtgaaatacatgcactcagggccggtagttgccatggtctggg
aggggctgaatgtggtgaagacgggccgagtcatgctcggggagaccaaccctgcagactcc
aagcctgggaccatccgtggagacttctgcatacaagttggcaggaacattatacatggcgg
tgattctgtggagagtgcagagaaggagatcggcttgtggtttcaccctgaggaactggtag
attacacgagctgtgctcagaactggatctatgaatga (SEQ ID NO:16) describes NM23-
H1 S120G mutant nucleotide sequence (NM23-H1: GENBANK ACCESSION AF487339).

MVLLSTLGIVFQGEGPPISSCDTGTMANCERTFIAIKPDGVQRGLVGEIIKRFEQKGFRLVG
LKFMQASEDLLKEHYVDLKDRPFFAGLVKYMHSGPVVAMVWEGLNVVKTGRVMLGETNPADS
KPGTIRGDFCIQVGRNIIHGGDSVESAEKEIGLWFHPEELVDYTSCAQNWIYE (SEQ ID
NO:17) describes NM23-H1 S120G mutant amino acid sequence (NM23-H1: GENBANK
ACCESSION AF487339).

atg tacaacatga tggagacgga gctgaagccg ccgggcccgc agcaaacttc
ggggggcggc ggcggcaact ccaccgcggc ggcggccggc ggcaaccaga
aaaacagccc ggaccgcgtc aagcggccca tgaatgcctt catggtgtgg
tcccgcgggc agcggcgcaa gatggcccag gagaacccca agatgcacaa

ctcggagatc agcaagcgcc tgggcgccga gtggaaactt ttgtcggaga
cggagaagcg gccgttcatc gacgaggcta agcggctgcg agcgctgcac
atgaaggagc acccggatta taaataccgg ccccggcgga aaaccaagac
gctcatgaag aaggataagt acacgctgcc cggcgggctg ctggcccccg
gcggcaatag catggcgagc ggggtcgggg tgggcgccgg cctgggcgcg
ggcgtgaacc agcgcatgga cagttacgcg cacatgaacg gctggagcaa
cggcagctac agcatgatgc aggaccagct gggctacccg cagcacccgg
gcctcaatgc gcacggcgca gcgcagatgc agcccatgca ccgctacgac
gtgagcgccc tgcagtacaa ctccatgacc agctcgcaga cctacatgaa
cggctcgccc acctacagca tgtcctactc gcagcagggc acccctggca
tggctcttgg ctccatgggt tcggtggtca agtccgaggc cagctccagc
ccccctgtgg ttacctcttc ctcccactcc agggcgccct gccaggccgg
ggacctccgg gacatgatca gcatgtatct ccccggcgcc gaggtgccgg
aacccgccgc ccccagcaga cttcacatgt cccagcacta ccagagcggc
ccggtgcccg gcacggccat taacggcaca ctgcccctct cacacatgtg
a (SEQ ID NO:18) describes human SOX2 nucleotide sequence (SOX2:GENBANK
ACCESSION NM_003106).






 (SEQ ID NO:19) describes human SOX2 amino acid sequence
(SOX2:GENBANK ACCESSION NM_003106).

atggcgggacacctggcttcagattttgccttctcgccccctccaggtggtggaggtgatgg
gccaggggggccggagccgggctgggttgatcctcggacctggctaagcttccaaggccctc
ctggagggccaggaatcgggccgggggttgggccaggctctgaggtgtgggggattccccca
tgccccccgccgtatgagttctgtggggggatggcgtactgtgggccccaggttggagtggg
gctagtgccccaaggcggcttggagacctctcagcctgagggcgaagcaggagtcggggtgg
agagcaactccgatggggcctccccggagccctgcaccgtcacccctggtgccgtgaagctg
gagaaggagaagctggagcaaaacccggaggagtcccaggacatcaaagctctgcagaaaga
actcgagcaatttgccaagctcctgaagcagaagaggatcaccctgggatatacacaggccg
atgtggggctcaccctgggggttctatttgggaaggtattcagccaaacgaccatctgccgc
tttgaggctctgcagcttagcttcaagaacatgtgtaagctgcggcccttgctgcagaagtg
ggtggaggaagctgacaacaatgaaaatcttcaggagatatgcaaagcagaaaccctcgtgc
aggcccgaaagagaaagcgaaccagtatcgagaaccgagtgagaggcaacctggagaatttg
ttcctgcagtgcccgaaacccacactgcagcagatcagccacatcgcccagcagcttgggct
cgagaaggatgtggtccgagtgtggttctgtaaccggcgccagaagggcaagcgatcaagca
gcgactatgcacaacgagaggattttgaggctgctgggtctcctttctcagggggaccagtg
tcctttcctctggccccagggccccattttggtaccccaggctatgggagccctcacttcac
tgcactgtactcctcggtccctttccctgagggggaagcctttccccctgtctctgtcacca
ctctgggctctcccatgcattcaaactga (SEQ ID NO:20) describes Human OCT4
nucleotide sequence.

MAGHLASDFAFSPPPGGGDGPGGPEPGWVDPRTWLSFQGPPGGPGIGPGVGPGSEVWGIPP
CPPPYEFCGGMAYCGPQVGVGLVPQGGLETSQPEGEAGVGVESNSDGASPEPCTVTPGAVKL
EKEKLEQNPEESQDIKALQKELEQFAKLLKQKRITLGYTQADVGLTLGVLFGKVFSQTTICR
FEALQLSFKNMCKLRPLLQKWVEEADNNENLQEICKAETLVQARKRKRTSIENRVRGNLENL

FLQCPKPTLQQISHIAQQLGLEKDVVRVWFCNRRQKGKRSSSDYAQREDFEAAGSPFSGGPV
SFPLAPGPHFGTPGYGSPHFTALYSSVPFPEGEAFPPVSVTTLGSPMHSN (SEQ ID NO:21)
describes Human OCT4 amino acid sequence.

atggccaacctggagcgcaccttcatcgccatcaagccggacggcgtgcagcgcggcctggt
gggcgagatcatcaagcgcttcgagcagaagggattccgcctcgtggccatgaagttcctcc
gggcctctgaagaacacctgaagcagcactacattgacctgaaagaccgaccattcttccct
gggctggtgaagtacatgaactcagggccggttgtggccatggtctgggaggggctgaacgt
ggtgaagacaggccgagtgatgcttggggagaccaatccagcagattcaaagccaggcacca
ttcgtggggacttctgcattcaggttggcaggaacatcattcatggcagtgattcagtaaaa
agtgctgaaaaagaaatcagcctatggtttaagcctgaagaactggttgactacaagtcttg
tgctcatgactgggtctatgaataa (SEQ ID NO:22) describes NM23-H2 nucleotide
sequence (NM23-H2: GENBANK ACCESSION AK313448).

MANLERTFIAIKPDGVQRGLVGEIIKRFEQKGFRLVAMKFLRASEEHLKQHYIDLKDRPFFP
GLVKYMNSGPVVAMVWEGLNVVKTGRVMLGETNPADSKPGTIRGDFCIQVGRNIIHGSDSVK
SAEKEISLWFKPEELVDYKSCAHDWVYE (SEQ ID NO:23) describes NM23-H2 amino acid
sequence (NM23-H2: GENBANK ACCESSION AK313448).

atggctgtcagcgacgcgctgctcccatctttctccacgttcgcgtctggcccggcgggaag
ggagaagacactgcgtcaagcaggtgccccgaataaccgctggcgggaggagctctcccaca
tgaagcgacttcccccagtgcttcccgccggcccctatgacctggcggcggcgaccgtggcc
acagacctggagagcgccggagccggtgcggcttgcggcggtagcaacctggcgcccctacc
tcggagagagaccgaggagttcaacgatctcctggacctggactttattctctccaattcgc
tgacccatcctccggagtcagtggccgccaccgtgtcctcgtcagcgtcagcctcctcttcg
tcgtcgccgtcgagcagcggccctgccagcgcgccctccacctgcagcttcacctatccgat
ccgggccgggaacgacccgggcgtggcgccgggcggcacgggcggaggcctcctctatggca
gggagtccgctccccctccgacggctcccttcaacctggcggacatcaacgacgtgagcccc
tcgggcggcttcgtggccgagctcctgcggccagaattggacccggtgtacattccgccgca
gcagccgcagccgccaggtggcgggctgatgggcaagttcgtgctgaaggcgtcgctgagcg
cccctggcagcgagtacggcagcccgtcggtcatcagcgtcacgaaaggcagccctgacggc
agccacccggtggtggtggcgccctacaacggcgggccgccgcgcacgtgccccaagatcaa
gcaggaggcggtctcttcgtgcacccacttgggcgctggaccccctctcagcaatggccacc
ggccggctgcacacgacttccccctggggcggcagctccccagcaggactaccccgaccctg
ggtcttgaggaagtgctgagcagcagggactgtcaccctgccctgccgcttcctcccggctt
ccatccccacccggggcccaattacccatccttcctgcccgatcagatgcagccgcaagtcc
cgccgctccattaccaagagctcatgccacccggttcctgcatgccagaggagcccaagcca
aagaggggaagacgatcgtggccccggaaaaggaccgccacccacacttgtgattacgcggg
ctgcggcaaaacctacacaaagagttcccatctcaaggcacacctgcgaacccacacaggtg
agaaaccttaccactgtgactgggacggctgtggatggaaattcgcccgctcagatgaactg
accaggcactaccgtaaacacacggggcaccgcccgttccagtgccaaaaatgcgaccgagc
attttccaggtcggaccacctcgccttacacatgaagaggcatttt (SEQ ID NO:24)
describes KLF4 nucleotide sequence (KLF4: GENBANK ACCESSION AF022184).









 (SEQ ID NO:25) describes KLF4
amino acid sequence (KLF4: GENBANK ACCESSION AF022184).

atggatttttttcgggtagtggaaaaccagcagcctcccgcgacgatgcccctcaacgttag
cttcaccaacaggaactatgacctcgactacgactcggtgcagccgtatttctactgcgacg
aggaggagaacttctaccagcagcagcagcagagcgagctgcagcccccggcgcccagcgag
gatatctggaagaaattcgagctgctgcccaccccgcccctgtcccctagccgccgctccgg
gctctgctcgccctcctacgttgcggtcacacccttctcccttcggggagacaacgacggcg
gtggcgggagcttctccacggccgaccagctggagatggtgaccgagctgctgggaggagac
atggtgaaccagagtttcatctgcgacccggacgacgagaccttcatcaaaaacatcatcat
ccaggactgtatgtggagcggcttctcggccgccgccaagctcgtctcagagaagctggcct
cctaccaggctgcgcgcaaagacagcggcagcccgaaccccgcccgcggccacagcgtctgc
tccacctccagcttgtacctgcaggatctgagcgccgccgcctcagagtgcatcgacccctc
ggtggtcttcccctaccctctcaacgacagcagctcgcccaagtcctgcgcctcgcaagact
ccagcgccttctctccgtcctcggattctctgctctcctcgacggagtcctccccgcagggc
agccccgagcccctggtgctccatgaggagacaccgcccaccaccagcagcgactctgagga
ggaacaagaagatgaggaagaaatcgatgttgtttctgtggaaaagaggcaggctcctggca
aaaggtcagagtctggatcaccttctgctggaggccacagcaaacctcctcacagcccactg
gtcctcaagaggtgccacgtctccacacatcagcacaactacgcagcgcctccctccactcg
gaaggactatcctgctgccaagagggtcaagttggacagtgtcagagtcctgagacagatca
gcaacaaccgaaaatgcaccagccccaggtcctcggacaccgaggagaatgtcaagaggcga
acacacaacgtcttggagcgccagaggaggaacgagctaaaacggagcttttttgccctgcg
tgaccagatcccggagttggaaaacaatgaaaaggcccccaaggtagttatccttaaaaaag
ccacagcatacatcctgtccgtccaagcagaggagcaaaagctcatttctgaagaggacttg
ttgcggaaacgacgagaacagttgaaacacaaacttgaacagctacggaactcttgtgcg
(SEQ ID NO:26) describes c-Myc nucleotide sequence (c-Myc:GENBANK ACCESSION
BC000917).

MDFFRVVENQQPPATMPLNVSFTNRNYDLDYDSVQPYFYCDEEENFYQQQQQSELQPPAPSE
DIWKKFELLPTPPLSPSRRSGLCSPSYVAVTPFSLRGDNDGGGGSFSTADQLEMVTELLGGD
MVNQSFICDPDDETFIKNIIIQDCMWSGFSAAAKLVSEKLASYQAARKDSGSPNPARGHSVC
STSSLYLQDLSAAASECIDPSVVFPYPLNDSSSPKSCASQDSSAFSPSSDSLLSSTESSPQG
SPEPLVLHEETPPTTSSDSEEEQEDEEEIDVVSVEKRQAPGKRSESGSPSAGGHSKPPHSPL
VLKRCHVSTHQHNYAAPPSTRKDYPAAKRVKLDSVRVLRQISNNRKCTSPRSSDTEENVKRR
THNVLERQRRNELKRSFFALRDQIPELENNEKAPKVVILKKATAYILSVQAEEQKLISEEDL
LRKRREQLKHKLEQLRNSCA (SEQ ID NO:27) describes c-Myc amino acid sequence (c-
Myc: GENBANK ACCESSION BC000917).

atgggctccgtgtccaaccagcagtttgcaggtggctgcgccaaggcggcagaagaggcgcc
cgaggaggcgccggaggacgcggcccgggcggcggacgagcctcagctgctgcacggtgcgg
gcatctgtaagtggttcaacgtgcgcatggggttcggcttcctgtccatgaccgcccgcgcc
ggggtcgcgctcgaccccccagtggatgtctttgtgcaccagagtaagctgcacatggaagg
gttccggagcttgaaggagggtgaggcagtggagttcacctttaagaagtcagccaagggtc
tggaatccatccgtgtcaccggacctggtggagtattctgtattgggagtgagaggcggcca
aaaggaaagagcatgcagaagcgcagatcaaaaggagacaggtgctacaactgtggaggtct
agatcatcatgccaaggaatgcaagctgccaccccagcccaagaagtgccacttctgccaga
gcatcagccatatggtagcctcatgtccgctgaaggcccagcagggccctagtgcacaggga
aagccaacctactttcgagaggaagaagaaatccacagccctaccctgctcccggaggc

acagaat (SEQ ID NO:28) describes LIN28 nucleotide sequence (LIN28: GENBANK
ACCESSION AF521099).

MGSVSNQQFAGGCAKAAEEAPEEAPEDAARAADEPQLLHGAGICKWFNVRMGFGFLSMTARA
GVALDPPVDVFVHQSKLHMEGFRSLKEGEAVEFTFKKSAKGLESIRVTGPGGVFCIGSERRP
KGKSMQKRRSKGDRCYNCGGLDHHAKECKLPPQPKKCHFCQSISHMVASCPLKAQQGPSAQG
KPTYFREEEEEIHSPTLLPEAQN (SEQ ID NO:29) describes LIN28 amino acid sequence
(LIN28: GENBANK ACCESSION AF521099).

atgtctcccgccccaagaccctcccgttgtctcctgctccccctgctcacgctcggcaccgc
gctcgcctccctcggctcggcccaaagcagcagcttcagccccgaagcctggctacagcaat
atggctacctgcctcccggggacctacgtacccacacacagcgctcaccccagtcactctca
gcggccatcgctgccatgcagaagttttacggcttgcaagtaacaggcaaagctgatgcaga
caccatgaaggccatgaggcgcccccgatgtggtgttccagacaagtttggggctgagatca
aggccaatgttcgaaggaagcgctacgccatccagggtctcaaatggcaacataatgaaatc
actttctgcatccagaattacacccccaaggtgggcgagtatgccacatacgaggccattcg
caaggcgttccgcgtgtgggagagtgccacaccactgcgcttccgcgaggtgccctatgcct
acatccgtgagggccatgagaagcaggccgacatcatgatcttctttgccgagggcttccat
ggcgacagcacgcccttcgatggtgagggcggcttcctggcccatgcctacttcccaggccc
caacattggaggagacacccactttgactctgccgagccttggactgtcaggaatgaggatc
tgaatggaaatgacatcttcctggtggctgtgcacgagctgggccatgccctggggctcgag
cattccagtgacccctcggccatcatggcacccttttaccagtggatggacacggagaattt
tgtgctgcccgatgatgaccgccggggcatccagcaactttatgggggtgagtcagggttcc
ccaccaagatgccccctcaacccaggactacctcccggccttctgttcctgataaacccaaa
aaccccacctatgggcccaacatctgtgacgggaactttgacaccgtggccatgctccgagg
ggagatgtttgtcttcaaggagcgctggttctggcgggtgaggaataaccaagtgatggatg
gatacccaatgcccattggccagttctggcggggcctgcctgcgtccatcaacactgcctac
gagaggaaggatggcaaattcgtcttcttcaaaggagacaagcattgggtgtttgatgaggc
gtccctggaacctggctaccccaagcacattaaggagctgggccgagggctgcctaccgaca
agattgatgctgctctcttctggatgcccaatggaaagacctacttcttccgtggaaacaag
tactaccgtttcaacgaagagctcagggcagtggatagcgagtaccccaagaacatcaaagt
ctgggaagggatccctgagtctcccagagggtcattcatgggcagcgatgaagtcttcactt
acttctacaaggggaacaaatactggaaattcaacaaccagaagctgaaggtagaaccgggc
taccccaagtcagccctgagggactggatgggctgcccatcgggaggccggccggatgaggg
gactgaggaggagacggaggtgatcatcattgaggtggacgaggagggcggcggggcggtga
gcgcggctgccgtggtgctgcccgtgctgctgctgctcctggtgctggcggtgggccttgca
gtcttcttcttcagacgccatgggacccccaggcgactgctctactgccagcgttccctgct
ggacaaggtc (SEQ ID NO:30) describes MMP14 nucleotide sequence (MMP14:
GENBANK ACCESSION BC064803).

MSPAPRPSRCLLLPLLTLGTALASLGSAQSSSFSPEAWLQQYGYLPPGDLRTHTQRSPQSLS
AAIAAMQKFYGLQVTGKADADTMKAMRRPRCGVPDKFGAEIKANVRRKRYAIQGLKWQHNEI
TFCIQNYTPKVGEYATYEAIRKAFRVWESATPLRFREVPYAYIREGHEKQADIMIFFAEGFH
GDSTPFDGEGGFLAHAYFPGPNIGGDTHFDSAEPWTVRNEDLNGNDIFLVAVHELGHALGLE
HSSDPSAIMAPFYQWMDTENFVLPDDDRRGIQQLYGGESGFPTKMPPQPRTTSRPSVPDKPK
NPTYGPNICDGNFDTVAMLRGEMFVFKERWFWRVRNNQVMDGYPMPIGQFWRGLPASINTAY
ERKDGKFVFFKGDKHWVFDEASLEPGYPKHIKELGRGLPTDKIDAALFWMPNGKTYFFRGNK
YYRFNEELRAVDSEYPKNIKVWEGIPESPRGSFMGSDEVFTYFYKGNKYWKFNNQKLKVEPG
YPKSALRDWMGCPSGGRPDEGTEEETEVIIIEVDEEGGGAVSAAAVVLPVLLLLLVLAVGLA

VFFFRRHGTPRRLLYCQRSLLDKV (SEQ ID NO:31) describes MMP14 amino acid
sequence (MMP14: GENBANK ACCESSION BC064803).

atgatcttactcacattcagcactggaagacggttggatttcgtgcatcattcgggggtgtt
tttcttgcaaaccttgctttggattttatgtgctacagtctgcggaacggagcagtatttca
atgtggaggtttggttacaaaagtacggctaccttccaccgactgaccccagaatgtcagtg
ctgcgctctgcagagaccatgcagtctgccctagctgccatgcagcagttctatggcattaa
catgacaggaaaagtggacagaaacacaattgactggatgaagaagccccgatgcggtgtac
ctgaccagacaagaggtagctccaaatttcatattcgtcgaaagcgatatgcattgacagga
cagaaatggcagcacaagcacatcacttacagtataaagaacgtaactccaaaagtaggaga
ccctgagactcgtaaagctattcgccgtgcctttgatgtgtggcagaatgtaactcctctga
catttgaagaagttccctacagtgaattagaaaatggcaaacgtgatgtggatataaccatt
atttttgcatctggtttccatggggacagctctccctttgatggagagggaggatttttggc
acatgcctacttccctggaccaggaattggaggagatacccattttgactcagatgagccat
ggacactaggaaatcctaatcatgatggaaatgacttatttcttgtagcagtccatgaactg
ggacatgctctgggattggagcattccaatgaccccactgccatcatggctccattttacca
gtacatggaaacagacaacttcaaactacctaatgatgatttacagggcatccagaaaatat
atggtccacctgacaagattcctccacctacaagacctctaccgacagtgcccccacaccgc
tctattcctccggctgacccaaggaaaaatgacaggccaaaacctcctcggcctccaaccgg
cagaccctcctatcccggagccaaacccaacatctgtgatgggaactttaacactctagcta
ttcttcgtcgtgagatgtttgttttcaaggaccagtggttttggcgagtgagaaacaacagg
gtgatggatggatacccaatgcaaattacttacttctggcggggcttgcctcctagtatcga
tgcagtttatgaaaatagcgacgggaattttgtgttctttaaaggtaacaaatattgggtgt
tcaaggatacaactcttcaacctggttaccctcatgacttgataacccttggaagtggaatt
ccccctcatggtattgattcagccatttggtgggaggacgtcgggaaaacctatttcttcaa
gggagacagatattggagatatagtgaagaaatgaaaacaatggaccctggctatcccaagc
caatcacagtctggaaagggatccctgaatctcctcagggagcatttgtacacaaagaaaat
ggctttacgtatttctacaaaggaaaggagtattggaaattcaacaaccagatactcaaggt
agaacctggacatccaagatccatcctcaaggattttatgggctgtgatggaccaacagaca
gagttaaagaaggacacagcccaccagatgatgtagacattgtcatcaaactggacaacaca
gccagcactgtgaaagccatagctattgtcattccctgcatcttggccttatgcctccttgt
attggtttacactgtgttccagttcaagaggaaaggaacaccccgccacatactgtactgta
aacgctctatgcaagagtgggtg (SEQ ID NO:32) describes MMP16 nucleotide sequence
(MMP16:GENBANK ACCESSION AB009303).










 (SEQ ID NO:33)
describes MMP 16 amino acid sequence (MMP16:GENBANK ACCESSION AB009303)

Human NME7-AB sequence optimized for *E. coli* expression:
(DNA) 
atggaaaaaacgctggccctgattaaaccggatgcaatctccaaagctggcgaaattatcgaaattatcaacaaagcg
ggtttcaccatcacgaaactgaaaatgatgatgctgagccgtaaagaagccctggattttcatgtcgaccaccagtctcgcccgtttttca
atgaactgattcaattcatcaccacgggtccgattatcgcaatggaaattctgcgtgatgacgctatctgcgaatggaaacgcctgctgg
gcccggcaaactcaggtgttgcgcgtaccgatgccagtgaatccattcgcgctctgtttggcaccgatggtatccgtaatgcagcacat
ggtccggactcattcgcatcggcagctcgtgaaatggaactgtttttcccgagctctggcggttgcggtccggcaaacaccgccaaatt
taccaattgtacgtgctgtattgtcaaaccgcacgcagtgtcagaaggcctgctgggtaaaattctgatggcaatccgtgatgctggcttt
gaaatctcggccatgcagatgttcaacatggaccgcgttaacgtcgaagaattctacgaagtttacaaaggcgtggttaccgaatatca
cgatatggttacggaaatgtactccggtccgtgcgtcgcgatggaaattcagcaaaacaatgccaccaaaacgtttcgtgaattctgtgg
tccggcagatccggaaatcgcacgtcatctgcgtccgggtaccctgcgcgcaatttttggtaaaacgaaaatccagaacgctgtgcact
gtaccgatctgccggaagacggtctgctggaagttcaatactttttcaaaattctggataattga (SEQ ID NO:34) describes
NME7-AB nucleotide sequence
(amino acids)

MEKTLALIKPDAISKAGEIIEIINKAGFTITKLKMMMLSRKEALDFHVDHQS
RPFFNELIQFITTGPIIAMEILRDDAICEWKRLLGPANSGVARTDASESIRALFGTDGIR
NAAHGPDSFASAAREMELFFPSSGGCGPANTAKFTNCTCCIVKPHAVSEGLLGKILM
AIRDAGFEISAMQMFNMDRVNVEEFYEVYKGVVTEYHDMVTEMYSGPCVAMEIQQ
NNATKTFREFCGPADPEIARHLRPGTLRAIFGKTKIQNAVHCTDLPEDGLLEVQYFFK
ILDN- (SEQ ID NO:35) describes NME7-AB amino acid sequence

GGFLGLSNIKFRPGSVVVQLTLAFREGTINVHDVETQFNQYKTEAASRYN LTISDVSVSDVPFPFSAQSGAC (SEQ ID NO:36) describes membrane proximal portion of human MUC1 receptor.

QFNQYKTEAASRYNLTISDVSVSDVPFPFSAQSGA (SEQ ID NO:37) describes "N-10," which is missing ten amino acids at the N-terminus of PSMGFR.

GTINVHDVETQFNQYKTEAASRYNLTISDVSVSDV (SEQ ID NO:38) describes amino sequence encompassing N-terminal adjacent portion of the amino acid sequence of SEQ ID NO:37.

GGFLGLSNIKFRPGSVVVQLTLAFRE (SEQ ID NO:39) describes self-aggregation domain of MUC1.

HHHHHH-SSSSGSSSSGSSSSGGRGDSGRGDS (SEQ ID NO:40) describes an irrelevant peptide.

### Induction of cells to less mature state

It was recently discovered that somatic cells can be reprogrammed to revert to the pluripotent state. The genes that code for transcription factors OCT4, SOX2, KLF4, NANOG, c-MYC and LIN28 or the proteins themselves can be introduced into somatic cells and cause a reversion to the pluripotent state. Many of these pluripotency factors were previously thought of as oncogenes. C-Myc is a well known oncogene and similarly, Klf4 has been shown to induce dysplasia (Foster et al., 2005). OCT4 was once thought of as the gold standard for identifying pluripotent stem cells. The presence of OCT4 in the nucleus was thought to indicate that the cell is pluripotent and its absence indicates that the cell has entered the differentiation process and is no longer able to differentiate into any cell type. Recently, it became known that OCT4 is also present in the nucleus of many cancer cells, but not in normal mature cells. The present inventors recently discovered that a cleaved form of the MUC1 transmembrane protein (SEQ ID NO:1), MUC1*, is a powerful growth factor receptor that is expressed on an estimated 75% of solid tumor cancers (Raina et al., 2009) and that it is also expressed in this "tumorigenic" form on all pluripotent stem cells, including embryonic stem (ES) cells as well as induced pluripotent stem (iPS) cells (Hikita et al., 2008; Smagghe et al, 2013). The present disclosure relates to MUC1* and MUC1* associated factors as well as methods employing them for the induction or maintenance of pluripotency or to enhance the efficiency of inducing pluripotency.

MUC1* is a primal growth factor receptor that mediates growth and pluripotency of stem cells and cancer cells. Introducing MUC1*-associated factors induces cells to revert to a less mature state than that of the starting cells. NM23 in a bivalent or dimeric form is a ligand of MUC1* growth factor receptor. Interruption of the NM23-MUC1* interaction induces expression of microRNA-145 (miR-145) which is a microRNA that signals pluripotent stem cells to exit from pluripotency and initiate a maturation process.

Treating somatic cells with a protein belonging to NME family of proteins such as dimeric NM23, a bivalent NM23, or NME7 in the presence or absence of pluripotency genes, caused mature cells to revert to a less mature state than the starting state of the cell. Fibroblasts that were transfected with two or more of the pluripotency genes, Oct4, Sox2, Klf4 and c-Myc, were induced to become pluripotent stem cells at a much faster rate and with enhanced efficiency of induction when they were cultured in a medium that contained dimeric NM23. Cells that were not transfected with any of the pluripotency genes but cultured in dimeric NM23 expressed Oct4 and reverted to a stem-like morphology within days. Additionally, somatic cells treated with a bivalent antibody that recognizes the PSMGFR portion of MUC1*, also reverted to a less differentiated state than the starting cells. Thus, bivalent MUC1* ligands, such as dimeric NME family proteins, in particular NME7 which has two binding sites for MUC1* and thus dimerizes MUC1*, NME family members in dimeric form or antibodies against the PSMGFR region, promote growth of undifferentiated stem cells, as well as inducing cells to revert to a less mature state, wherein the cells that can be reverted to a less mature state are chosen from the group comprising luripotent stem cells, multipotent stem cells as well as differentiated cells.

In addition to making mature cells revert to a less mature state or further to a pluripotent state, we have also demonstrated that treating stem cells with a MUC1 ligand such as NM23 dimers or NME7 causes the stem cells to revert to a less mature state, i.e. a more pluripotent state.

### Stem cell rescue

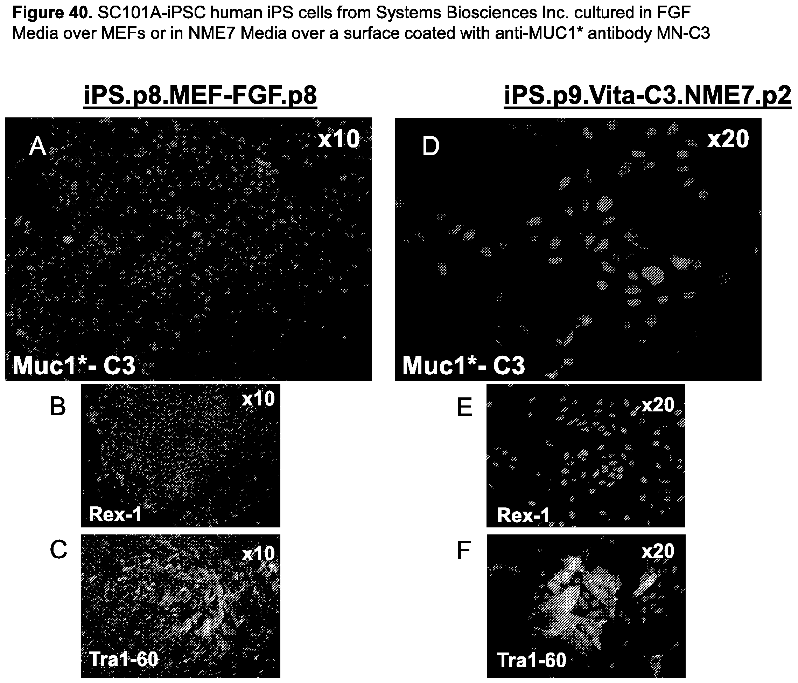

Stem cell lines often "go bad" as evidenced by their inability to differentiate properly. Researchers may use a stem cell line for months that can be directed to differentiate into cardiomyocytes, for example, then efficiency of differentiation begins to decline and eventually, the cell line just stops working. When the cell lines are assayed for the presence of typical pluripotency markers, the reduction in the expression of convenient surface markers like Tra 1-60, SSEA4 or Rex-1 is slight. However, if these cells are then assayed for the presence of MUC1*, we found that there was minimal expression of MUC1*. Treatment of these "pluripotent" stem cells (iPS or ES) with NM23 dimers or NME7 caused a dramatic increase n the expression of MUC1* that coincided with increased expression of the pluripotency markers. Some experiments that studied this aspect of the disclosure are detailed in Example 14, Figures 40-44. Once the cells were treated with NM23 dimers or NME7, the cells differentiated with a much higher efficiency than the starting cell.

Because stem cells differentiate into mature adult cells in stages, it is not necessary to bring cells all the way back to a pluripotent stem cell state before having them differentiate into mature cells. Cells can be differentiated to a desired cell type from an interim state. Therefore, cells can merely be induced to revert to a less mature state from which they are able to differentiate into the desired cell or tissue type. Some refer to this interim, less mature state as a pre-iPS state. The technique is referred to as "transdifferentiation" and sometimes "direct differentiation." For example, somatic cells or mature cells such as fibroblasts or dermablasts can be induced to become somewhat pluripotent, and then directed to differentiate or be allowed to differentiate into some desired cell type (Iede et al 2010; Efe et al 2011). For example, cardio fibroblasts can be brought to a less mature state and then differentiated into beating cardiomyocytes. In other cases, it is advantageous to start with cells from the same lineage as the desired final cell type. In this way, cells are only reverted to a less mature state, which is earlier than some decision point, and then directed to differentiate into the desired cell type.

Methods for directing differentiation into cardiomyocytes, neuronal cells, islet cells and the like are known by those skilled in the art. The hurdles that need to be overcome include very low efficiency of directed differentiation and the use of plasmids and viral vectors to introduce agents to induce pluripotency. Protein agents that induce cells to revert to a less mature state, increase efficiency of that induction and/or increase the efficiency of directed differentiation would solve these problems that have thus far prevented clinical application of stem cell therapies. NM23 and/or other MUC1* associated factors can be introduced to cells to induce them to become less mature or more stem-like. NM23 can be used alone or in conjunction with other factors to induce cells to become stem-like. In one embodiment, NM23 in the dimeric form or in a bivalent form is provided to cells to induce cells to become less mature. In other embodiments, NM23 (H1 or H6 dimers or NME7) is added along with other genes, proteins, or small molecules that induce cells to become more stem-like. From this stem-like or semi-pluripotent state, the induced cells can be allowed to differentiate into a desired cell type by merely placing the cells in an environment of cells of the desired cell type or in an environment of factors that will influence the induced cells to differentiate into the desired cell or tissue type.

In one, the cells that are induced to become less mature are cells present in a host animal or human. In some cases, the factor(s) that induce the cells to become less mature are added systemically. In other cases, the factor(s) that induce the cells to become less mature are added locally. To facilitate the local introduction of these factor(s), the inducing factors can be impregnated into or attached to a dressing, for example, to expedite wound healing. Alternatively, they can be injected locally, alone, or in a carrier material, which could be a hydrogel or other material. The inducing factor(s) could also be attached to a biocompatible material that could be topically applied, surgically inserted or ingested. Cartilage repair could be facilitated by introducing factor(s) that induce the cells to become less mature into a joint. Persons suffering from neurodegenerative diseases such as Alzheimer's or Parkinson's diseases could be treated by inducing local brain cells to revert to a less mature state from which they would be able to differentiate into functional brain cells.

The disclosure also includes attaching factors that induce cells to become less mature to substrates that perform an unrelated function, such as stents for blood vessel repair, tape-like materials to hold two pieces of substance together while encouraging cellular regeneration in the gap, scaffolds to shape the formation of tissues either over or within the structure, substrates that are patterned for example for the formation of nerves and other biological structures. The disclosure further includes attaching factors that induce a semi-pluripotent state to substrates for the generation of structured cells and tissues such as those that make up the eye.

In some cases, factors that direct the pre-iPS cells to differentiate are added either concurrently or at a later time to the site of the cells that were induced to become less mature. In other cases, no factors that direct differentiation are added. Instead, factors secreted by the local environment are relied upon to direct the induced cells to differentiate into the desired cell type or tissue type.

In a preferred case, the factors that induce cells to become less mature are MUC1 *-associated factors, including but not limited to bivalent anti-MUC1* antibodies and antibody-like proteins, enzymes or agents that increase MUC1 cleavage, as well as introduction of genes that increase expression of MUC1 or NM23 (H1 or H6 dimers or NME7). The disclosure includes introducing a nucleic acid that codes for a MUC1 cleavage product whose extra cellular domain is comprised essentially of the PSMGFR sequence which is the approximately 45 amino acids that are membrane proximal. In an especially preferred case the MUC1* associated factor is NM23 in a bivalent or dimeric form, except when NME7 is used as it is a natural "dimer" having two binding sites for MUC1* and able to dimerize it. MUC1* associated factors that induce cells to become less mature can be added alone or together with other pluripotency inducing factors including but not limited to OCT4, SOX2, KLF4, NANOG, c-MYC and/or LIN28.

### Sequence homology of MUC1 among mammals

The portions of MUC1 that are membrane proximal are highly conserved among mammals. The membrane proximal portion of human MUC1 is: N-*GGFLGLSNIKFRPGSVVVQLTLAFRE*GTINVHDVET**QFNQYKTEAASRYNLTISDVSV SDVPFPFSAQSGAC** (SEQ ID NO:36).

We previously showed that the MUC1* activating ligand NM23 binds to the portion of MUC1* that contains the sequence **QFNQYKTEAASRYNLTISDVSVSDVPFPFSAQSGA** (SEQ ID NO:37), also referred to in our previous patent applications as "N-10," which is missing ten amino acids at the N-terminus of PSMGFR. This portion of MUC1 is 72% homologous between human and mouse with 58% identity. The N-terminal adjacent portion that contains the sequence GTINVHDVET**QFNQYKTEAASRYNLTISDVSVSDV** (SEQ ID NO:38) is 71% homologous between human and mouse with 47% sequence identity. The portion of MUC1 that we previously showed is a self-aggregation domain, *GGFLGLSNIKFRPGSVVVQLTLAFRE* (SEQ ID NO:39), is 85% homologous between human and mouse with 69% identity.

Because of the great sequence conservation, ligands that recognize human MUC1* receptor, also recognize murine MUC1* receptor. For example, human NM23 in dimeric or bivalent form binds to MUC1 on mouse embryonic stem cells and enables growth while maintaining as well as inducing pluripotency. The addition of human NM23 dimers in minimal stem cell media (MM) completely abolished the need for LIF. Further, growth of mouse ES cells in LIF increased the percentage of cells that expressed pluripotency markers and also increased expression of MUC1, which is itself a pluripotency factor. Therefore, human or mouse NM23 in dimer or bivalent form promotes growth and pluripotency of mouse stem cells, embryonic or hematopoietic, in the absence of any other growth factors. In addition, human or mouse NM23 induces pluripotency in mouse somatic cells. In addition to the natural ligand, NM23, bivalent antibodies that recognize the membrane proximal portion of MUC1 promote and maintain as well as induce pluripotency in murine cells. Because of the great sequence conservation in the membrane proximal regions of MUC1, the invention includes the use of NM23 as well as bivalent antibodies, which recognize the approximately 50 membrane proximal amino acids, for the growth, maintenance and induction of pluripotency in mammalian cells and in mammals in general.

The present disclosurc also encompasses using MUC1* associated factors, which include protein factors, genes that encode them, or small molecules that affect their expression, to induce or improve the efficiency of generating iPS cells. We have shown that a cleaved form of the MUC1 transmembrane protein - MUC1* - is a primal growth factor receptor that mediates the growth of both cancer and pluripotent stem cells. Complete disruption of the interaction between MUC1* extracellular domain and its activating ligand, NM23, is lethal to pluripotent stem cells (Hikita et al., 2008), while treatment with lower concentrations of these inhibitors induced differentiation. The interaction between MUC1* and NM23 was specifically interrupted by treating with an anti-MUC1* Fab to block NM23-induced dimerization of the MUC1* receptor or by adding a synthetic peptide having the same sequence as the MUC1* extra cellular domain so that it would competitively inhibit the interaction between NM23 and its target MUC1* extra cellular domain. These findings indicate that the MUC1-NM23 pathway is critical for pluripotency. NM23 is a ligand that activates MUC1* (Mahanta et al., 2008, Hikita et al, 2008; Smagghe et al, 2013) (SEQ ID NOS:12-17, 22-23, and 34-35). In addition to its ability to stimulate pluripotent stem cell growth, while inhibiting differentiation, NM23 has been reported to induce transcription of c-Myc (Dexheimer at al., 2009), which is a known pluripotency factor. In addition, stimulation of MUC1*, by either NM23 or a bivalent anti-MUC1* antibody, activates the MAP kinase proliferation pathway, which increases cell survival (Mahanta et al., 2008). NANOG expression induces pluripotency; the tumor suppressor p53 suppresses Nanog expression (Lin et al., 2007). Therefore, the need for NANOG for inducing pluripotency is reduced or eliminated by suppressing p53. An ectopically expressed 72-amino acid fragment of the MUC1 cytoplasmic tail (MUC1-CT) has been shown to be present in the nucleus of cancer cells where it binds to the p53 promoter (Wei et al., 2007). The approximately 72 amino acid fragment of MUC1-CD such as shown in SEQ ID NO:11 can be used in combination with other pluripotency-inducing factors to induce or enhance iPS cell generation. However, this peptide does not correspond to a naturally occurring MUC1 species, and therefore may produce undesired effects. The present inventors disclose that MUC1* translocates to the nucleus (Examples 1 and 9, and Figure 6) and therefore is used alone or in combination with other pluripotency-inducing factors to induce or enhance iPS cell generation. In support of this approach, it has been reported that several genes from the core set of pluripotency genes regulate transcription of MUC1, its cleavage enzyme and/or its activating ligand NM23 (Boyer et al., 2005). OCT4 and SOX2 bind to the MUC1 promoter and also to the promoter of its cleavage enzyme, MMP-14. SOX2 and NANOG bind to the NM23 promoter. Given that MUC1* is critical for maintenance of hESCs and is the target of the key pluripotency genes, we disclose that the introduction of MUC1*, or agents that increase cleavage of MUC1 to the MUC1* form, along with its activating ligand, NM23 can be used to replace some or all of the previously identified pluripotency-inducing factors to induce or enhance the generation of iPS cells.

Described novel reagents and methods, involving MUC1* and its ligands, for inducing cells to revert to a less mature state and even to a pluripotent state. These reagents and methods are used to induce pluripotency in somatic or mature cells. Alternatively, they can be used to induce cells to a less mature state wherein the starting cells are mature cells, progenitor cells or cells that are partially differentiated. In another aspect of the disclosure, they are used to increase the efficiency of inducing pluripotency in mature cells. In yet another aspect of the disclosure they are used to maintain immature cells in an immature state. In another aspect of the disclosure they are used to inhibit differentiation. In another aspect of the disclosure, these reagents and methods are used for maintaining stem cells in the pluripotent state.

The invention involves reversing differentiation or maintaining stem-like characteristics by introducing to mature cells, or somewhat differentiated cells, genes or gene products that affect the expression of MUC1* and its associated factors. MUC1* is the cleaved form of the transmembrane protein MUC1. MUC1* associated factors include, but are not limited to, enzymes that cleave MUC1, MUC1* activating ligands and also transcription factors that affect the expression of MUC1 or MUC1*. The invention is also drawn to the introduction of the genes or gene products for MUC1* or MUC1* associated factors to mature cells or somewhat differentiated cells, which will induce pluripotency or stem-ness in those cells or their progeny. The present application describes their use for maintaining pluripotency in stem cells. Agents that affect expression of MUC1* or MUC1* associated factors, such as NM23-H1 dimers or NM23-H7 monomers, can be added in combination with, or to replace one or more genes or gene products that are already known to induce pluripotency including OCT4, SOX2, KLF4, NANOG, c-MYC and LIN28.

Forced expression of combinations of the transcription factors, Oct4, Sox2, Klf4 and c-Myc or Oct4, Sox2, Nanog and Lin28 have been shown to cause mature cells to revert to the pluripotent state (Takahashi and Yamanaka, 2006). Each of the transcription factors that induce pluripotency regulates the transcription of about a dozen genes. Among these were several that the inventor has identified as being MUC1-associated factors. OCT4 and SOX2 bind to the MUC1 promoter itself. SOX2 and NANOG bind to the NM23 (NME7) promoter. NM23 (also known as NME) was previously identified, by the present inventor, as the activating ligand of MUC1* (Mahanta et al., 2008). NM23-H1 (also called NME1) binds to MUC1* extra cellular domain and induces dimerization of MUC1* if the NM23-H1 is in dimer form; at higher concentrations, or without mutations that make NM23 prefer dimer formation, NM23 wild type is a hexamer, which does not bind to MUC1* or dimerize it. Therefore, it is only NM23-H1 in dimeric form that induces pluripotency in cells. NM23-H6, also called NME6 can also be dimeric and as such binds to and dimerizes MUC1* growth factor receptor which induces pluripotency. NME7 is also an activating ligand of MUC1*. However, NME7 as a monomer has two binding sites for the MUC1* extra cellular domain and dimerizes MUC1*, thus inducing and maintaining pluripotency. OCT4 and SOX2 both bind to the promoter for MMP16 which we disclose herein is a cleavage enzyme of MUC1. OCT4, SOX2 or NANOG also bind to promoter sites for cleavage enzymes MMP2, MMP9, MMP10, ADAM TSL-1, ADAM TS-4, ADAM-17 (a MUC1 cleavage enzyme), ADAM-TS16, ADAM-19 and ADAM-28. Some or all of these cleavage enzyme may be upregulated to enhance the cleavage of MUC1 to the MUC1* form to induce pluripotency or maintain it (Boyer et al, 2005).

Our previous work with embryonic stem cells, which only express the cleaved form of MUC1, MUC1*, showed that dimerization of its extracellular domain stimulate growth and inhibit differentiation (Hikita et al., 2008). These effects were achieved by dimerizing the MUC1* extracellular domain using either a bivalent anti-MUC1* antibody, recombinant NM23, or a mutant NM23 (S 120G) that preferentially forms dimers (Kim et al., 2003). Inhibition of MUC1* extracellular domain using the monovalent anti-MUC1* Fab was lethal within hours. These findings indicate that MUC1* is a significant factor in maintaining stem cells or causing reversion of cells to a less mature state. In addition, OCT4 and SOX2 bind to the MUC1 gene promoter and also to the promoter of its cleavage enzymes. SOX2 and NANOG bind to the NM23 (NME7) promoter. Since blocking the extracellular domain of MUC1* are lethal to hESCs, it follows that the pluripotency genes, OCT4, SOX2, and NANOG, all induce expression of MUC1, its cleavage enzyme and its activating ligand. One or more of the genes or gene products that have already been shown to induce pluripotency can be replaced by transfecting the gene or introducing the gene product, for MUC1* alone or in addition to its cleavage enzymes and/or activating ligands, NME7, NME1, NME2, NME6 or an antibody that dimerizes the PSMGFR epitope of MUC1 or MUC1*.

As those who are skilled in the art are familiar, nucleic acids that encode the pluripotency genes or the proteins or peptides themselves can be modified with moieties or sequences that enhance their entry into the cell. Similarly, signal sequences can direct the localization of the transfected gene or gene product. Examples of signal sequences are given as SEQ ID NOS:2-4. The invention contemplates the use of gene and protein modifications to any of the pluripotency genes described above to enhance cellular entry of nucleic acids encoding the proteins or the proteins themselves, wherein the proteins include MUC1, MUC1*, NME7, NME1, NME6 and variants thereof. MUC1* is generally described as a truncated form of the transmembrane receptor MUC1, wherein most of the extra cellular domain is not present and the remaining extra cellular domain contains most or all of the PSMGFR sequence. However, MUC1 may be cleaved by different enzymes depending on tissue type or cell type. For example, in stem cells, MUC1 is cleaved to MUC1* by MMP14, MMP16 and ADAM17, whereas in breast cancer T47D cells, MUC1 cleavage is dominated by MMP16 and in DU145 prostate cancer cells it is cleaved by MMP14. Therefore, MUC1* extra cellular domain essentially consists of the PSMGFR sequence, but may be further extended at the N-terminus to comprise additional amino acids. The invention contemplates that the N-terminal domain of MUC1* may be truncated or extended by up to nine (9) amino acids without substantially altering its activity. MUC1* exemplified as SEQ ID NO:5 and variants whose extracellular domain is essentially comprised of the sequences given in SEQ ID NOS: 6, 7, 8 and 9 are preferred.

MUC1, MUC1*, or associated factors, including those listed above, can substitute for one or more of the genes or gene products that induce pluripotency and may be used to induce pluripotency or transition to a less mature state or to maintain that state.

The invention contemplates using any mature cell, including without limitation, somatic cells, which include without limitation, fibroblasts, dermablasts, blood cells, hematopoietic progenitors, nerve cells and their precursors and virtually any kind of cell that is more differentiated than a pluripotent stem cell. In one case, somatic cells such as fibroblasts,dermal fibroblasts, blood cells or nerve cells are transfected with a gene that encodes the MUC1 protein, which aids in inducing stem cell-like features and in some cases induces progeny to become pluripotent stem cells. In another aspect of the disclosure, a gene for MUC1* is transfected into cells to induce a reversion to a less mature or stem cell-like state and in some cases induce generation of actual pluripotent stem cells. Each of the MUC1 or MUC1* genes may be introduced to the cell alone or in combination with other genes that aid in inducing pluripotency or stem cell-like characteristics. For example, DNA encoding MUC1 or preferentially MUC1* is introduced to the cell along with one or more of the genes that encode OCT4, SOX2, NANOG, LIN28, KLF4, and/or c-MYC. DNA encoding a truncated form of MUC1, preferentially MUC1*, is transfected into fibroblasts along with one or more of the genes encoding OCT4, SOX2, NANOG, and LIN28 (Yu et al., 2007). In another case, DNA encoding a truncated form of MUC1, preferentially MUC1*, is transfected into somatic cells, fibroblasts, or other cells, along with genes encoding OCT4, SOX2, KLF4, and c-Myc (Takahashi et al., 2007). Similarly, DNA encoding MUC1* and/or its activating ligand, NM23 is transfected into cells to induce reversion to a less mature state. In a preferred embodiment, the NM23 family member is NME1 or the S120G mutant of NME1 that prefers dimer formation, NME6 in dimer form, or NME7. In a preferred embodiment, the NME family member is added to the cell culture medium. In a more preferred embodiment, the NME family member is NME7 which may optionally consist of subunits A and B, devoid of the "M" leader sequence: NME7-AB (SEQ ID NOS: 34-35) MUC1* and/or NM23 may be introduced to cells along with other genes such as OCT4, SOX2, NANOG, LIN28, KLF4, and/or c-MYC to induce pluripotency or stem cell-like characteristics. DNA encoding antibodies that recognize MUC1* or MUC1 may also be transfected into cells alone or with other genes to induce stem cell characteristics in the cells or their progeny. If secreted, anti-MUC1* antibodies will dimerize and thus activate the MUC1* receptor, which will function to promote or maintain stem-like characteristics. Alternatively, an anti-MUC1* antibody is exogenously added to cells undergoing induction to a less mature state. In a preferred case, the MUC1* antibody is attached to a surface upon which cells are attached.

Similarly, factors such as nucleic acids, proteins, modified proteins or small molecules that affect the expression of MUC1, MUC1* or their associated factors are introduced to cells to induce characteristics of stem cells or to induce a return to pluripotency. For example, genes or gene products for MUC1 cleavage enzymes, MMP14, MMP16, MMP2, MMP9, MMP10, ADAM TSL-1, ADAM TS-4 ADAM-17 (a MUC1 cleavage enzyme), ADAM-TS16, ADAM-19 and ADAM-28 are introduced to cells to induce pluripotency or similar characteristics.

In case, non-protein agents are added to cells to induce or enhance the induction of pluripotency. For example the phorbol ester phorbol 12-myristate 13-acetate (PMA) is a small molecule that increases the cleavage of MUC1 to MUC1* (Thathiah et al., 2003). In one aspect of the disclosure, phorbol ester is added to cells undergoing conversion to pluripotency to induce or increase the efficiency of iPS generation.

In another example, ligands that interact with MUC1 or MUC1* are added to somatic cells, dermal fibroblasts, fibroblasts, or somewhat differentiated cells to induce pluripotency either alone or in combination with other genes to induce or maintain stem-like features or pluripotency. For example, one or more of the genes encoding OCT4, SOX2, NANOG, LIN28, KLF4, and/or c-MYC are transfected into fibroblasts or other cells and then are cultured in the presence of ligands that activate MUC1 or MUC1*. Dimeric, protein ligands of MUC1* are preferred. In a preferred case, a bivalent anti-MUC1* antibody is added to cells that have been transfected with genes that influence cells or their progeny to become pluripotent stem cells.

In a preferred embodiment, NME7 or NME7-AB is introduced to cells, as the gene that encodes it, as the protein itself or as a protein bearing a leader sequence such as a poly-arginine tract, to facilitate entry into the cell, to aid in the induction or maintenance of pluripotency. The inventors recently showed that when NM23 is secreted by pluripotent stem cells (and cancer cells), it is an activating ligand of the cleaved form of MUC1 - MUC1* - and triggers the MAP kinase proliferation pathway. NM23 stimulation of MUC1* was shown to promote the growth of pluripotent hESCs and inhibited their differentiation (Hikita et al., 2008). NM23 also induces the transcription of c-Myc (Dexheimer at al., 2009) and replaces the need for c-MYC. NM23 is added exogenously either in its native state to activate the MUC1* growth factor receptor or with a poly arginine tract to facilitate entry into the cell and nucleus where it induces c-MYC expression. NM23 (NME) may be added as the encoding nucleic acid, or as the expressed protein with or without a modification that facilitates entry into the cell. NME-H1 or -H6 can be used in their native state or in mutant forms that favor the dimeric state, such as the S 120G mutation. NME7 is used as the monomeric protein, optionally as a human recombinant protein that is expressed from a construct that encodes the A and B domains but is devoid of the M leader sequence, which we call NME7-AB (SEQ ID NOS:34-35).

In another aspect of the disclosure, a bivalent antibody that binds to the extracellular domain of MUC1* (PSMGFR) or a dimeric MUC1* ligand, such as NM23, or genes encoding them are added to MUC1 *-expressing cells to induce pluripotency, increase the efficiency of the induction of pluripotency, to maintain pluripotency or to inhibit differentiation. The cells to which these MUC1 or MUC1* interacting proteins are added may be naturally occurring cells or those into which genes to induce stem cell-like characteristics have been added, or have already entered the differentiation process or may be stem cells.

Genes for inducing pluripotency may be introduced on the same or different plasmids, which may be lenti viral vector driven or adenovirus vectors or any integrating or non-integrating viral or non-viral vector, or any other system that facilitates introduction of these genes into the desired cells.

In many cases, it is preferential to achieve the effects of pluripotency-inducing proteins by introducing the proteins themselves rather than the nucleic acids or genes that encode them. The disclosure encompasses genes disclosed herein for the induction of stem-like characteristics or pluripotency that can be replaced by the gene products, the proteins, either in their native state or modified with leader sequences such as poly-arginine tracts to allow entry into the cells. The products of these genes, i.e. proteins, or other proteins which interact with one or more of the products of the transfected genes are introduced to cells to induce or maintain pluripotency or other stem-cell like characteristics.

In other cases, it may be beneficial to introduce synthetic agents, such as small molecules, to induce stem-ness in mature or differentiated cells (Lyssiotis et al. 2009). In one aspect of the disclosure, small molecules are added to cells that either directly or indirectly induce the transcription of genes that induce pluripotency. In other cases, small molecules that directly or indirectly increase the production of MUC1* are added. In one instance, these small molecules increase cleavage of MUC1 to the MUC1* form, which is a characteristic of stem cells. Phorbol ester, for example, is a small molecule that increases cleavage of MUC1 to MUC1*, so when added to cells, it promotes induction or maintenance of pluripotent state.

### Use of P53 inhibitor

P53, which is also known as a tumor suppressor, promotes apoptosis. It would therefore be advantageous to inhibit p53 when culturing stem cells or inducing pluripotency in somatic or other cells. The present disclosure anticipates using p53 suppressors along with other reagents and methods as described herein to maintain stem-ness or induce stem-like or pluripotent characteristics. P53 can be suppressed by a number of methods. Small molecules such as Pifithrin-µ inhibits the pro-apoptotic effects of p53 (Strom, et al., 2006 Sep; Komarov, et al., 1999) and thus are optionally added to cells to increase efficiency of induction of pluripotency or to maintain stem-ness. In a preferred case p53 inhibitors are used along with genes or gene products that up-regulate MUC1 or MUC1*, including but not limited to the MUC1 or MUC1* genes or gene products, their activating ligands and their cleavage enzymes.

Another method for suppressing p53 activity to increase the efficiency of inducing pluripotency or maintaining stem-ness is by the introduction of the MUC1* protein to cell cultures. The MUC1* protein or portions thereof, such as the cytoplasmic domain alone, can be modified by adding on a poly-arginine tract to facilitate entry into the cell. It has been reported that the overexpression of the cytoplasmic tail, alone, of MUC1 (MUC1-CD) resulted in its translocation to the nucleus where it was found to bind to the p53 promoter. These studies could not determine whether MUC1-CD down or up-regulated p53. The present disclosure is also drawn to the repression of p53 by the ectopic expression of MUC1*, to increase the efficiency of inducing pluripotency or other stem-like characteristics. MUC1* can be introduced by inserting the gene into the cell, by adding the protein itself exogenously or by adding the MUC1* protein that is optionally modified with a poly-arginine tract.

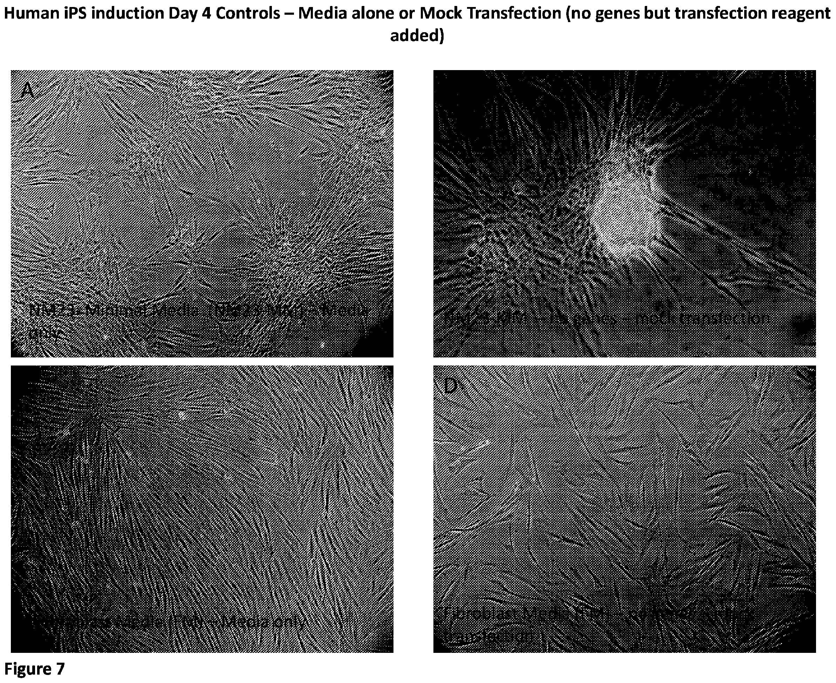

In one aspect herein a MUC1* ligand is added into cell culture media; cells, which may be somatic, differentiated or somewhat differentiated are contacted with the media over the course of several days to several weeks until cells have reverted to the desired state which is a less mature state than the starting cells. In a preferred embodiment, the MUC1* ligand is NME1 in dimeric form. In a more preferred embodiment, the MUC1* ligand is monomeric NME7, which may be devoid of the "M" leader sequence (NME7-AB). Contacting cells with a MUC1* ligand alone is sufficient to make cells revert to a less mature state. In a preferred embodiment, cells to be reverted to a less mature state are contacted by two different types of MUC1* ligand: one that enables attachment of the cells to a surface, such as an anti-MUC1* antibody, and the other a ligand free in solution or media, such as dimeric NM23-H1 or NME7. Optionally a rho kinase inhibitor can also be added to the cell culture media. Evidence of cells reverting to a less mature state by contacting the cells with a MUC1* ligand can be seen in Figure 7 A,B, Figure 9, Figure 13, Figure 15, Figure 17 and Figure 26.

### NME causes expression of MUC1 and MUC1*

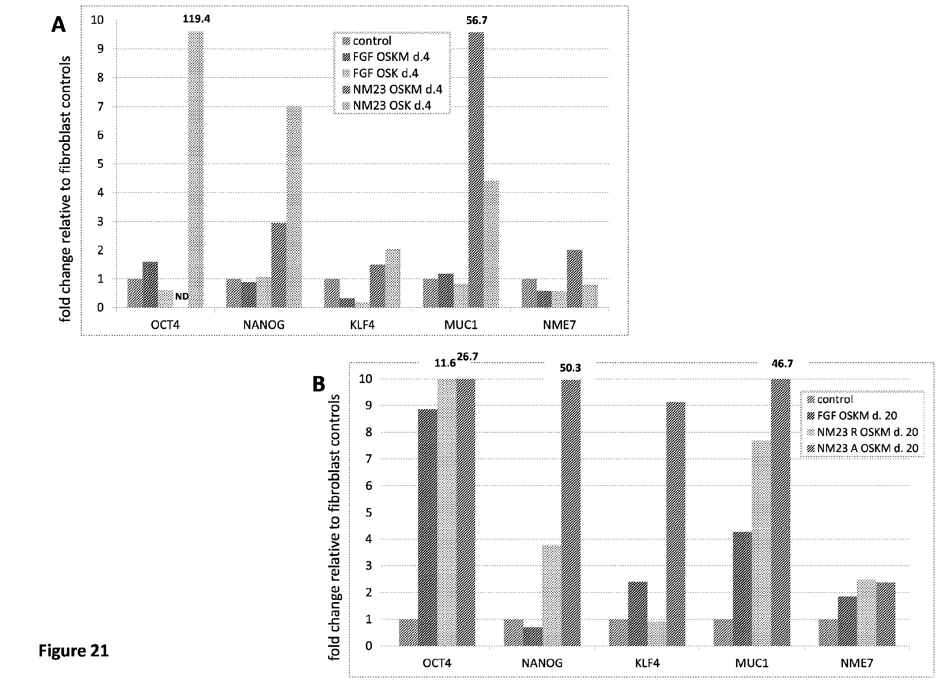

Culturing cells in NM23-H1 dimers or NME7 causes expression of MUC1 and MUC1* in particular to be increased. Increased expression or activity of MUC1 or MUC1* makes cells revert to a less mature state, which can be a pluripotent state. Evidence of this is documented by detecting a concomitant increase in markers of the pluripotent stem cell state, such as OCT4, SSEA4, Tra 1-60, REX-1, NANOG, KLF4 and others known to those skilled in the art. RT-PCR measurements show that cells cultured in NM23 media have increased expression of MUC1; because PCR measures the RNA transcript, it cannot tell whether or not the protein will be post-translationally modified, such as cleaved to produce MUC1*. However, immunocytochemistry experiments, clearly show that culturing cells or contacting cells with NM23-H1 dimers or with NME7 causes a dramatic increase in the amount of MUC1* expressed. For example, when human fibroblasts were transfected with three (3) or four (4) of the pluripotency inducing genes, also called the "Yamanaka factors" (Oct4, Sox2, Klf4 and c-Myc) and cultured either by the standard method in FGF media or in NM23 media (dimeric form of NM23-H1) then assayed by RT-PCR to quantify expression levels of pluripotency markers as well as MUC1 and MUC1* ligand, NME7, it was observed that as the cells increased expression of the pluripotency markers, there was an associated increase in the expression of MUC1 and the MUC1* ligand NME7. A representative graph of several RT-PCR experiments that showed this effect can be seen in Figure 21 and is detailed in Example 11. In addition, immunocytochemistry experiments were performed to assay for the presence of MUC1* as well as pluripotency markers. Experiments showed that contacting cells with a MUC1* ligand, such as NM23-H1 dimers or NME7 caused an increase in the expression of MUC1*, accompanied by an increase in the expression of some of the pluripotency markers, such as Tra 1-60. Representative experimental data are shown in Figures 40 - 44.

In another aspect of the disclosure, cells are reverted to a less mature state and even further to a pluripotent state by contacting the cells with a MUC1* ligand, such as NM23-H1 dimers, NME7, NME7-AB and/or an anti-MUC1* antibody, while also being contacted with other biological or chemical agents that induce pluripotency. In a preferred embodiment, the agents that induce pluripotency are the genes or nucleic acids that encode them, or the proteins themselves, selected from the group comprising OCT4, SOX2, KLF4, c-MYC, NANOG and LIN28. It is known that ectopic expression of two or more of the pluripotency genes selected from the group above will cause cells to revert to the pluripotent state. The state of the art for inducing pluripotency in a more mature cell is to cause the cells to express one or more of the pluripotency genes, while in culture in a medium containing bFGF and sometimes bFGF and TGF-beta. The efficiency of inducing pluripotency (making induced pluripotent stem (iPS) cells) is very low.

We demonstrated that substituting NM23 media for bFGF media vastly improves the efficiency of inducing pluripotency. In addition, the use of feeder cells can be substituted for a layer of extra cellular matrix proteins, or fragments thereof, or a layer of a MUC1* ligand. In a preferred embodiment, cells undergoing induction to a less mature state are plated over a layer of an anti-MUC1* antibody that recognizes MUC1* on stem cells. Table 2 (see Drawings section) shows that substitution of bFGF for NM23 dimers in the media resulted in as much as a 100-fold increase in the efficiency of iPS generation wherein efficiency is calculated by the number colonies generated with stem-like morphology divided by the number of cells required to produce that number of colonies, which is also referred to as an induction rate.

In yet another aspect of the disclosure, cells are reverted to a less mature or pluripotent state by contacting the cells with a biological or chemical agent that increase expression of MUC1 or MUC1*. Cells transfected with pluripotency genes OCT4, SOX2, KLF4 and c-MYC and cultured in fibroblast serum-containing media then FGF media as is the standard practice for making iPS cells, causes an increase in MUC1 expression that coincides with the expected increases in expression of pluripotency markers such as OCT4, Tra 1-60 and the like. An even greater increase in MUC1 expression is obtained when pluripotency genes are caused to be expressed and cells are contacted with a MUC1* ligand in a media or attached to a surface. However, the disclosure contemplates culturing cells in suspension or on other surfaces including surfaces coated with extra cellular matrix proteins, fragments of ECM proteins such a fibronectin fragments, vitronectin, feeder cells, cancer cells and the like. Figure 21 shows one such example, with a 4.3-fold increase in MUC1 expression by Day 20 when fibroblasts were transfected with OCT4, SOX2, KLF4 and c-MYC and cultured in FGF media according to the standard method for making iPS cells. This shows that as cells transition to a less mature state, expression of MUC1 increases. Culturing the same cells in media containing a MUC1* ligand, such as NM23 dimers or NME7 causes a an approximate 10-100-fold increase in MUC1 expression by Day 20 (Figure 21). The greatest increases in the expression of the pluripotency genes resulted from cells that were cultured in NM23 dimer media. This result shows that contacting cells with a MUC1* ligand induces cells to revert to a less mature state above and beyond the actions of the transfected pluripotency genes.

Human fibroblasts were subjected to the standard method for inducing pluripotency, wherein one or more of the genes encoding the Yamanaka factors OCT4, SOX2, KLF4 and c-MYC were used. However, to assess methods described herein for their ability to increase the efficiency of iPS generation or to induce pluripotency on their own, we used a culture medium that contained NM23 dimers or NME7 instead of the standard bFGF. In one condition of the experiment, no genes were transfected, but the fibroblasts were cultured in a serum-free media with NM23-S120G in dimeric form or NME7 as the only exogenously added growth factor. Some or all of the pluripotency genes were transfected in another arm of the experiment. Another variable of the experiment was that NM23 media was introduced either from the onset of the experiment or at Day 7, when according to the standard protocol, fibroblast medium (FM) would be exchanged for a serum-free medium containing 4 ng/mL of bFGF. At this timepoint, according to the standard protocol, the cells would be moved to fibroblast feeder cells. This was done, but in addition, NM23 cultured cells were moved to a plastic culture plate that had been coated with an mouse monoclonal anti-MUC1* antibody called MN-C3 that the inventors developed for attaching human stem cells to surfaces. "MN-C3" (short hand "C3") and "MN-C8" (short hand "C8") are mouse monoclonal antibodies developed by the inventors to specifically bind to MUC1* as it appears on human stem cells. When surfaces are coated with either of these antibodies, it enables human stem cell adhesion, whereas pluripotent human stem cells are non-adherent cells.

Resultant cells were characterized by photographs, RT-PCR quantification of the pluripotency genes, immunocytochemistry and FACS to assess the presence of pluripotency markers; characterization was performed on cells taken between Day 4 and Day 30.

Fibroblasts cultured in either NM23 (dimers) in serum-free media without any genes transfected revert to a less mature state as evidenced by a dramatic change in their morphology, going from fibroblast morphology to a stem cell-like morphology within days. By Day 20, there were no visible differences between the mock transfection cells and actual pluripotent stem cells. Measurement of pluripotency markers indicated that the cells expressed increased levels of pluripotency markers. Mock transfectants cultured in FGF media showed no changes in morphology or in the measurement of pluripotency markers. Cells transfected with some or all of the pluripotency genes Oct4, Sox2, Klf4, c-Myc, Lin28, or Nanog, that were cultured in NM23-S120G dimers consistently expressed pluripotency markers before comparable cells cultured in FGF media, and had a much higher efficiency of inducing pluripotency than the standard FGF method. Several experiments were performed and representative data are described below.

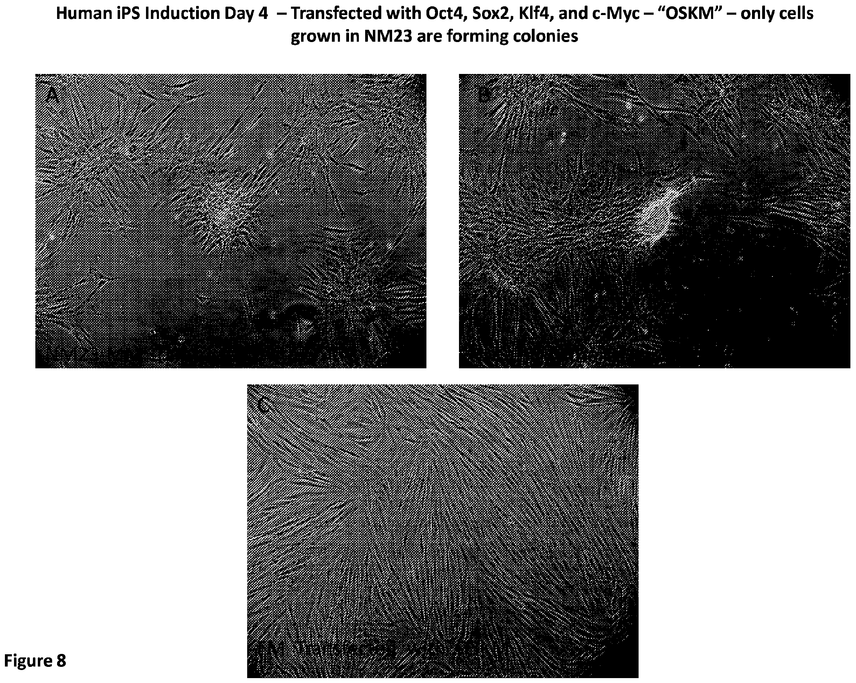

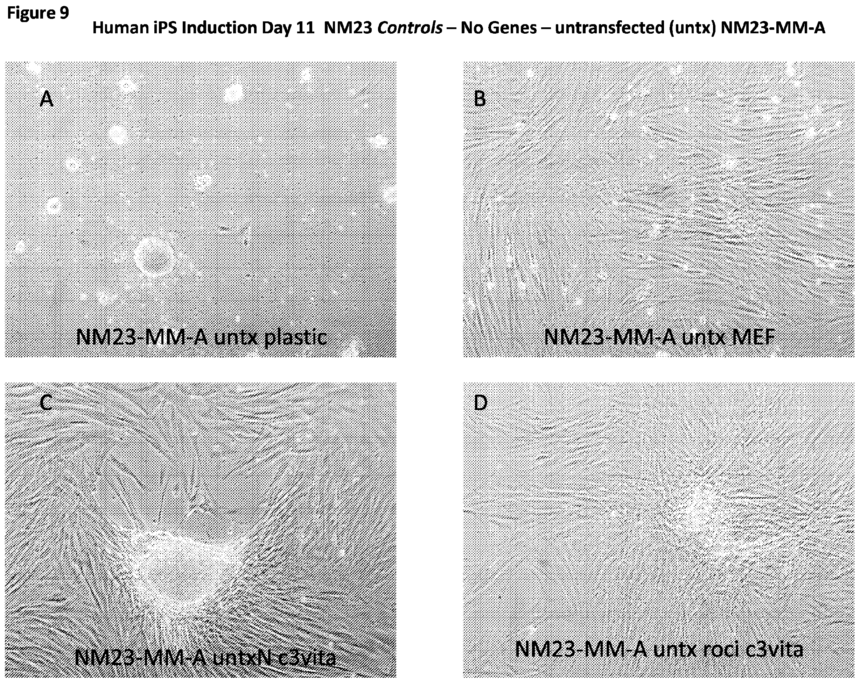

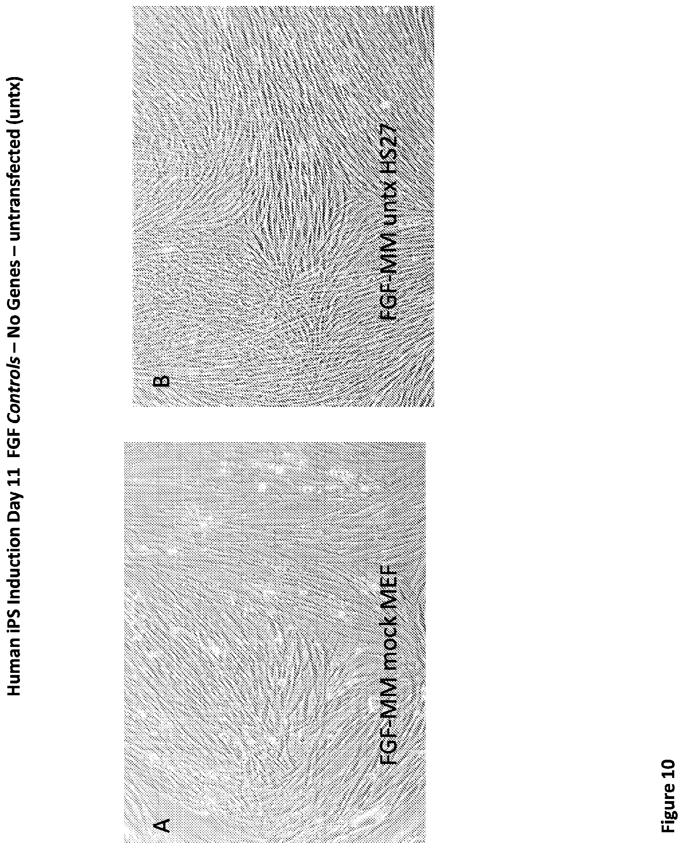

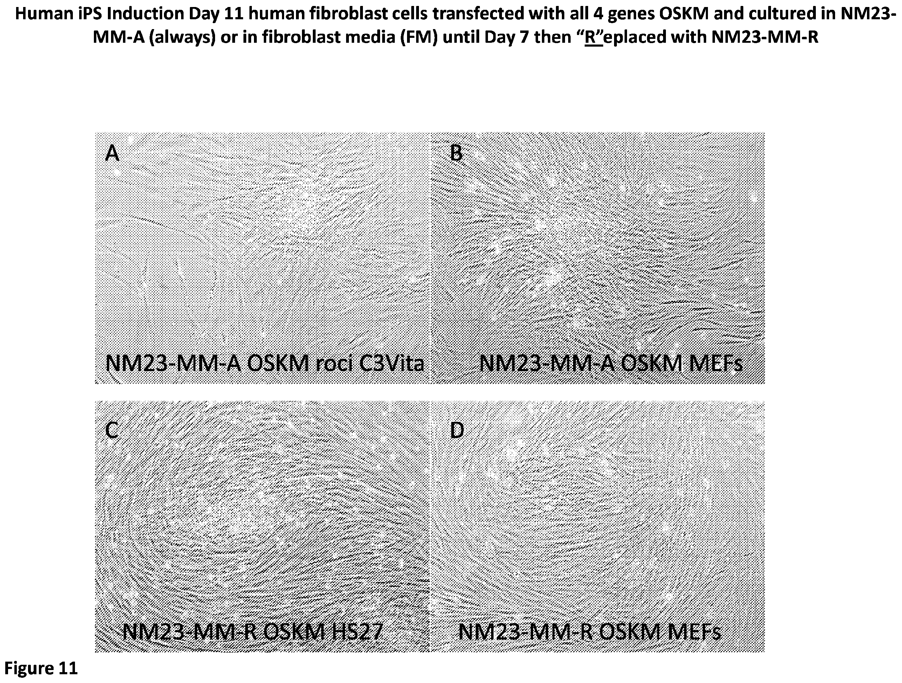

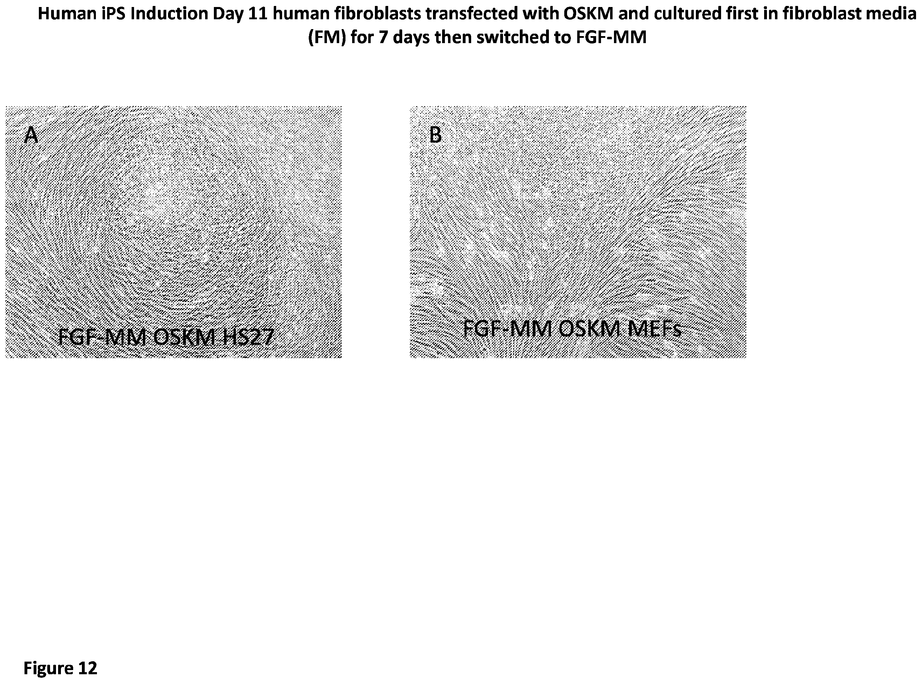

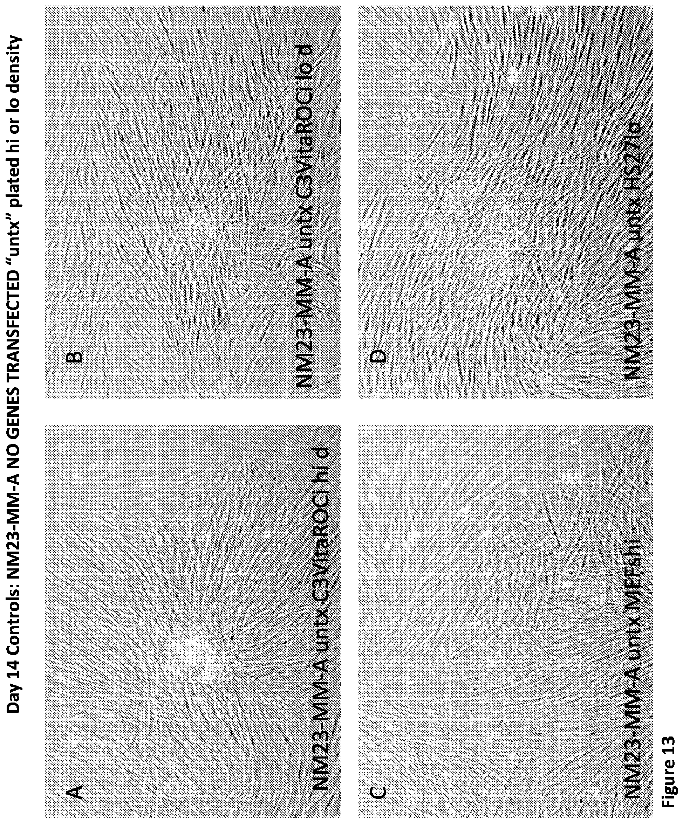

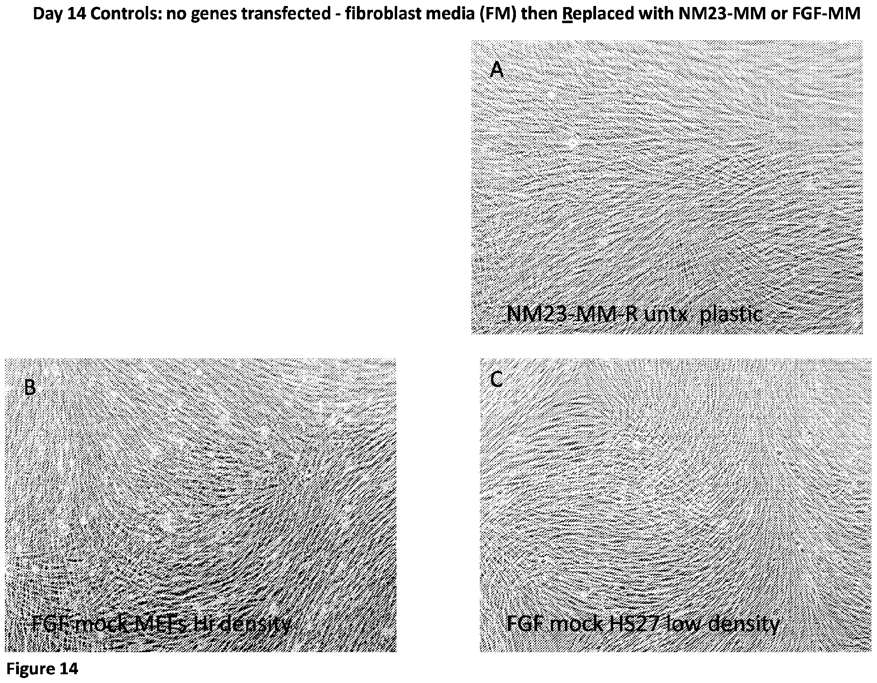

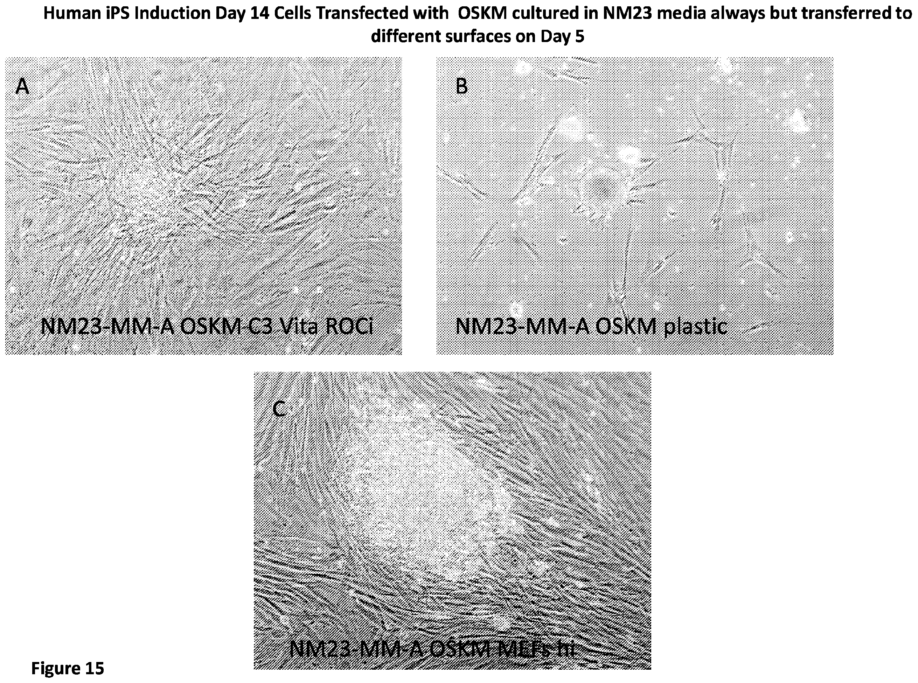

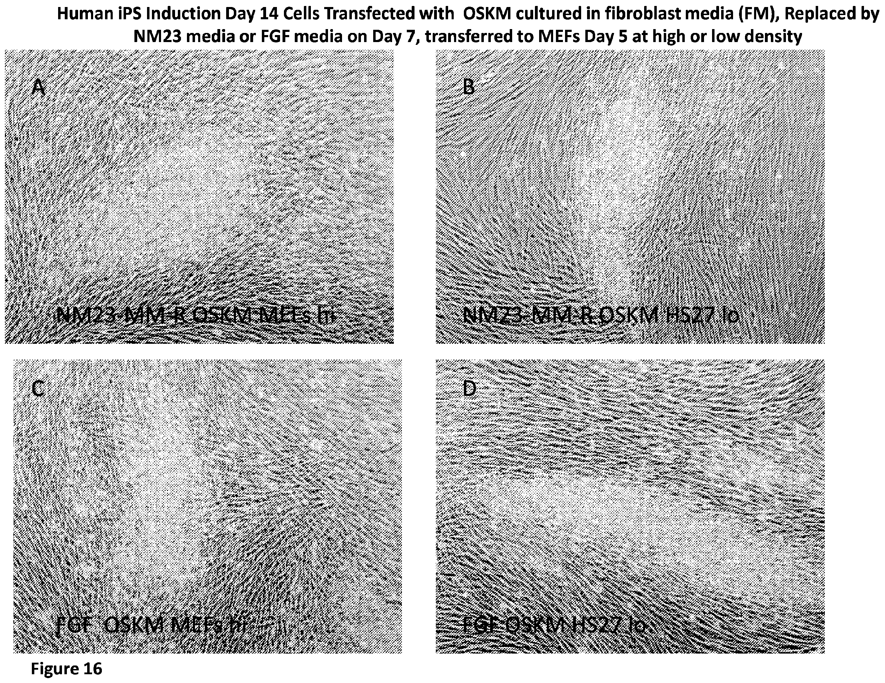

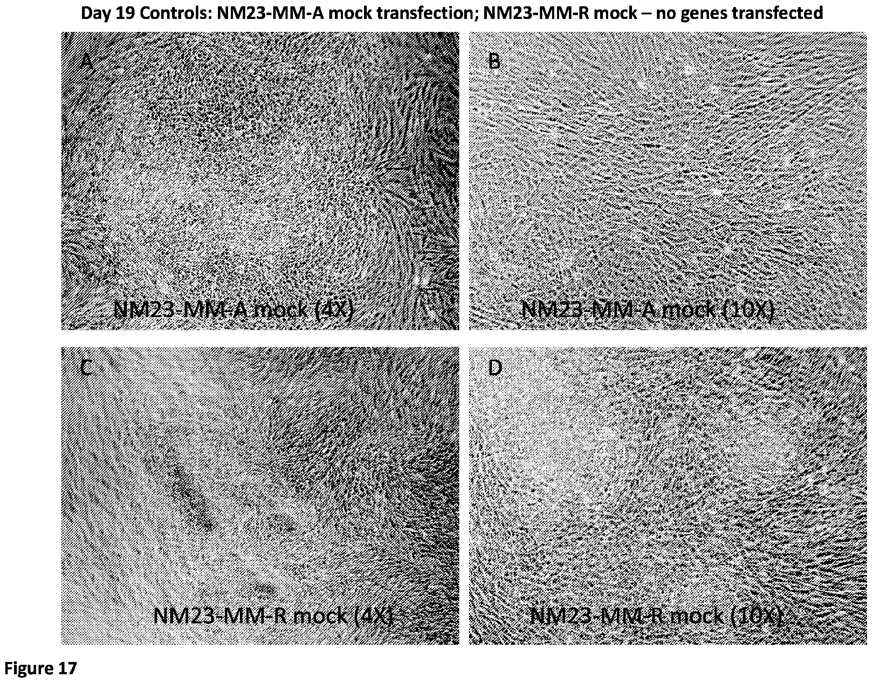

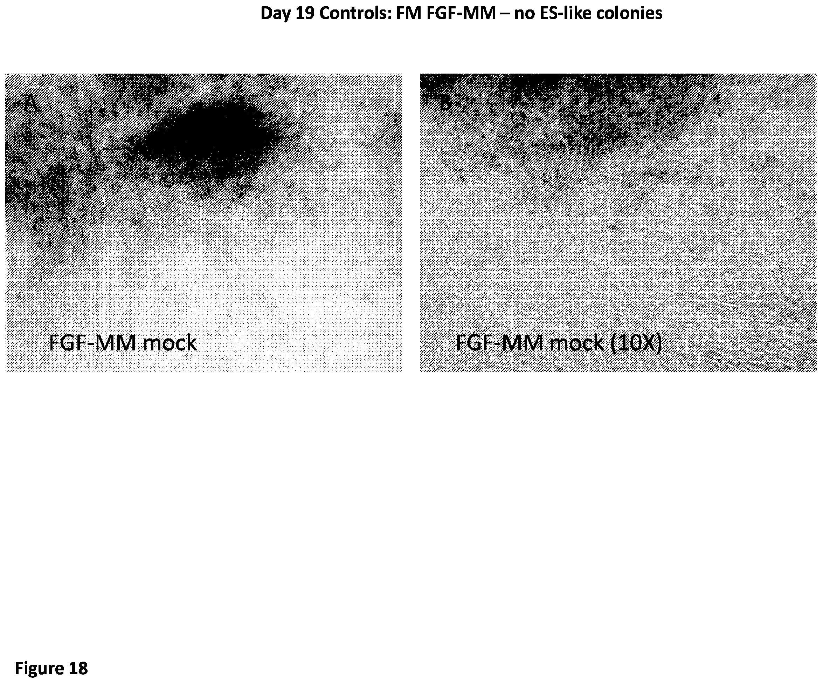

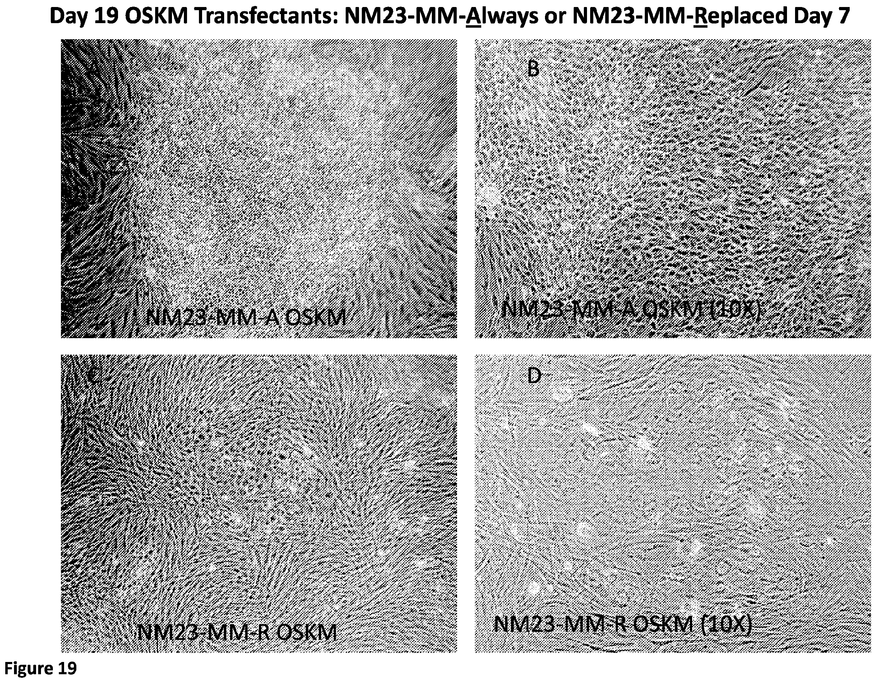

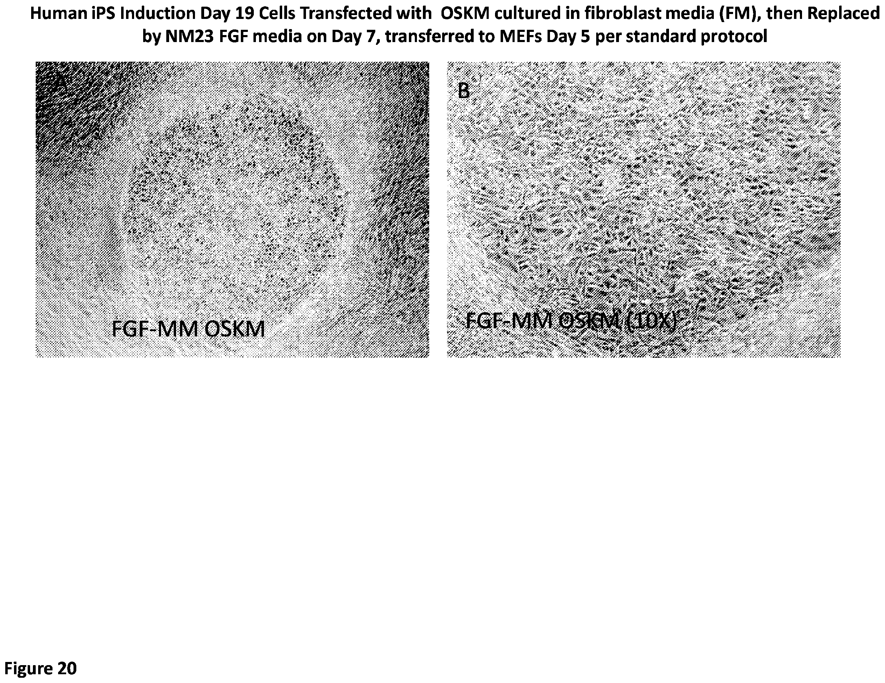

Figure 7 shows photographs taken Day 4 of fibroblasts cultured in either NM23 (dimers) in serum-free media without any genes transfected(A) or in a mock transfection in which reagents were added, but no genes (B); Figure 7 panels C and D show the corresponding cells cultured in fibroblast media with no transfection or a mock transfection. Note that the fibroblasts cultured in the NM23 media by Day 4 are changing so that they do not look like fibroblasts and are moving into colony-like clusters, but the fibroblasts cultured in serum-containing fibroblast media without NM23 are not. Figure 8 shows that also on Day 4, fibroblasts that were transfected with all four Yamanaka genes, Oct4, Sox2, Klf4 and c-Myc (OSKM) showed even more striking changes in morphology with cluster and colony-like morphology if and only if they were cultured in NM23 media (A and B) but not if they were cultured in fibroblast media (C), which is the standard protocol. According to the standard procedure, cells are moved off of plastic plates and onto inactivated human or mouse feeder cells on Day 5, then switched from fibroblast media to standard bFGF media two days later which is Day 7. Figure 9 shows photos taken on Day 11, of cells that had not been transfected with any genes, but had been cultured in the NM23 serum-free media, and left on uncoated plastic (A), were moved to inactivated mouse feeder cells (MEFs) (B), transferred to plastic coated with anti-MUC1* MN-C3 antibody (C) or transferred to the antibody coated surface but also wherein a rho kinase inhibitor was added to the culture media (D). As can be seen in the photos, clusters and colonies of cells have floated off the plastic surface, consistent with the idea that they had become stem-like because stem cells are non-adherent whereas fibroblasts adhere quite well to plastic. Cells that had been transferred to feeder cells lost their stem-like morphology. But cells that were not transfected with any genes, but cultured in NM23 media and moved to a surface (Vita^{™} plate) coated with an anti-MUC1* antibody (Figure 9, C, D) remained attached to the surface and appear as stem-like colonies. These results are consistent with the idea that culturing the cells in the presence of NM23 increased expression of the cleaved form of MUC1, causing the cells to adhere to a surface coated with the cognate antibody. By Day 11, the corresponding cells that were not transfected with genes but were cultured in fibroblast media, then transferred to inactivated feeder cells and switched to media containing bFGF, show no signs of stem-like morphology (Figure 10) whether transferred to mouse feeder cells (A) or human feeder cells (B). On Day 11, photos were also taken (Figure 11) of the cells that had been transfected with all four of the pluripotency genes, OSKM, cultured in NM23 media from the start of the experiment (labeled A for always) (panels A and B) or cultured in fibroblast media until Day 7 then switched to the NM23 media (labeled R for replaced) (panels C and D). The figure shows stem-like colony formation for OSKM transfected cells cultured in NM23 and then transferred to an anti-MUC1* antibody surface (A) and when the cells were transferred to a surface of human feeder cells (C). Figure 12 reflects this same advantage for cells transferred onto human feeder cells. Figure 12 Ashows stem-like morphology for OSKM transfected cells cultured in bFGF media and transferred Day5 onto human HS27 feeder cells but not so much for cells transferred to mouse feeder cells (B). Figure 13 shows that cells that were not transfected and cultured in NM23 media, which were then transferred to plastic coated with the MN-C3 antibody have stem-like colonies developing more when cells were plated at high density (A) than low density (B), no colonies were visible after cells were transferred to mouse feeder cells (C) but small stem-like colonies were visible for cells transferred to human feeder cells (D). No stem-like colonies appeared for untransfected cells that were cultured in fibroblast media then switched to NM23 media on Day 7 and plated onto uncoated plastic (Fig. 14 A) or for untransfected cells cultured in bFGF media whether transferred to mouse feeder cells (B) or human feeder cells (C). Figure 15 shows that cells that were transfected with all four pluripotency genes, OSKM, and cultured in NM23 from the start, formed large stem-like colonies when plated onto plastic coated with anti-MUC1* antibody MN-C3 (panel A) but not for the same cells plated onto uncoated plastic (B). However, large stem-like colonies did appear by Day 14 when cells were transferred to feeder cells (Figure 15 C; Figure 16 A-D), wherein cells were cultured in NM23 media (A,B) or in bFGF media (C,D). Figure 17 shows that even in the absence of any ectopically expressed genes, NM23 induced somatic cells to revert to a stem-like state by Day 19. 10X magnification shows complete loss of fibroblast morphology for cells cultured continuously in NM23 (B) and displaying the characteristic cobblestone pattern of stem cells also having a large nucleus to cytoplasm ratio. No such transition to a less mature state could be observed for mock transfections wherein cells were cultured in bFGF media (Fig. 18 A,B). Comparison of continuous culture in NM23 media or replacing fibroblast media with NM23 media at Day 7 (Figure 19), shows that cells transfected with OSKM reverted to the most stem-like state when cultured in NM23 media continuously (A,B). By Day 19, cells transfected with OSKM but cultured in bFGF media and on feeder cells after Day 5, also showed formation of stem-like colonies (Fig. 20 A,B).

RT-PCR (real time PCR) was also performed at various timepoints for the cells pictured in the figures described above in order to quantify expression levels of key pluripotency genes, such as OCT4, NANOG, KLF4, and sometimes SOX2. Human fibroblasts were transfected with either three (3) or four (4) of the pluripotency genes Oct4, Sox2, Klf4 and c-Myc. It can be seen that by Day 4 post transfection cells cultured in NM23-H1 dimers, which are also a MUC1* ligand that dimerizes the MUC1* extra cellular domain, expressed increased amounts of the pluripotency markers OCT4, NANOG and KLF4, whereas the same cells cultured in fibroblast media showed only a modest increase in OCT4 by Day 4. Therefore, it is concluded that MUC1* ligand NM23 induces pluripotency or reverts the cells to a less mature state over and above that which is due to transfection of the pluripotency genes alone. At the same time, cells induced to revert to a less mature state also have increased expression of MUC1 and NME7, a MUC1* ligand. Recall that RT-PCR detects the nucleic acid so that this assay cannot differentiate MUC1 from MUC1*, since MUC1 is post-translationally cleaved to yield MUC1*. By Day 20, the cells undergoing the standard method for inducing pluripotency wherein FGF media replaces fibroblast media (serum-containing) on Day 7, show increased expression of pluripotency markers OCT4, NANOG and KLF4 as well as a dramatic increase in the expression of MUC1 and NME7.

These results show that the pluripotency genes Oct4, Sox2, Klf4 and c-Myc induce expression of MUC1 and a MUC1* ligand. From our earlier work (Hikita et al) and Figure 2 of the present application, we know that on pluripotent stem cells, essentially all of MUC1 is cleaved to the MUC1* form. Thus, the Yamanaka pluripotency genes Oct4, Sox2, Klf4 and c-Myc induce expression of MUC1*. Conditions wherein the transfectants were cultured in NM23 dimers had the highest amounts of the pluripotency markers OCT4, NANOG, KLF4 as well as the highest amounts of MUC1 and NME7. Additionally, these data strongly argue that MUC1 and particularly MUC1* is a pluripotency marker. The RT-PCR experiments were performed several times. For each experiment, there were three (3) replicate measurements for each of three (3) separate samples per condition. GAPDH was the internal control and data is plotted as fold-change, normalized to the control, untransfected human fibroblasts cultured in fibroblast media. Exemplary experiments are described in Example 12 and Examples 14-15 and shown in Figures 21, 22, and Figures 35-44.

Transfectants cultured in fibroblast media showed less than a 2-fold increase in expression of Oct4, Nanog and Klf4 by Day 4, whereas cells transfected with OSK and cultured in NM23 media showed significant increases in the expression of the key pluripotency genes. OCT4 expression increased by 70-fold higher than cells transfected with OSKM but cultured in fibroblast media. NANOG increased 7-fold, and KLF4 increased 4.5-fold over the same cells transfected with all four pluripotency genes but cultured in fibroblast media. Importantly, we note that contacting cells with the MUC1* ligand, NM23 caused a 4.5 increase in the expression of MUC1. Figure 21 shows that contacting cells with nucleic acids that cause OCT4, SOX2, KLF4 and c-MYC to be expressed, also increase the expression of MUC1. Cells transfected with OSKM and cultured in fibroblast media to Day 7, then switched to bFGF-containing media increase MUC1 expression 4.3-fold by Day 20, compared to 7.8-fold if the cells were cultured in the MUC1 ligand, dimeric NM23-H1, in this case. The same results were also obtained when the cells were cultured in NME7 wherein NME7 was used in monomeric form.

MUC1* ligands induce pluripotency and expression of MUC1*. In the induction of pluripotency experiments described above, it was observed that whenever there was an increase in the expression of pluripotency markers, there was an associated increase in the expression of MUC1, MUC1 * and MUC1 * ligand NME7. Figure 21, which shows graphs of RT-PCR experiments performed on Day 4 (A) or Day 20 (B), illustrates this point. Human fibroblasts were transfected with either three (3) or four (4) of the pluripotency genes Oct4, Sox2, Klf4 and c-Myc. It can be seen that by Day 4 post transfection cells cultured in NM23-H1 dimers, which are also a MUC1* ligand that dimerizes the MUC1* extra cellular domain, expressed increased amounts of the pluripotency markers OCT4, NANOG and KLF4, whereas the same cells cultured in fibroblast media showed only a modest increase in OCT4 by Day 4. Therefore, it is concluded that MUC1* ligand NM23 induces pluripotency or reverts the cells to a less mature state over and above that which us due to transfection of the pluripotency genes alone. At the same time, note that the cells induced to revert to a less mature state also have increased expression of MUC1 and NME7, a MUC1* ligand (Figure 21 A). Recall that RT-PCR detects the nucleic acid so that this assay cannot differentiate MUC1 from MUC1*, since MUC1 is post-translationally cleaved to yield MUC1*. By Day 20 (Figure 21 B), the cells undergoing the standard method for inducing pluripotency wherein FGF media replaces fibroblast media (serum-containing) on Day 7, show increased expression of pluripotency markers OCT4, NANOG and KLF4 as well as a dramatic increase in the expression of MUC1 and an approximate 2-fold increase in the expression of NME7. These data show that the pluripotency genes Oct4, Sox2, Klf4 and c-Myc induce expression of MUC1 and a MUC1* ligand. From our earlier work (Hikita et al) and Figure 2 of the present disclosure, we know that on pluripotent stem cells, essentially all the MUC1 is cleaved to the MUC1* form. Thus, the pluripotency genes Oct4, Sox2, Klf4 and c-Myc induce expression of MUC1*. Conditions wherein the transfectants were cultured in NM23 dimers had the highest amounts of the pluripotency markers OCT4, NANOG, KLF4 as well as the highest amounts of MUC1 and NME7. Additionally, these data strongly argue that MUC1 and particularly MUC1* is a pluripotency marker. The RT-PCR experiments were performed several times. For each experiment, there were 3 replicate measurements for each of three (3) separate samples per condition. GAPDH was the internal control and data is plotted as fold-change, normalized to the control, untransfected human fibroblasts cultured in fibroblast media.

Immunocytochemistry experiments were performed so that MUC1* could be measured directly. iPS and ES cells that were previously cultured in FGF over a layer of mouse feeder cells (MEFs) were switched to culture in a serum-free media containing either NME7 or NM23 dimers as the single growth factor; no other growth factors or cytokines were added. Subsequent analysis by immunocytochemistry of pluripotency markers as well as MUC1* showed that human embryonic stem (hES) and induced pluripotent stem (hiPS) that had been cultured long term in FGF media, which drives human pluripotent stem cells from the naive state to the primed state (Hanna et al. a, 2010 and Hanna et al. b, 2010)), showed minimal expression of MUC1* that was dramatically increased after being cultured in either NM23 dimer media or NME7 (Figures 40-44, Example 15). Culturing stem cells in MUC1* ligand, such as dimeric NM23, induced pluripotent human stem cells to revert from the primed state to the less mature naive state (Smagghe et al, Figure 6). Culturing the cells in MUC1* ligand NME7 or NM23, in dimer form, the pluripotent stem cells results in higher expression of naive state markers and lower expression of the primed state markers. In addition, immunocytochemistry experiments showed that female human stem cells cultured in NM23 dimers reverted to a less mature state, in fact a more pluripotent state, characterized by both X chromosomes being in the active state. Subsequent exposure of the naive state stem cells to FGF media caused them to leave the less mature naive state and enter the more differentiated primed state (Smagghe et al, Figure 7). Thus, culturing cells in media that contains a MUC1* ligand such as NM23 dimers or NME7, causes cells to revert to a less mature state.

Consistent results were obtained that were essentially that: 1) culturing cells in NM23-H1 dimers or in NME7 increased the efficiency of iPS generation wherein two or more of the pluripotency genes were ectopically expressed; 2) culturing cells in NM23-H1 dimers or NME7 caused fibroblasts to revert to a stem-like state without ectopic expression of pluripotency genes; 3) culturing cells in NM23-H1 dimers or NME7 caused an increase in the amount of MUC1 or MUC1* that the cells expressed; and 4) forced expression of pluripotency genes Oct4, Sox2, Klf4 and/or c-Myc caused an increase in the amount of MUC1 or MUC1* that the cells expressed.

### Variations in number of the Yamanaka pluripotency genes used

In this set of experiments, human fibroblasts were transfected with either all four pluripotency genes OSKM, or Three (3) OSK or OSM and cultured in the standard media or in NM23-MM-A or NM23-MM-R. To further characterize the molecular makeup of the cells induced to become pluripotent, cells were transferred from plastic to chamber slides at Day 5, grown until Day 10, then stained for the presence of pluripotency marker Tra 1-60 and for nuclei using DAPI.

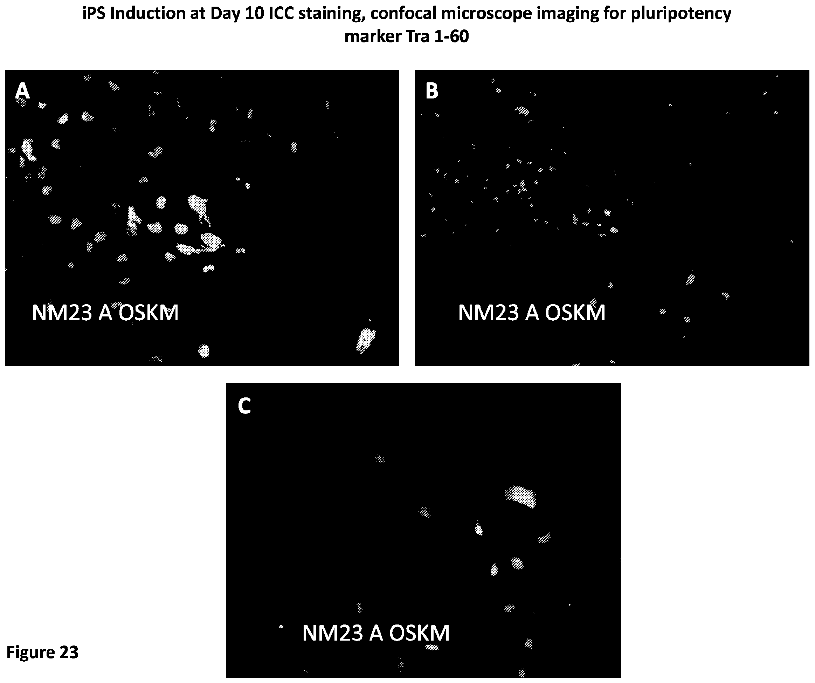

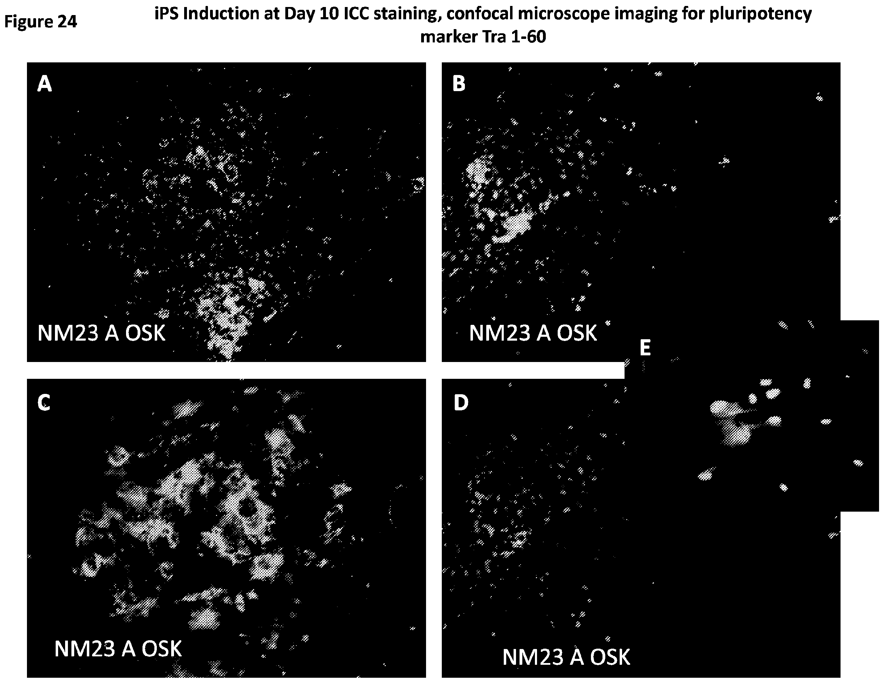

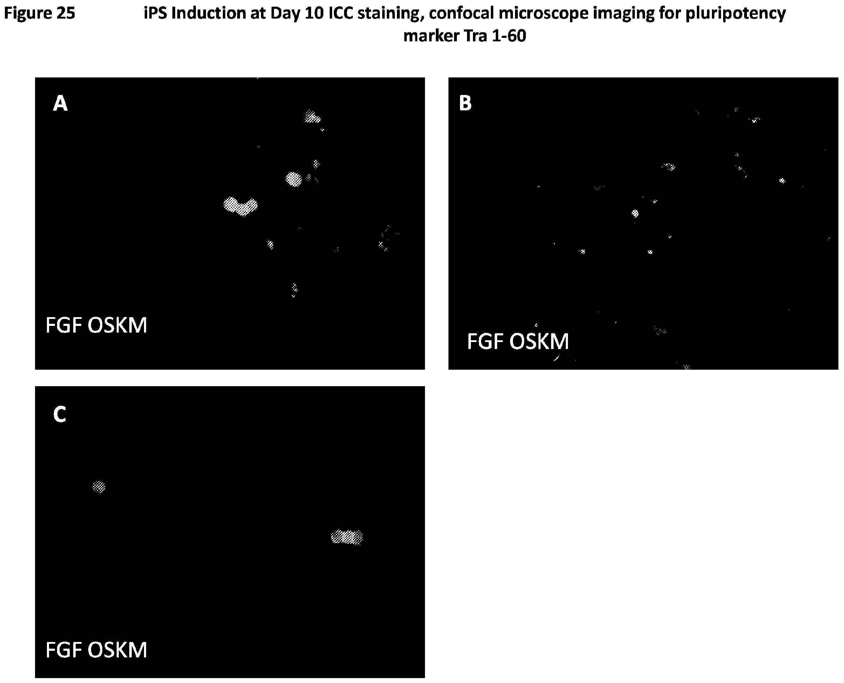

Figure 23 shows cells that were transfected with OSKM and cultured in NM23-MM-A. The green stain for Tra 1-60 highlights those cells that have been induced to pluripotency. In contrast, Figure 24 shows that when cells are transfected with only OSK and cultured in NM23 dimers , there is an abundance of cells staining positive for the pluripotency marker Tra 1-60. There was no other condition that allowed detection of 4 or more pluripotent cells in a single view. Figure 25 shows that using the standard media and all four pluripotency genes OSKM, only a few cells could be located.

### Cells and source of cells

The disclosure is not meant to be limited by the type of cell or the source of the cell. We have demonstrated that contacting a cell with NM23-H1 dimers, NM23 mutants or variants that induce dimerization of MUC1, or NME7 induces cells to revert to a less mature state and showed that the progression to a less mature state or a fully pluripotent state occurs over a period of time. In addition to contacting cells with an NME family member, we showed that contacting the cells with a biological or chemical agent that induces expression of one or more pluripotency gene increases the efficiency of reverting the cells to a less mature state. We have demonstrated these discoveries using embryonic stem cells and iPS cells, which are fully pluripotent cells. We have also demonstrated these discoveries using fibroblasts. This choice of demonstration cell types, thus covers the range from the most primitive pluripotent cell to a mature cell. The disclosure can be used to make virtually any type of cell revert to a less mature state or a completely pluripotent state. Starting cell types include but are not limited to somatic cells, cells from cord blood, bone marrow cells, peripheral blood cells, mobilized blood cells, hematopoietic stem cells, dermablasts, fibroblasts, neuronal cells, nerve cells, hair follicules, mesenchymal stem cells and cells from cerebrospinal fluid.

Cells to be used with methods of the invention may be derived from any one of a number of sources. Cells may be obtained from a patient for autologous uses, or from donors.

In a preferred embodiment, the cells are human. However, we have demonstrated that we can grow mouse stem cells in human NM23-H1 dimers or in NME7 and it alone is sufficient to maintain mouse stem cells in the pluripotent state, without the use of the conventionally "required" LIF. Conversely, the NM23 proteins need not be human because of the large degree of conservation among species. However, human NM23-H1, NME6 and NME7 are preferred. Mutant NM23 proteins, such as the S120G mutation in NM23-H1, that favor dimerization are preferred.

### Uses of stem cells

Methods of the invention are envisioned to be used in a number of *in vitro* and *ex vivo* applications. In one aspect, methods of the invention are used to make induced pluripotent stem (iPS) cells *in vitro,* which can then be used as is or differentiated for any number of applications, including research, drug testing, toxicology, or therapeutic uses.

### Trans-differentiation

In another aspect, methods of the invention are used to make cells revert to a less mature state and then differentiated such that they develop into a desired cells type. Methods of the invention can therefore be used in trans-differentiation techniques, which are also called direct differentiation techniques, wherein cells are only partially reverted to a pluripotent state. Current techniques for trans-differentiation involve inducing expression of two or more of the pluripotency genes (Oct4, Sox2, Klf4, c-Myc, Lin28 and Nanog) for shorter periods of time than is required to make cells fully pluripotent, then introducing biological or chemical agents that direct differentiation to a particular cell lineage or cell type. For example, methods of the invention can be used to induce cells to revert to a less mature state, whereupon the cells are differentiated to a desired state. This can be carried out *in vitro,* or *ex vivo.* In one aspect of the disclosure, the cells are *in vivo,* for example in a patient, where they are reverted to a less mature state and then induced to differentiate to a desired state. For example, about half of the heart cells are fibroblasts. Therefore a treatment for heart conditions that could be ameliorated by increasing the number of healthy cardiomyocytes, is to cause the cardio fibroblasts *in situ* to revert to a less mature state using methods described herein and then contacted them with other agents to induce them to differentiate into cardiomyocytes.

Techniques of the described herein need not be carried out completely *in vitro* or *in vivo.* In another aspect, cells can be harvested from the cerebrospinal fluid, or from another source, reverted to a less mature state using methods of described herein, then differentiated into some other desired cell type or lineage, such as neuronal cells and then introduced into patient, for example into the spinal fluid, which has access to the brain. The source cells may be obtained from the patient and then re-introduced in a differentiated or semi-differentiated state. Alternatively, cells could be harvested from a patient or donor, which may be derived from the desired lineage and reverted to a less mature state, then introduced to site where they are influenced to become the desired cell type. In this way cells can be harvested from a patient or donor, reverted to a less mature state and then introduced to the part of the patient in need of therapeutic cells, wherein the local environment or the addition of exogenous agents would make the cells differentiate into the desired cell type.

### Wound healing

In yet another aspect, methods of the disclosure can be used in settings suitable for the promotion of wound healing. In this aspect, agents are in a medium or immobilized on a support, which may be a bandage, a stent, a scaffold, a scaffold for tissue regeneration, a scaffold or support for spinal cord regeneration and the like. In one example, a bandage coated with a MUC1* ligand such as NM23-H1 dimers or NME7 would revert cells, proximal to an injury, to a stem-like state whereupon they would accelerate healing.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. The following examples are offered by way of illustration of the present invention and not by way of limitation.

The following examples show that: 1) MUC1 associated factors induce pluripotency in a cell that is not pluripotent; 2) MUC1 associated factors increase the efficiency of iPS generation; 3) MUC1 associated factors replace the requirement for one or more genes that are currently thought to be required for inducing pluripotency. NM23 is a ligand of MUC1* and is a MUC1* associated factor.

### EXAMPLES

EXAMPLE 1. MUC1* promotes growth and cell death resistance.

MUC1* promotes clonogenic growth (colony expansion) of fibroblasts. Single cell clones of 3Y1 cells transfected with either full-length MUC1 (SEQ ID NO:1), MUC1*₁₁₁₀ (SEQ ID NO:5) or empty vector were plated at 1000 cells per 60mm dish in DMEM media containing 10% fetal bovine serum, penicillin/streptomycin and G418 (600 µg/ml). Cells were grown for 9 days and then fixed in 4% paraformaldehyde for 15 minutes at room temperature. Dishes were washed with water and then stained with 1% crystal violet in 70% methanol for 20 minutes at room temperature. Dishes were washed three times with water and allowed to dry overnight at room temperature and photographed. Figure 1A shows that the amount of crystal violet that is absorbed (an indicator of cell number) is much higher where MUC1* single cell clones #3 and #44 are growing. In contrast, cells that transfected with full-length MUC1 (single cell clones #8 and #17) showed no growth rate increase over cells transfected with the empty vector. This shows that the cleaved form, MUC1*, confers a growth and/or survival advantage and not the full-length protein.

**EXAMPLE 2.** Anti-MUC1* Fab blocks resistance to cell death by TAXOL^{®} in trastuzumab (HERCEPTIN^{®})-resistant cells (made resistant by culture in 1ug/m1 HERCEPTIN^{®}). Fessler et al., 2009 reported that HERCEPTIN^{®} resistant cells are also resistant to TAXOL^{®}, doxorubicin and cyclophosphamide. As reported, these drug resistant cancer cells achieve resistance by overexpressing MUC1*. The following experiment showed that blocking the PSMGFR portion of the MUC1* extracellular domain reversed acquired drug resistance in cancer cells. Parental (BT474) or resistant (BTRes1) cells were plated at a density of 10,000 cells/well in 96 well plates, 4 wells/condition. The following day, Anti-MUC1* Fab, control Fab, or no Fab were added to cells in the presence or absence of TAXOL^{®} (Paclitaxel Sigma T7191). Two days later, cells were resuspended in 50 µl trypsin, and counted in the presence of trypan blue. Percent cell death was calculated as percent trypan blue uptake. BT474 cells underwent cell death in response to TAXOL^{®} under each condition, and BTRes1 cells only underwent cell death in the presence of MUC1* antibody (Figure 1B).

**EXAMPLE 3.** MUC1* acts as a growth factor receptor, and is activated by dimerization of its extracellular domain using an artificial (anti-MUC1* antibody) or its natural ligand, NM23 (NME). MUC1*-positive ZR-75-30 cells, 6000/well, or control (MUC1-negative) HEK293 cells 4000/well, were plated in 96 well plates. The following day, zero hour cell counts were taken, and different concentrations of anti-MUC1* antibody or Fab were added in medium with low (0.1%) serum every 24 or 48 hours. After several days of incubation, cells were resuspended in trypsin and counted, and percent normalized growth was calculated. Stimulation of ZR-75-30 cells, shown as a bell-shaped curve, as is demonstrated for ligand-induced growth stimulation, but not HEK293 cells (Figure 1C). In a similar experiment, using MUC1*-positive T47D breast cancer cells stably transfected with siRNA targeting MUC1, or control siRNA, stimulation of growth only occurred with controltransfected cells, further demonstrating specificity of antibody (Figure 1D). Identical results were demonstrated for MUC1*'s natural ligand, NM23 (Figure 1E).

EXAMPLE 4. NM23 binds specifically to the PSMGFR peptide which is comprised essentially of the extracellular domain of MUC1*. Binding was measured by Surface Plasmon Resonance, using a Biacore3000 instrument and BiaEvaluation software. Histidine-tagged MUC1*_{1110-ecd} (SEQ ID NO:5) or irrelevant peptide (HHHHHH-SSSSGSSSSGSSSSGGRGDSGRGDS - SEQ ID NO:40) were immobilized on separate flow channels of 5.7% NTA-Ni⁺⁺ SAM-coated SPR chips, prepared in our lab as described in Mahanta et al. 2008. 35 µL plugs of NM23, purified bovine or recombinant human, were injected into a constant flow stream of 5uL/minute and sensograms were recorded. NM23 purified from bovine liver (Sigma N-2635) was diluted in PBS alone. Affinities were measured over a wide range of concentrations using a 1:1 Langmuir model. Actual affinities may vary as first order kinetics cannot adequately describe this system. (Figure 1F).

EXAMPLE 5. MUC1* Growth Factor Receptor and its ligand NM23 are on undifferentiated hESC, but not differentiated hESC. Human embryonic stem cells in the undifferentiated (pluripotent) state or in the newly differentiating state were analyzed by immunocytochemistry (ICC). Human embryonic stem cells (hESCs) were manually dissected and plated in 8-well chamber slides (Nunc) that had been pre-coated with matrigel. For undifferentiated cells, cells were fixed 5-7 days after plating. For differentiated cells, bFGF was removed from the culture medium 5-7 days after plating and cells were allowed to differentiate for 14 days before fixation. Cells were washed with PBS prior to fixation with 4% paraformaldehyde in 0.1M cacodylate buffer for 15 minutes at 4°C. Cells were blocked for 1 hour with 1% BSA and 1% donkey or goat serum in PBS. 0.1% NP-40 was used with antibodies against intracellular antigens. Primary antibodies were diluted in block and incubated with cells for 1 hour at 4°C. The primary antibodies for the following proteins were used: OCT4 (Santa Cruz, Clone Clones H-134 and C-10, 1:100 dilution), full-length MUC1 (VU4H5, Santa Cruz Biotechnology, 1:50 dilution), MUC1* (Minerva, 1:250 dilution), or NM23 (Santa Cruz, Clone NM301, 1:100 dilution)). Cells were washed 3 times in PBS for 5 minutes prior to incubation for 30 minutes with secondary antibodies: AlexaFluor 488 Goat anti-rabbit IgG, AlexaFluor 555 Goat anti-mouse IgG, AlexaFluor 350 Goat anti-rabbit IgG (Invitrogen, 1:200); Goat anti-mouse IgM-TR (Santa Cruz, 1:100). Cells were washed 3 times in PBS for 5 minutes prior to coverslip mounting using an antifade mounting medium (Biomeda). Nuclei were visualized by DAPI staining (1µg/ml) for 5 minutes. Immunostained cells were visualized on an Olympus BX-51 epifluorescent microscope. Results of these experiments show that MUC1* is on the surface of undifferentiated cells (pluripotent stem cells) (Fig 2A, 3B, 3C) but is not on differentiated hESCs (Fig 2 D). Figure 3 shows that the ligand of MUC1*, NM23, co-localizes with MUC1* (Figs. 3 A-C). MUC1* and its ligand NM23 are only expressed on pluripotent stem cells (OCT4-positive cells) and not on those that have differentiated, Figs. 3C and 3F (DAPI stains nuclei of OCT4-negative cells).

**EXAMPLE 6.** MUC1* mediates growth of pluripotent stem cells.

The following experiment was performed to determine the effect of stimulating MUC1*, using a bivalent anti-MUC1*, on pluripotent stem cells. The results show that adding a MUC1* dimerizing ligand stimulates pluripotent (OCT4-positive) stem cell growth and also enables their growth in the absence of feeder cells, their extracts or bFGF.

Long term growth of pluripotent (OCT4-positive) hESC is mediated by MUC1* stimulation. hESCs were trypsin-dissociated and seeded in 8-well chamber slides pre-coated with matrigel at 4 x 10⁴ cells/well. Media was changed and antibodies added every other day at a final concentration of 1 µg/ml for bivalent anti-MUC1* until discrete colonies were visible. Culture conditions include 'minimal stem cell medium' (hESC media without feeder-conditioned medium) and Hs27-conditioned medium, with and without bFGF supplementation. For each condition, cells were grown in quadruplicate. Cells were washed with PBS and fixed, and OCT4 immunostaining was conducted as described above. Figure 4, panels A-D are photos of cells grown over matrigel and conditioned medium from fibroblast feeder cells added. Panels E-H are photos of cells grown over matrigel in which no conditioned medium from fibroblast feeder cells was added. The addition of anti-MUC1* antibody to cell cultures (Fig. 4 C, D) resulted in more pluripotent stem cells than growth supplemented by bFGF (Fig. 4 A, B). The addition of anti-MUC1* antibody to cells cultured in the absence of conditioned medium from fibroblast feeder cells (Fig. 4 G, H) resulted in an abundance of pluripotent stem cells, in sharp contrast to cells grown by adding bFGF (Fig. 4 E, F), which resulted in no pluripotent cells (absence of OCT4).

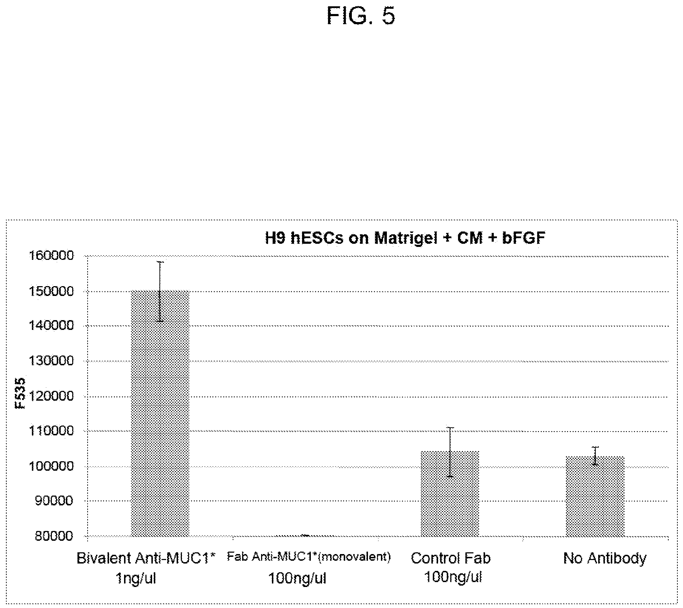

**EXAMPLE 7.** The effect of stimulating MUC1* to enhance the growth of pluripotent stem cells was directly measured in a quantitative Calcein assay. Human embryonic stem cells (hESCs) were manually dissected and grown on matrigel-coated wells of a 96 well plate at a density of 1.9x 10⁴ cells/well. Culture media contained hESC media supplemented with 30% Hs27-conditioned medium and 4 ng/ml bFGF. Antibodies were added at a final concentration of 1 µg/ml for bivalent anti-MUC1* and 100 µg/ml for monovalent anti-MUC1*. Experiments were performed in triplicate. 41 hours-post antibody treatment, live and dead cells were quantified with the LIVE/DEAD viability/cytotoxicity kit (Molecular Probes), following manufacturer's instructions. Fluorescence was measured using a Victor3V plate reader (Perkin Elmer). The bar graph of Figure 5 shows that stimulation of MUC1* using a dimerizing ligand (anti-MUC1*) enhanced stem cell growth, while blocking the extracellular domain of MUC 1 *, with the anti-MUC1* Fab, resulting in total stem cell death.

**EXAMPLE 8.** A long-term stem cell growth experiment was done to compare the effects of stimulating the growth of stem cells using a bivalent anti-MUC1* antibody, NM23, NM23-mutant, or bFGF. hESCs were dissociated with trypsin and seeded in 8-well chamber slides pre-coated with Matrigel at a cell density of 8.2x10⁴ cells/well. Media was changed and antibodies or wild type or mutant NM23 proteins were added every other day at final concentrations of 80ng/ml for Anti-MUC1* antibody, 1nM for wild type recombinant NM23 or mutant (S120G) NM23, or recombinant bFGF at a final concentration of 4ng/ml in 'minimal stem cell medium' (hESC media without feeder-conditioned medium). Cells were also grown as a control in minimal stem cell medium with 30% conditioned medium from Hs27 fibroblasts and 4ng/ml recombinant bFGF (Peprotech #100-18B). Results of this experiment show that MUC1* ligands do a better job of stimulating growth in minimal media of pluripotent colonies than does conditioned media plus bFGF, the 'normal' growth medium of these cells on Matrigel. Table 1 details the results.

**Table 1. hESCs cultured in minimal media for 4 weeks**

| **Growth condition** | **Week 1st colony appeared** | **Number of colonies** | **Morphology** |
|---|---|---|---|
| *Minimal Stem Cell Growth Media* | | | |
| NM23 1nM | Week 2 | 2 colonies | 2 large undifferentiated colonies in 1 of 1 wells; centers of colonies appear to begin to differentiate during week 3; by end of week 4, most of each colony remains undifferentiated |
| NM23-S 120G mutant 1nM | Week 2 | 7 colonies | 7 large undifferentiated colonies in 1 of 1 wells; centers of colonies appear to begin to differentiate during week 3; by end of week 4, most of each colony remains undifferentiated |
| anti-MUC1* 80 ng/ml | Week 2 | 5 colonies | 7 large undifferentiated colonies in 1 of 2 wells; centers of colonies appear to begin to differentiate during week 3; by end of week 4, most of each colony remains undifferentiated |
| bFGF 4 ng/ml | - | 0 | No colonies |
| nothing | Week 2 | 2 colonies | 2 very small, differentiated colonies |

| ***Control** - 30% Conditioned Media from Hs27 Fibroblast Feeder Cells* | | | |
|---|---|---|---|
| bFGF 4 ng/ml | Week 2 | 5 | 5 mostly differentiated colonies |

**EXAMPLE 9.** MUC1* translocates to nucleus of cells. Anti-MUC1* monoclonal Ab was labeled *in vitro* with Alexa 555 dye, and bound at 4°C to HCT-116 cells (MUC1-negative) transfected with MUC1*, that had been washed in cold PBS, at 4°C. After 20 min, cells were washed twice in cold PBS, and cells were either fixed in 4% paraformaldehyde, or incubated with pre-warmed growth medium. Cells were washed after 40 minutes, and fixed with 4% paraformaldehyde for 5 minutes, then blocked and permeabilized with 2.5% BSA, 2.5% FBS and 0.1% NP-40 in PBS. Endosomes were stained using an anti-EEA1 antibody (Cell Signaling Technologies, 2411S) and Alexa 488 (Invitrogen 1:200) (Fig. 6).

In the examples described below, cells are cultured in either standard fibroblast media (FM), bFGF-based media (FGF-MM) or NM23-_{S120G-dimer} in a minimal stem cell media (NM23-MM) or NME7 devoid of its M leader sequence such that it only contains its A and B domains (referred to as simply NME7 herein or NME7-AB).

**Fibroblast Media, "FM":** for 500mLs: 435mL DMEM (ATCC #30-2002 Manassas, VA), 50mL fetal bovine serum (FBS, #35-011-cv, Mediatech, Manassas, VA), 5mL of 100X stock Glutamax, (#35050061, LifeTechnologies, Carlsbad, CA), 5mL 100X non-essential amino acids (#11140050, LifeTechnologies), 5mL 100X penicillin/streptomycin (#17-602E, Lonza, Allendale, NJ)

**bFGF Media, "FGF-MM"** for 500 mLs: 400mL DMEM/F12 (#10565-042), 100mL Knock Out Serum Replacement, "KOSR" (#10828-028), 5mL 100X non-essential amino acids (#11140050), 0.9mL 100X stock 2-mercaptoethanol (#21985-023), all from LifeTechnologies and 2ug bFGF (#100-18B, Peprotech, Rocky Hill, NJ).

**NM23 Media, "NM23-MM"** for 500 mLs: 400mL DMEM/F12 (#10565-042), 100mL Knock Out Serum Replacement, "KOSR" (#10828-028), 5mL 100X non-essential amino acids (#11140050), 0.9mL 100X stock 2-mercaptoethanol (#21985-023), all from LifeTechnologies and 8nM NM23_{dimer} (Minerva, Waltham, MA).

### Abbreviations:

**OSKM:** Oct4, Sox2, Klf4, c-Myc are the pluripotency genes, combinations of which were used in these experiments to induce pluripotency. Several experiments were performed with results being consistent across all experiments. In some cases a lenti viral system was used to cause ectopic expression of the pluripotency genes, while in other cases nucleic acids were used. In still other cases, the proteins themselves were used rather than the genes that encode them.
**NM23-MM-R or NM23-R:** NM23-MM Replaces the fibroblast media (FM) on Day 7 instead of the standard method of replacing the FM with bFGF-based media (bFGF-M).
**NM23-MM-A or NM23-A:** NM23-MM is Always present from the onset of the experiment.
**TC-MUC1* Ab:** Fibroblasts are plated onto a cell culture plate (often tissue culture treated, but not necessarily) that has been coated with an anti-MUC1* antibody (mAb MN-C3 and MN-C8 at 12.5 ug/mL especially preferred here) instead of plastic, then transferring to a layer of fibroblast feeder cells at Day 5.
**Vita**^{™}**-MUC1* Ab:** Fibroblasts are plated onto a cell culture plate (Vita^{™}: ThermoFisher) that has been coated with an anti-MUC1* antibody (mAb MN-C3 and MN-C8 at 12.5 ug/mL especially preferred here) instead of plastic, then transferring to a layer of fibroblast feeder cells at Day 5.
**HS27:** human foreskin fibroblast feeder cells (inactivated)
**MEFs:** mouse embryonic fibroblast feeder cells (inactivated)

**EXAMPLE 10.** The effect of using NM23 or NME7 on inducing cells to revert to a less mature or pluripotent state.

The procedure for iPS generation was performed wherein all four genes (OSKM) were transfected using a lenti virus system. In this experiment, human fibroblasts were transfected with the four (4) pluripotency genes (ref Yamanaka): Oct4, Sox2, Klf4 and c-Myc, hereafter referred to as OSKM. The standard protocol is to first plate dermablasts or fibroblasts (human fibroblasts "hFFn": #PC501A-hFF, System Biosciences, Mountain View, CA) on plastic and culture them in fibroblast media (FM), changed every 24 hours. After 5 days, the cells are transferred to a surface coated with inactivated fibroblast feeder cells, which can be mouse (MEFs) or human (HS27). For the next 2 days, cells remain in FM. On Day 7 the media is changed to bFGF-M, described above, and media is changed every 24 hours. ~2-4 weeks post initial plating, colonies (clones) that have embryonic stem (ES) cell-like morphology are selected and individually plated into new wells coated with inactivated feeder cells (MEFs, mouse or HS27 human fibroblasts) and sequentially passaged every 3-4 days. Wells that continue to grow as ES-like cells were propagated and tested for the presence of pluripotency markers.

Contrary to the standard protocol, we tested the effect of NM23 media added Always (NM23-MM-A) or at Day 7 (NM23-MM-R) to replace the fibroblast media (FM) after cells had been transferred onto a layer of fibroblast feeder cells. As controls, the transfection reagent was added but the genes were omitted, "mock transfection" or neither the genes nor the transfection reagents was added, "untransfected," or the cells were treated according to standard protocol as described above.

Figures 7A-D are magnified photos of the Control cells on Day 4 of the experiment. A,B show that NM23-MM alone causes the fibroblast to start forming ES-like colonies after 4 days. In contrast, C,D in which the standard fibroblast media, FM, was used do not show any change in cell morphology; they remain like fibroblasts. In these control experiments, no genes were transfected into the fibroblasts.

Figures 8A-C show magnified photos of the cells transfected with OSKM on Day 4 of the experiment. Panels A,B show that the transfectants cultured in NM23-MM-A have begun to form ES-like colonies. Panel C in which according to standard method, cells are cultured in FM, show no changes in fibroblast cell morphology.

Figures 9A-D show magnified photos of the control cells on Day 11 of the experiment. These images show the difference that the surface makes when untransfected cells are cultured in NM23-MM-A over plastic (A), MEFs (B), anti-MUC1* antibody, C3 (C), or anti-MUC1* antibody, C3 plus a Rho kinase inhibitor (ROCi). It was observed that in the absence of any ectopically expressed genes, NM23-MM alone causes the development of ES-like colonies. These resultant cells become non-adherent and float off a regular plastic surface (A), do not form in the presence of MEFs, form best on an anti-MUC1* antibody surface (C) in the absence of ROCi (compare C to D).

Figure 10A-B show magnified photos of the Control, untransfected cells on Day 11 of the experiment, which have been cultured in the standard FM for 7 days then in FGF-MM over MEFs. There is no change from typical fibroblast morphology when cultured in FGF-MM.

Figures 11A-D show magnified photos of fibroblasts induced to become pluripotent with OSKM on Day 11 of the experiment. This experiment compares cells cultured in NM23-MM-A (always), panels A, B to cells first cultured for 7 days in fibroblast media, FM, then switched to NM23-MM-R (replaced), panels C, D. Also compared are growth over a surface coated with anti-MUC1* antibody (A), MEFs (B, D), HS27s (C). The images show that NM23-MM always is better than starting the fibroblasts off in FM and that human fibroblast feeders (HS27) work better than mouse feeders (MEFs) for inducing ES-like colonies.

Figures 12A-B show magnified photos of fibroblasts cultured in FGF-MM and induced to become pluripotent with OSKM on Day 11 of the experiment. This experiment compares morphology of cells plated over a layer of human feeders (A) versus mouse feeders (B).

Figures 13A-D show magnified photos of the Control, untransfected cells on Day 14 of the experiment, which have been cultured in NM23-MM-A (always) over anti-MUC1* antibody (A,B) or over fibroblast feeder cells (C,D). Panels A, C shows cells that had been plated at high density, while B, D were plated at low density. The experiment shows again that anti-MUC1* antibody surface and NM23-MM supports formation of ES-like colonies, i.e. induces pluripotency in the absence of transfection with pluripotency genes and that surface of human feeder cells with NM23-MM also support this induction of pluripotency, albeit to a lesser extent.

Figures 14A-C show magnified photos of the Control, untransfected cells on Day 14 of the experiment, which have been cultured in standard FM then FGF-MM and show no signs of induction of pluripotency.

Figures 15A-C show magnified photos of the fibroblasts transfected with OSKM on Day 14 of the experiment, which have been cultured in NM23-MM-A over an anti-MUC1* antibody surface (A), over plastic (B) or over MEFs (C). Panels A and B show well formed ES-like colonies.

Figures 16A-D show magnified photos of the fibroblasts transfected with OSKM on Day 14 of the experiment, which have been cultured in NM23-MM-R (FM until Day7, then NM23-MM). Panels A and C show colonies formed on MEFs and B, D show colonies formed on HS27s.

Figures 17A-D show magnified photos of the Control, untransfected cells on Day 19 of the experiment, which have been cultured in either NM23-MM-A (always) or NM23-MM-R (replaced). In the absence of transfected genes, NM23-MM induces pluripotent cell morphology.

Figures 18 A-B show magnified photos of the Control, untransfected cells on Day 19 of the experiment, which have been cultured in FM, then FGF-MM. No induction of pluripotency can be seen.

Figures 19A-D show magnified photos of the fibroblasts transfected with OSKM on Day 19 of the experiment, which have been cultured in either NM23-MM-A (A,B) or NM23-MM-R (C,D). The images show that NM23-MM always enhances induction of pluripotency.

Figures 20A-B show cells transfected with OSKM on Day 19, wherein cells have been cultured in FM for 7 days then FGF-MM.

The results of the experiment and the rates of iPS induction are shown in Table 2. As can be seen in Table 2, fibroblasts that were not transfected with any genes, but cultured in NM23-S 120G in dimer form in a media devoid of serum or other growth factors or cytokines produced colonies with stem-like morphology at a rate at least double that of cells transfected with Oct4, Sox2, Klf4 and c-Myc (OSKM) and cultured according to standard methods, which includes culture in FGF media after Day 7. Fibroblasts transfected with three (3) or four (4) of the pluripotency genes and cultured in NM23-S120G in dimer form in a media devoid of serum or other growth factors or cytokines produced colonies with stem-like morphology at a rate of up to 100-times that of the standard method which uses FGF media. Thus, the efficiency of generating induced pluripotent stem cells or cells that are in a less mature state than the starting cells is far greater when cells are contacted by a MUC1* ligand, wherein NM23-H1 in dimeric form or NME7 are preferred. Induction rate is calculated as the number of colonies with stem-like morphology divided by the number of starting cells.

**EXAMPLE 11.** The effect of NM23 on iPS generation wherein only three (3) of the pluripotency genes were transfected using a lenti virus system. In this experiment, we tested the effect of omitting one of the pluripotency genes. Human fibroblasts (hFFn) were transfected with either the four (4) pluripotency genes, Oct4, Sox2, Klf4 and c-Myc, "OSKM", or three (3), OSK, or OSM. The gene expression levels of certain genes that are indicative of pluripotency were assessed on Day 4 and on Day 20 using RT-PCR techniques and immunocytochemistry. Note that the primers used in these experiments are designed such that they will not amplify the genes that are being ectopically expressed. RT-PCR was used to quantify the expression level of genes Oct4, Nanog, Klf4, which are indicators of pluripotency. Expression of MUC1 was also measured.

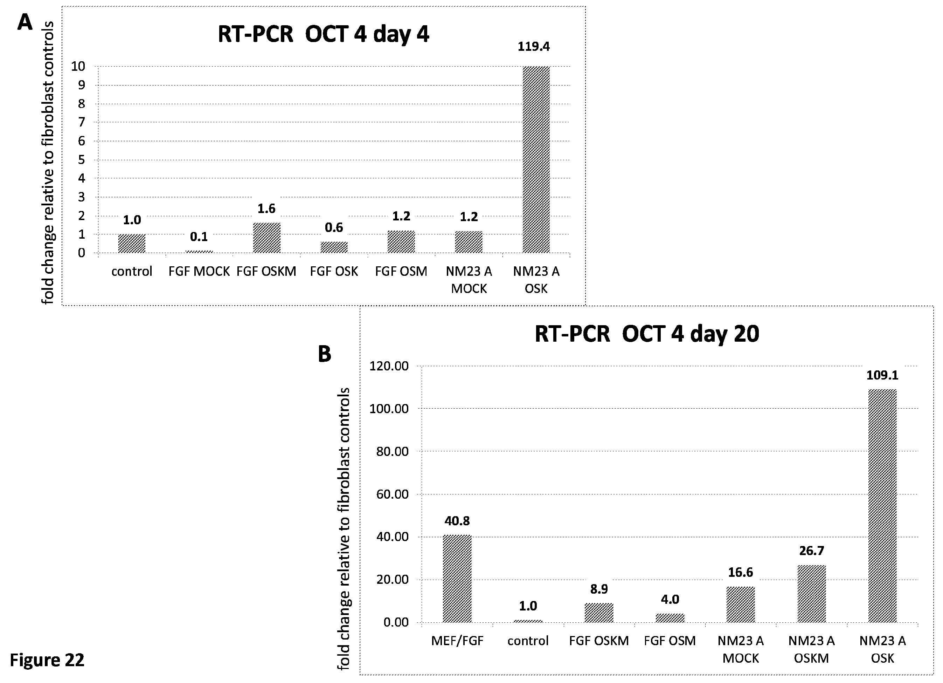

The results of the RT-PCR experiments are summarized in the graphs of Figures 21 and 22. The experiments showed that transfectants cultured in MUC1* ligand, NM23-S120G in dimer form, had an earlier and more pronounced increase in the expression of pluripotency markers than the standard method in which cells were cultured in FGF media. In addition, the experiments showed that three (3) of the pluripotency genes was sufficient for inducing pluripotency if the transfectants were cultured in NM23 media, but not if they were cultured in FGF media (Figure 22). Immunocytochemistry experiments were performed also on Day 10 of the experiment, wherein cells were assayed for the expression of pluripotency marker Tra 1-60. Cells transfected with all four (4) pluripotency genes (OSKM) or only three (3) OSK and cultured in NM23 media showed a vast increase in the expression of Tra 1-60 (Figure 23 and Figure 24) over the same cells cultured in FGF media (Figure 25). No Tra 1-60 positive cells could be found for the condition of cells transfected with OSK and cultured in FGF media on Day 10.

**EXAMPLE 12.** The use of NM23 media enabled the elimination of at least 1 of the 4 pluripotency genes.

In this set of experiments, human fibroblasts were transfected with either all four pluripotency genes OSKM, or three (3) OSK or OSM and cultured in the standard media or in NM23-MM-A or NM23-MM-R. To further characterize the molecular makeup of the cells induced to become pluripotent, cells were transferred from plastic to chamber slides at Day 5, grown until Day 10, then stained for the presence of pluripotency marker Tra 1-60 and for nuclei using DAPI. Figure 23 shows cells that were transfected with OSKM and cultured in NM23 dimers in minimal media from the onset of induction (always: NM23-MM-A). The green stain for Tra 1-60 highlights those cells that have been induced to pluripotency. In contrast, Figure 24 shows that when cells are transfected with only OSK and cultured in NM23-MM-A, there is an abundance of cells staining positive for the pluripotency marker Tra 1-60. There was no other condition that allowed detection of 4 or more pluripotent cells in a single view. Figure 25 shows that using the standard media and all four pluripotency genes OSKM, only a few cells could be located. We note that in other experiments, transfection of OSKM and culturing in NM23 media did produce pluripotent stem cells with good efficiency. However, over several experiments, transfection of OSK and omitting c-Myc seemed to give the highest efficiency of inducing pluripotency.

FACS was done on populations of all the cells transfected with 4 or 3 genes. Sorting was done to identify cells that were positive for pluripotency markers Tra 1-60 and SSEA4 but negative for the fibroblast marker CD13. The results, shown in Table 3 below show that cells transfected with OSK and not c-Myc and cultured in NM23-MM-A had the highest number of pluripotent stem cells.

**Table 3**

| | SSEA4 | SSEA4 & TRA 1-60 | TRA 1-60 |
|---|---|---|---|
| FGF OSKM | 16 | 281 | 21 |
| NM23-R OSKM | 124 | 402 | 26 |
| NM23-A OSKM | 254 | 243 | 12 |
| NM23-R OSK | 4 | 18 | 5 |
| NM23-A OSK | 1258 | 2539 | 400 |

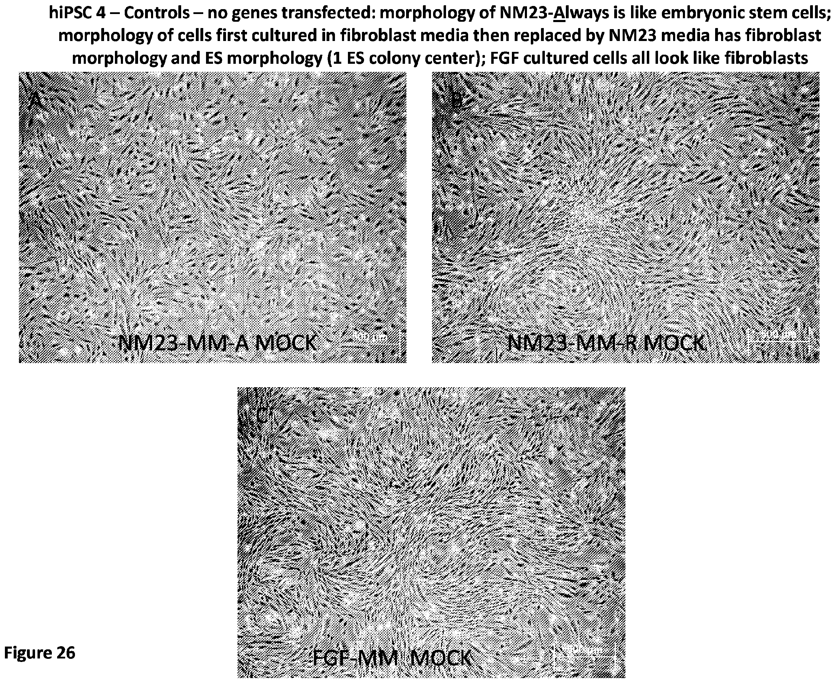

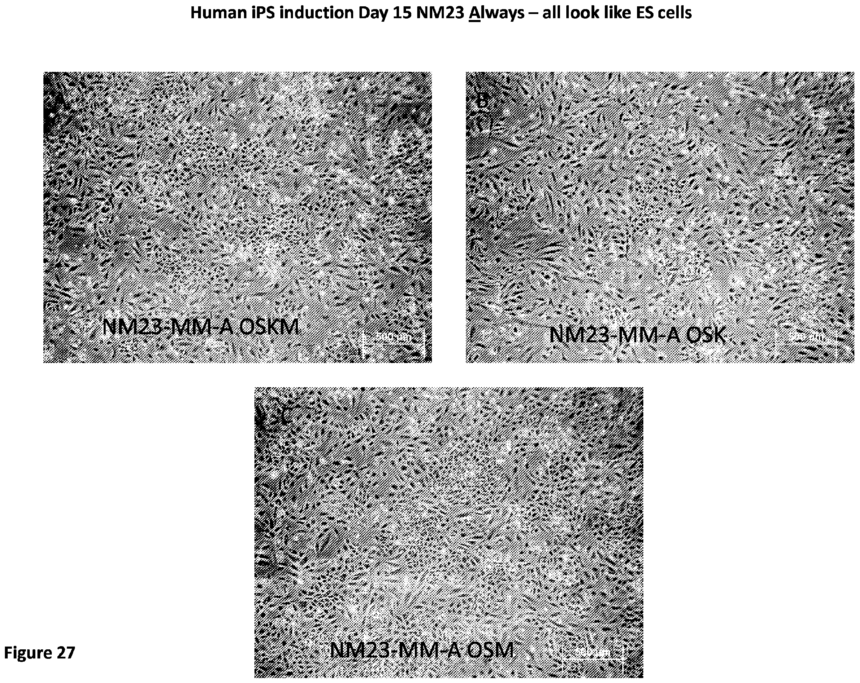

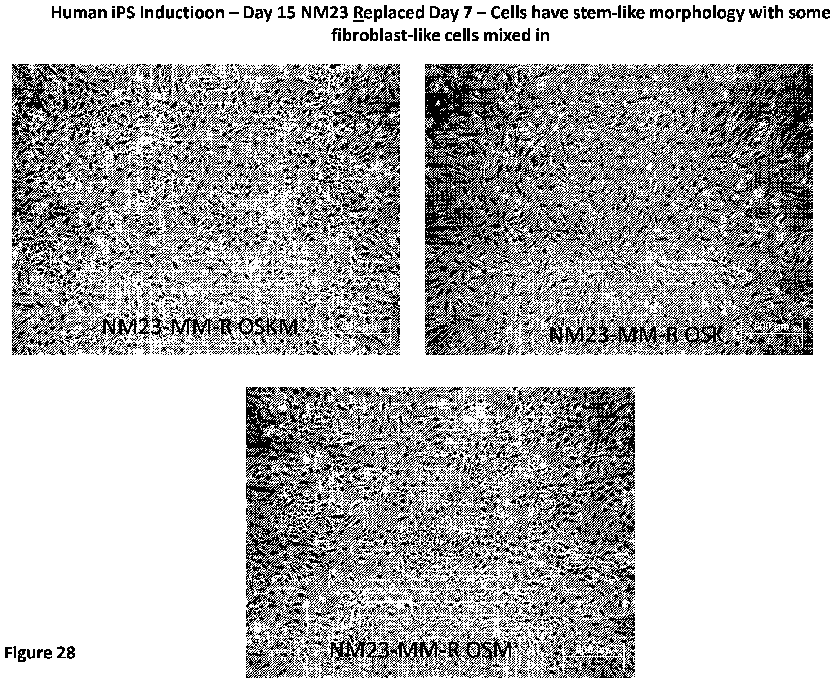

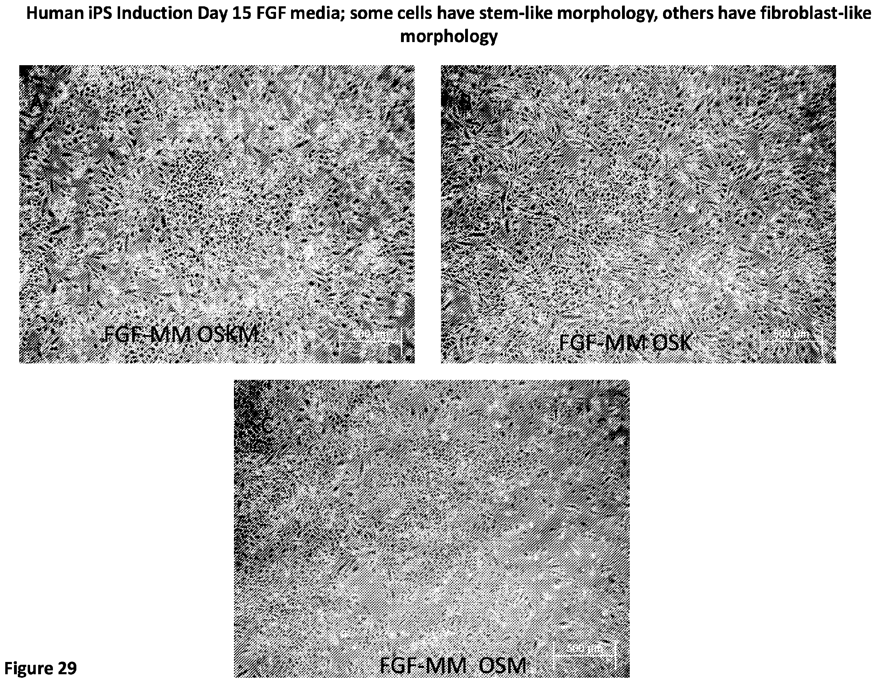

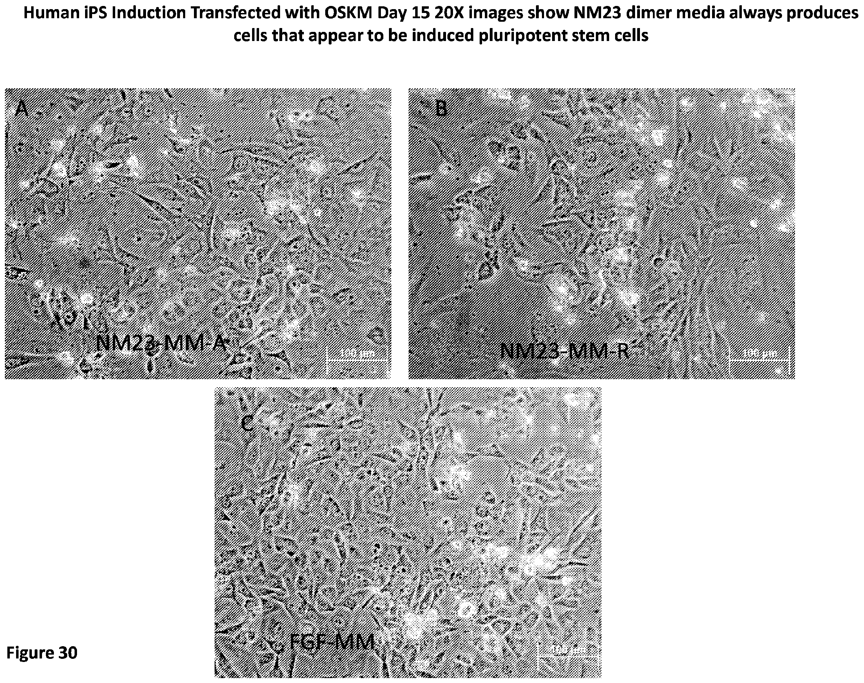

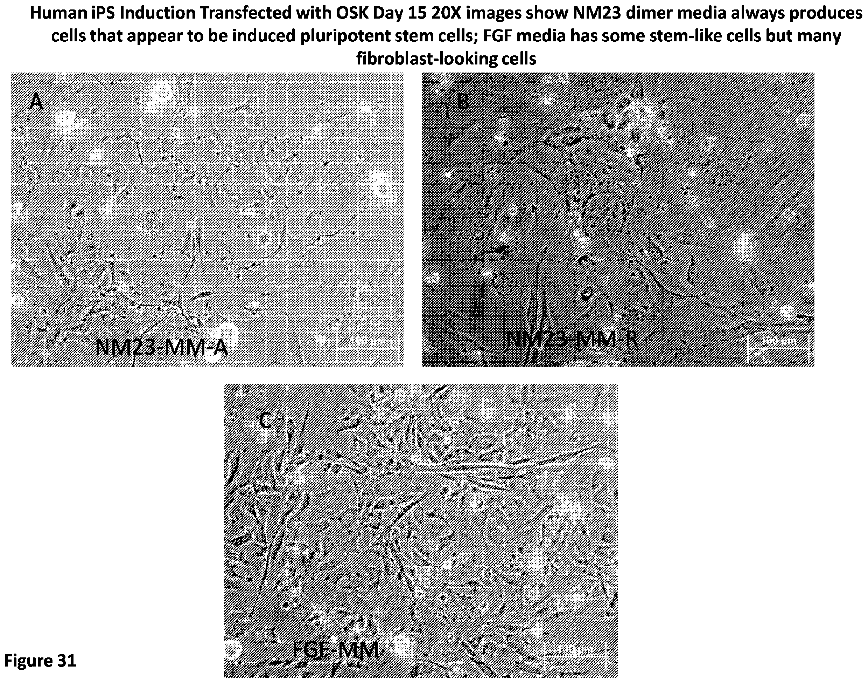

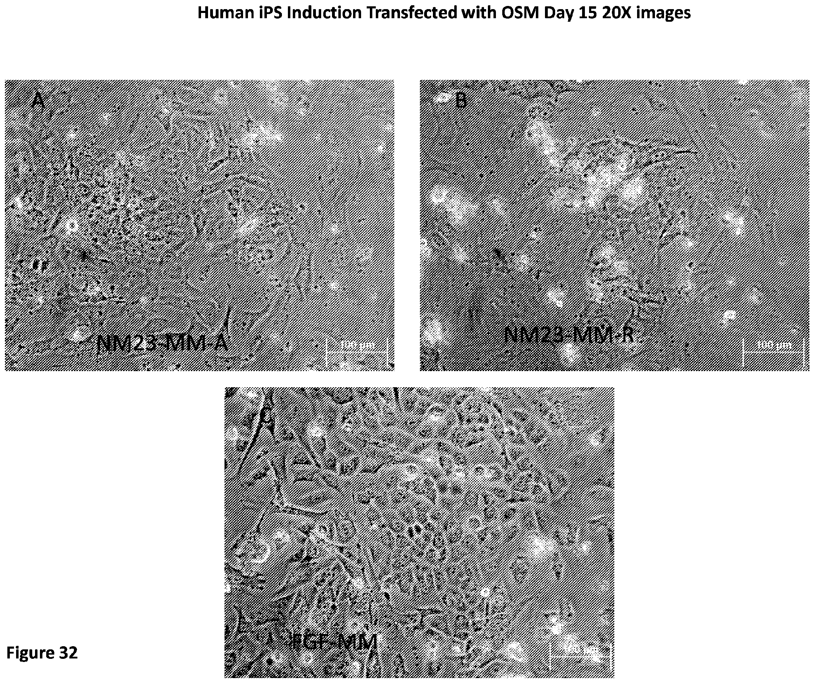

Figures 26 - 32 show bright field images of the cells of the experiment on Day 15.

In addition, cells induced to be pluripotent by transfecting genes OSKM, OSK, or OSM when cultured in NM23-MM or bFGF-MM were analyzed by RT-PCR to quantify the amount of the pluripotency marker OCT4 they expressed on Day 4 then again at Day 20. As the graphs of Figures 21 and 22 show, cells cultured in NM23-MM express increased amounts of the pluripotency gene OCT4 as early as Day 4. Even after 20 days of inducing pluripotency, the cells in NM23-MM express higher levels of OCT4 and/or have more cells that are OCT4 positive. The condition that generated the highest efficiency of induction of pluripotency as measured by OCT4 expression was culture in NM23-MM after transfection of genes OCT4, SOX2 and KLF4 but without transfection of c-Myc.

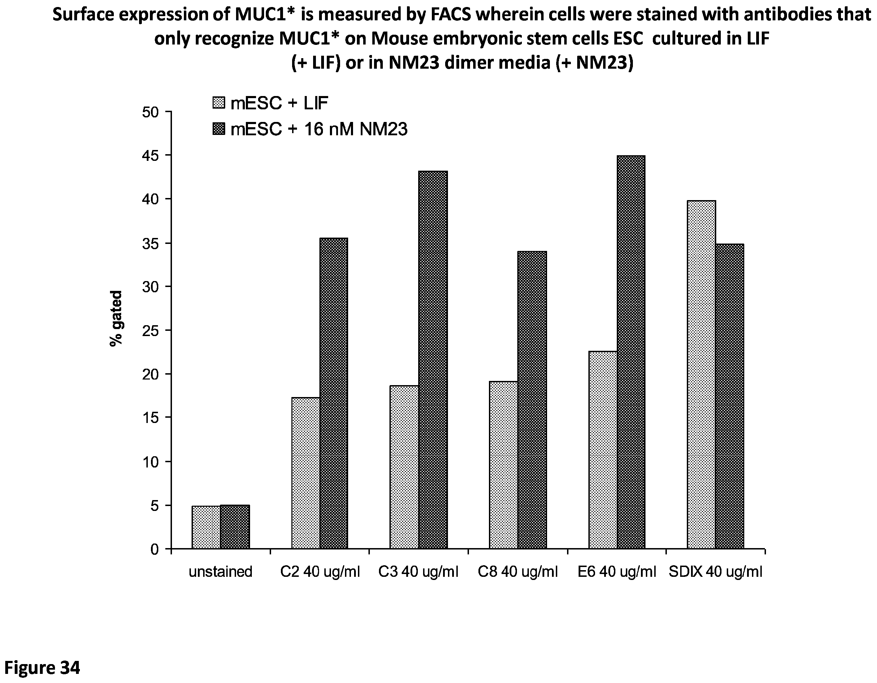

**EXAMPLE 13.** Because of the great conservation of MUC1 proximal regions among mammals, we compared growth of mouse stem cells in the standard media with LIF as the growth factor to growth using NM23 as the growth factor. Mouse ES cells grew as well or better in NM23-MM than mouse ES cells cultured in the standard media containing LIF as the growth factor. Figure 34 shows that using SSEA4 as a measure of pluripotency, cells cultured in NM23 produced more SSEA4-positive cells, i.e. pluripotent cells than cells cultured in LIF. In addition, when these cells were stained with antibodies raised against the human MUC1* sequence, PSMGFR, we found that growth in NM23 increased the amount of MUC1* that the cells expressed, see Figure 25. These results taken together show that other mammalian cells, including mouse stem cells, can be cultured in NM23 rather than the traditional mouse ES growth factor LIF. In addition, the fact that more of those cells recognized MUC1* antibodies argues that culturing stem cells in NM23 increases MUC1* expression.

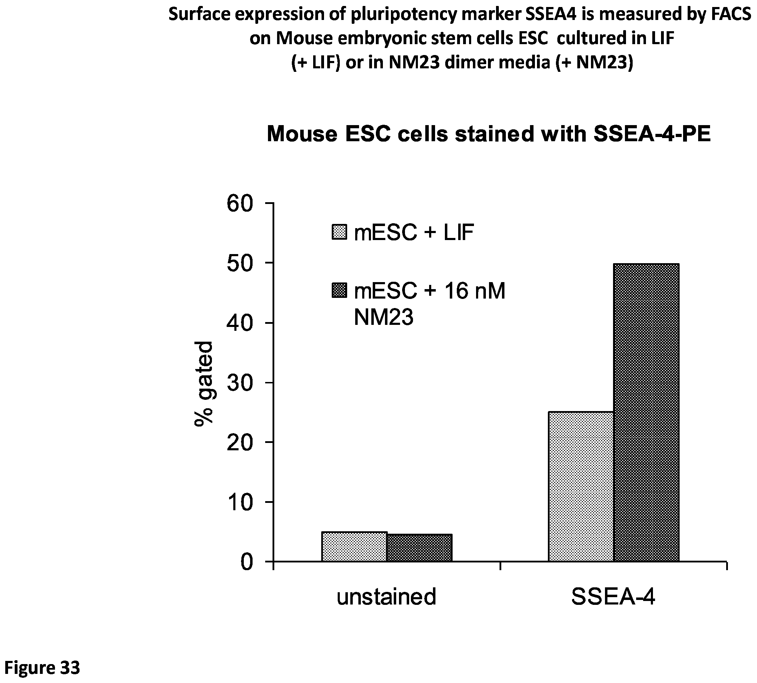

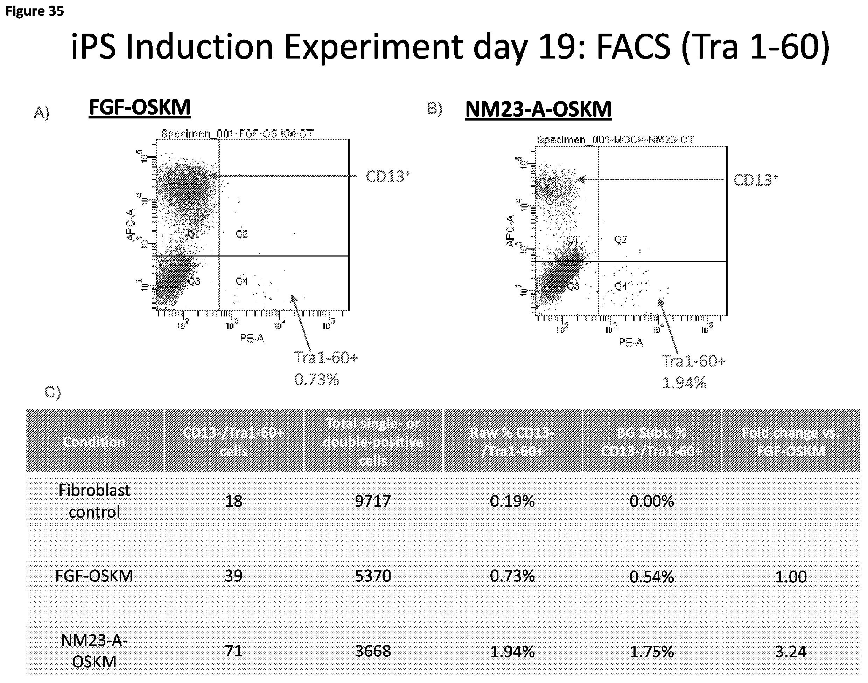

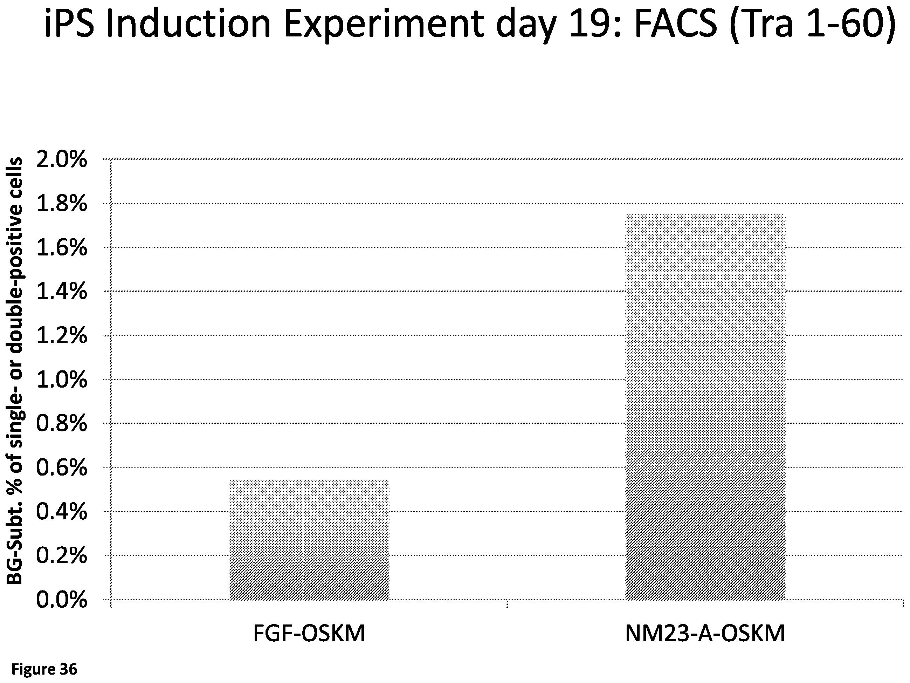

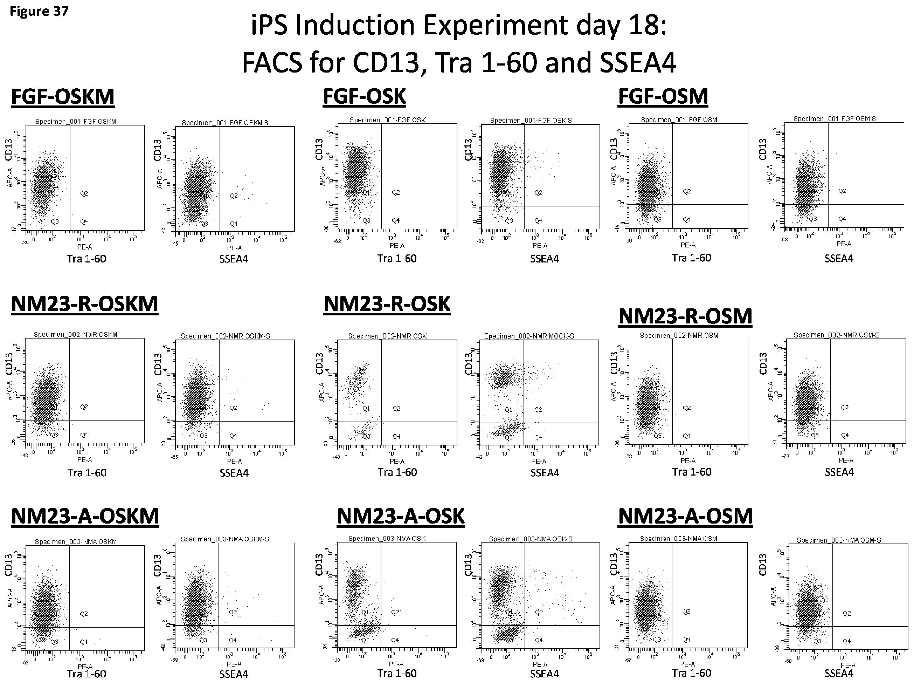

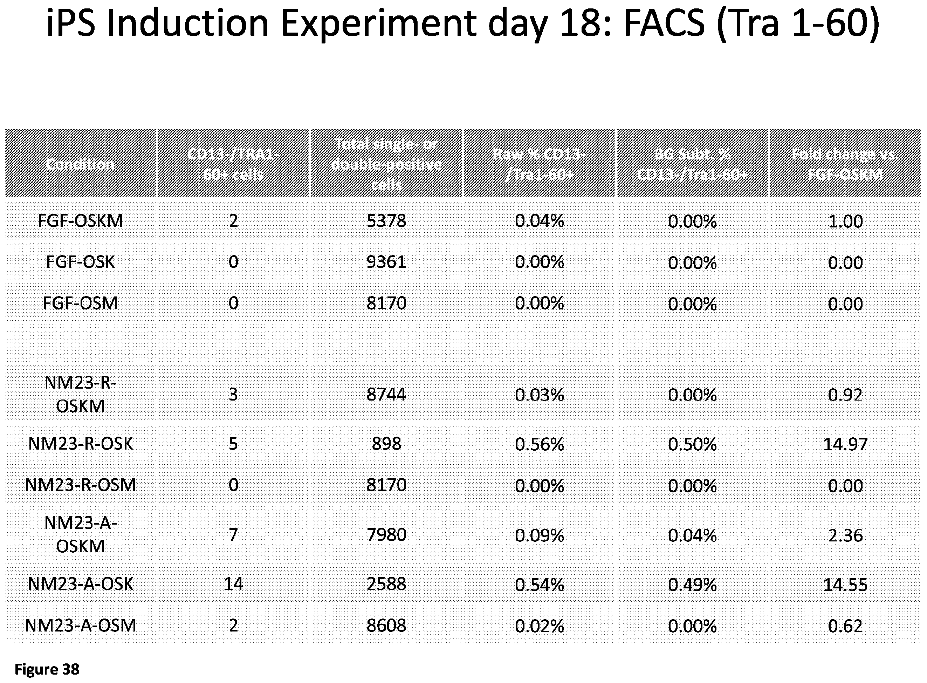

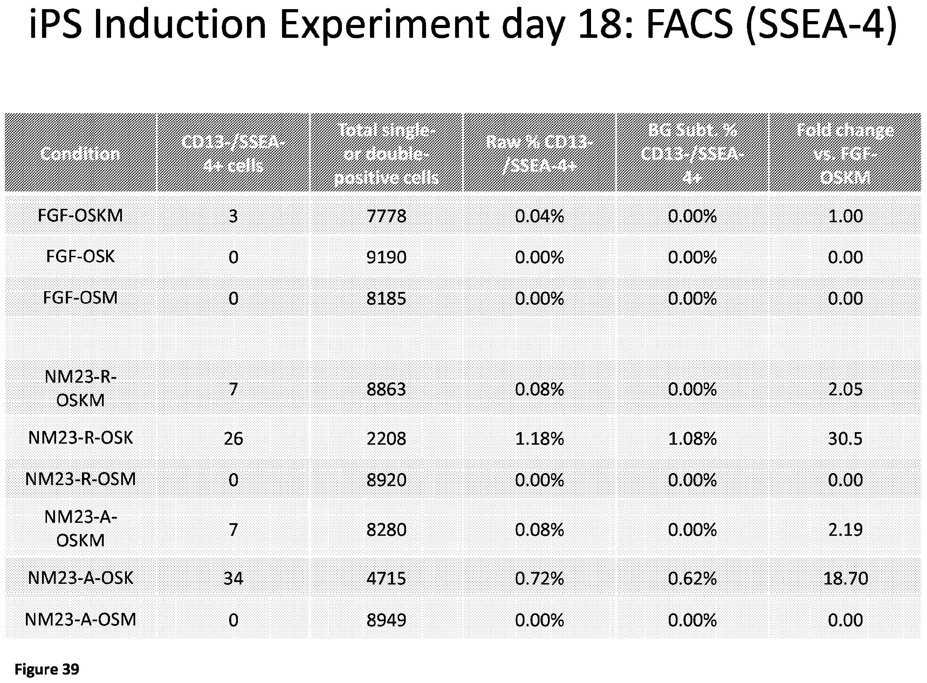

**EXAMPLE 14.** The resultant cells of Examples 10-12 described above, which were repeated four (4) times, were also subjected to FACS analysis to determine the percentage of cells subjected to the pluripotency induction methods that expressed surface markers of pluripotency. FACS analysis should indicate human fibroblast cells induced to revert to a less mature state and to a pluripotent state. The standard method for generating iPS cells takes about 3-4 weeks to develop clones that are pluripotent as evidenced by the expression of pluripotency markers familiar to those skilled in the art. For example, the starting cells in these experiments were fibroblasts. CD13 is a fibroblast marker, whereas cells that have effectively begun to revert to a pluripotent state are CD13-negative. It is known in the art that cells that are pluripotent are positive for pluripotency markers OCT4, KLF4, NANOG, REX-1 and others. Cytoplasmic or nuclear markers are often detected by RT-PCR as described above, while other surface markers are convenient for detecting or sorting live cells by fluorescence activated cell sorting (FACS). At Day 18 and Day 19 after fibroblasts were transfected with some or all of the four "Yamanaka" pluripotency genes, resultant cells were analyzed by FACS for the presence of pluripotency surface proteins SSEA4 and Tra 1-60; cells were also stained for CD13 so that gates could be chosen to exclude any cell that remained positive for the fibroblast marker. Results are shown in Figures 33-39. Figures 35 and 36 show that cells transfected in parallel with all four pluripotency genes, Oct4, Sox2, Klf4, and c-Myc (OSKM) but cultured in NM23 dimer media instead of the standard FGF media produced more than 3-times the number of cells that were positive for pluripotency marker Tra 1-60. Figure 37 shows FACS scans of cells transfected with either 3 or 4 of the pluripotency genes that were cultured for 18 days using NM23 dimer media or the standard FGF media. In this case, cells were stained for CD13, SSEA4 and Tra 1-60. The table of Figure 38 shows that cells transfected with only 3 pluripotency genes, OSK, and cultured in NM23 dimer media produced ~15-times more cells that stained positive for pluripotency marker Tra 1-60 than FGF-cultured cells that had been transfected with all 4 genes. FGF cultured cells were unable to induce pluripotency when transfected with only OSK or OSM. The table of Figure 39 shows that cells transfected with OSK and cultured in NM23 dimer media produced 20-30-times more cells that stained positive for pluripotency marker Tra 1-60 than cells transfected with all four (4) genes and cultured in FGF media.

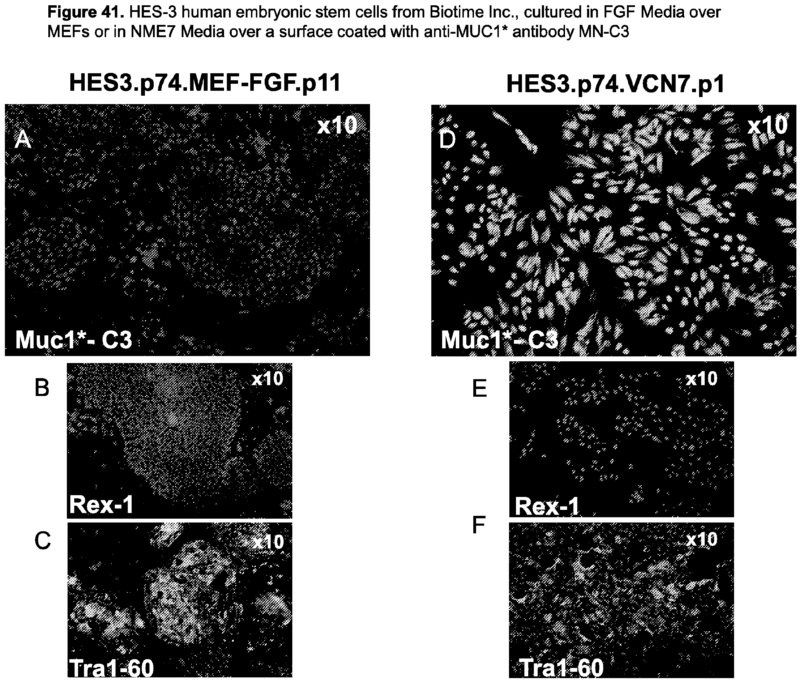

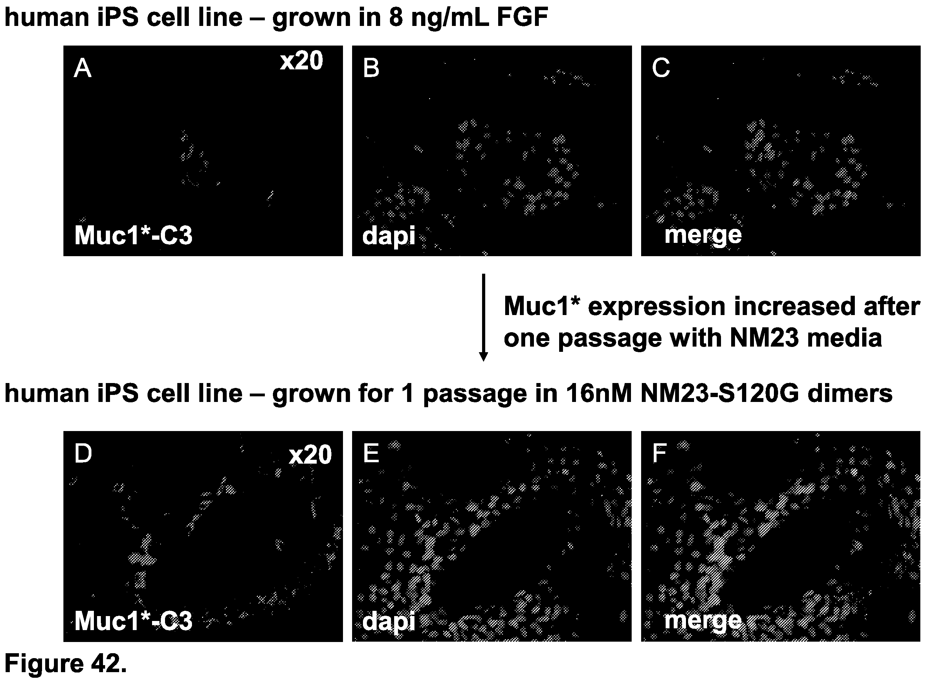

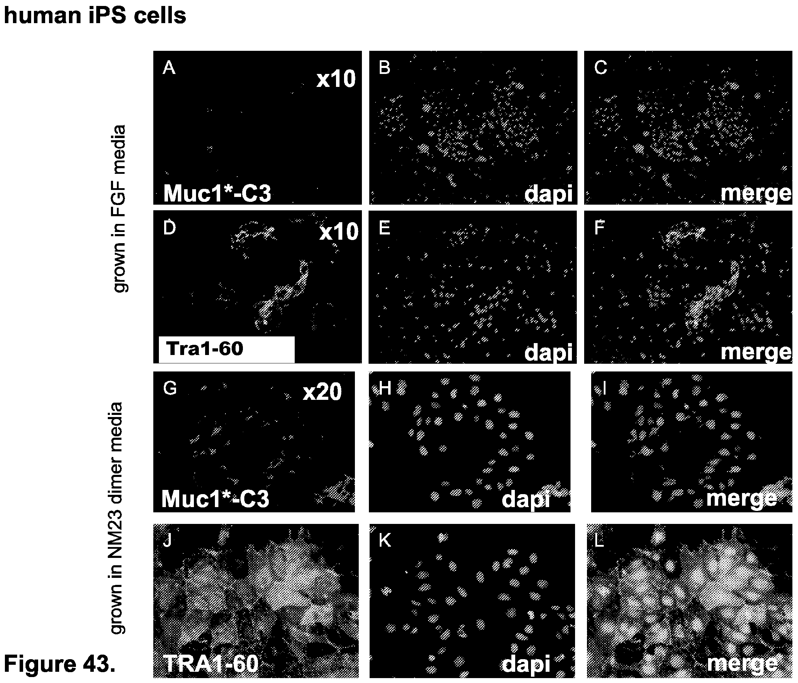

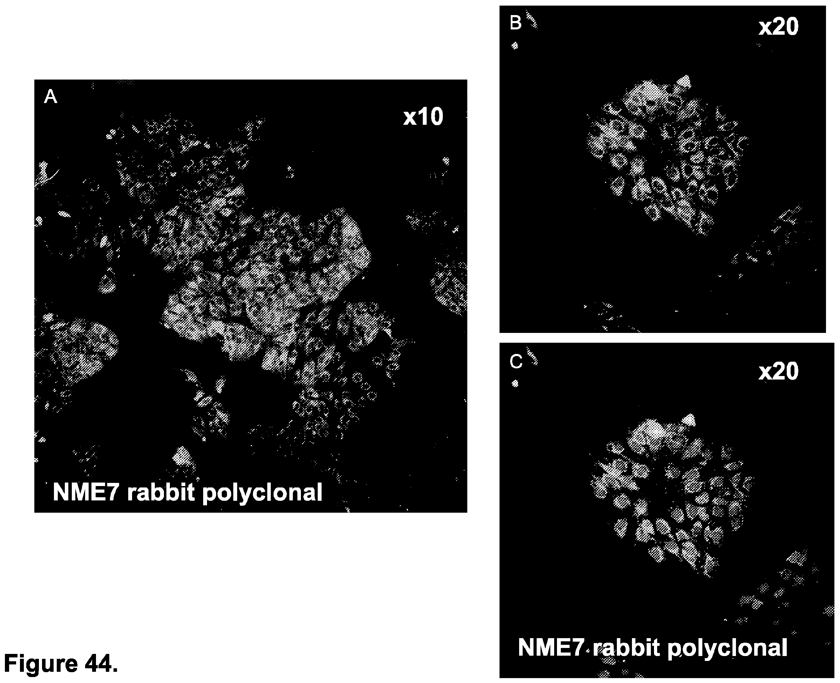

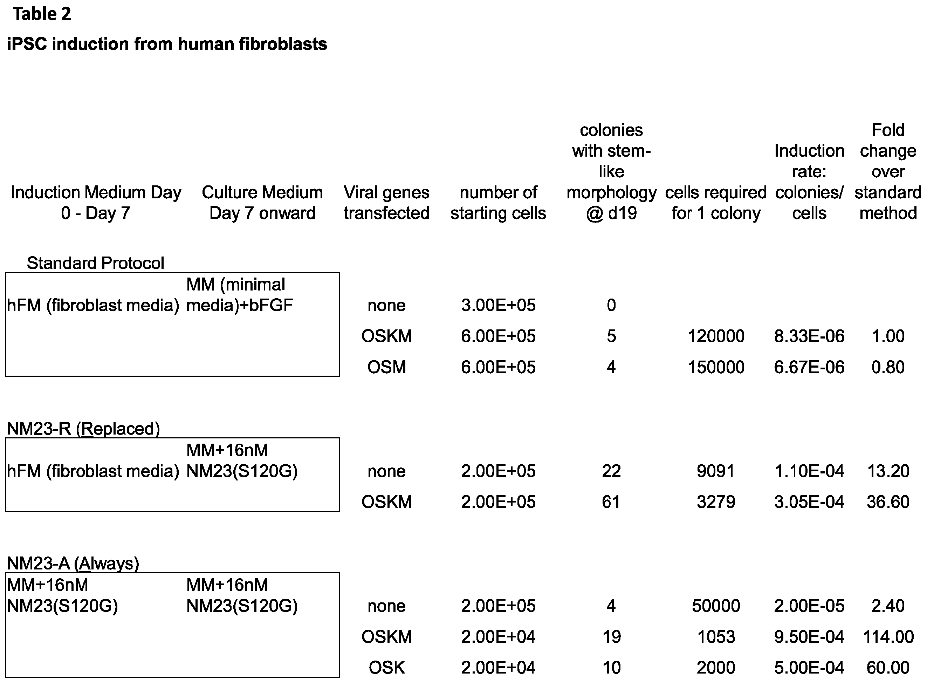

REFERENCE [00239] \ **EXAMPLE 15.** MUC1* ligands increase expression of MUC1*. Experiments were performed with human commercially available human induced pluripotent stem (iPS) cells as well as with human embryonic stem (ES) cells in which we tested the ability of MUC1* ligands to increase the expression or activity of MUC1* as well as their ability to induce cells to revert to a less mature (more pluripotent) state. SC101A-iPSC human iPS cells from Systems Biosciences Inc. were cultured in FGF (4-8ng/mL) Media over MEFs or in NME7 (a MUC1* ligand - 8-16nM) Media over a surface coated with anti-MUC1* antibody MN-C3 (also a MUC1* ligand-12.5 ug/mL coated onto a Vita^{™} plate). Except for the growth factor, FGF or NME7, the base media was identical and is described above as "Minimal Media". Resultant cells were assayed by immunocytochemistry to determine the extent of cells that stained positive for pluripotency markers. The results showed that contacting the cells with MUC1* ligand NME7 caused an increase in the expression of MUC1*, which coincided with an increase in the percentage of cells that stained positive for pluripotency markers. Nuclear stain, DAPI, shows many nuclei that are not also stained by the markers for pluripotency when the cells are cultured in FGF. Figure 40 shows photos of a human induced pluripotent stem (iPS) cell line cultured in either FGF media over a layer of MEFs (A-C) or cultured in NME7 media over a layer of anti-MUC1* antibody, and assayed by immunocytochemistry for the presence of MUC1* (A,D) and pluripotency markers Rex-1 (B,E) and Tra 1-60 (C,F). The same experiment was performed with HES-3 human embryonic stem cells from Biotime Inc., and the same results were obtained. Figure 41 shows photos of a human embryonic stem (ES) cell line cultured in either FGF media over a layer of MEFs (A-C) or cultured in NME7 media over a layer of anti-MUC1* antibody, and assayed by immunocytochemistry for the presence of MUC1* (A,D) and pluripotency markers Rex-1 (B,E) and Tra 1-60 (C,F). Similar experiments were performed, but instead of using NME7 as the growth factor, a mutant NM23-H1 S120G (16nM) was used wherein the NM23 was refolded and purified by FPLC such that it was a pure population of dimers. Herein, this NM23 is referred to simply as NM23 media, NM23 dimer media or NM23-S120G. The results were that after a single passage in NM23 media, there was a vast increase in the amount of MUC1* that was expressed. We note that the monoclonal antibody MN-C3 only recognizes the cleaved MUC1* and does not bind to full-length MUC1. Figure 42 shows photos of a human iPS cell line cultured in either FGF media over a layer of MEFs (A-C) or cultured in NM23-S120G dimer media over a layer of anti-MUC1* antibody (D-F), and assayed by immunocytochemistry for the presence of MUC1* (A,D), nuclear stain DAPI (B,E) and merged images (C,F). The cells were also stained for the pluripotency marker Tra 1-60 and nuclear stain DAPI. In each case, cells cultured in MUC1* ligand, dimeric NM23, caused increased expression of MUC1* that also caused the cells to revert to a less mature state. When cells lose expression of pluripotency markers, they have differentiated out of the pluripotent state. Inspection of the merged images of Figure 43 (C,F,I,L) shows that after treatment with the MUC1* ligand, every nucleus is associated with the stain for a pluripotency marker and MUC1*. Figure 43 shows photos of a human iPS cell line cultured in either FGF media over a layer of MEFs (A-F) or cultured in NM23-S120G dimer media over a layer of anti-MUC1* antibody (G-L), and assayed by immunocytochemistry for the presence of MUC1* (A,G), pluripotency marker Tra 1-60 (D,J), nuclear stain DAPI (B,E,H,K) and merged images (C,F,I,L). The cells were additionally stained for the presence of another MUC1 ligand, NME7. Figure 44 shows photos of a human iPS cell line cultured in NM23-S120G dimer media over a layer of anti-MUC1* antibody and assayed by immunocytochemistry for the presence of NME7 (A,B,C) and nuclear stain DAPI (C).

### REFERENCES

1. Aoi T, Yae K, Nakagawa M, Ichisaka T, Okita K, et al. (2008) Generation of Pluripotent Stem Cells from Adult Mouse Liver and Stomach Cells. pp. 699-702.
2. Boyer LA, Lee TI, Cole MF, Johnstone SE, Levine SS, et al. (2005) Core transcriptional regulatory circuitry in human embryonic stem cells. Cell 122: 947-956.
3. Dexheimer TS, Carey SS, Zuohe S, Gokhale VM, Hu X, et al. (2009) NM23-H2 may play an indirect role in transcriptional activation of c-myc gene expression but does not cleave the nuclease hypersensitive element III(1). Mol Cancer Ther 8: 1363-1377.
4. Efe JA, Hilcove S, Kim J, Zhou H, Ouyang K, et al. (2011) Conversion of mouse fibroblasts into cardiomyocytes using a direct reprogramming strategy. Nat Cell Biol 13: 215-222.
5. Fessler SP, Wotkowicz MT, Mahanta SK, Bamdad C (2009) MUC1* is a determinant of trastuzumab (Herceptin) resistance in breast cancer cells. Breast Cancer Res Treat 118: 113-124.
6. Foster KW, Liu Z, Nail CD, Li X, Fitzgerald TJ, et al. (2005 ) Induction of KLF4 in basal keratinocytes blocks the proliferation-differentiation switch and initiates squamous epithelial dysplasia. Oncogene 24: 1491-1500.
7. Hanna J, Cheng AW, Saha K, Kim J, Lengner CJ, et al. (2010) Human embryonic stem cells with biological and epigenetic characteristics similar to those of mouse ESCs. Proc Natl Acad Sci U S A 107: 9222-9227.
8. Hanna JH, Saha K, Jaenisch R (2010) Pluripotency and cellular reprogramming: facts, hypotheses, unresolved issues. Cell 143: 508-525.
9. Hikita ST, Kosik KS, Clegg DO, Bamdad C (2008) MUC1* mediates the growth of human pluripotent stem cells. PLoS One 3: e3312.
10. Huang L, Chen D, Liu D, Yin L, Kharbanda S, et al. (2005) MUC1 oncoprotein blocks glycogen synthase kinase 3beta-mediated phosphorylation and degradation of beta-catenin. Cancer Res 65: 10413-10422.
11. Huangfu D, Maehr R, Guo W, Eijkelenboom A, Snitow M, et al. (2008) Induction of pluripotent stem cells by defined factors is greatly improved by small-molecule compounds. Nat Biotechnol 26: 795-797.
12. Huangfu D, Osafune K, Maehr R, Guo W, Eijkelenboom A, et al. (2008) Induction of pluripotent stem cells from primary human fibroblasts with only Oct4 and Sox2. Nat Biotechnol 26: 1269-1275.
13. Ieda M, Fu JD, Delgado-Olguin P, Vedantham V, Hayashi Y, et al. (2010) Direct reprogramming of fibroblasts into functional cardiomyocytes by defined factors. Cell 142: 375-386.
14. Jaenisch R, Young R (2008) Stem cells, the molecular circuitry of pluripotency and nuclear reprogramming. Cell 132: 567-582.
15. Kaji K, Norrby K, Paca A, Mileikovsky M, Mohseni P, et al. (2009) Virus-free induction of pluripotency and subsequent excision of reprogramming factors. Nature 458: 771-775.
16. Kawamura T, Suzuki J, Wang YV, Menendez S, Morera LB, et al. (2009) Linking the p53 tumour suppressor pathway to somatic cell reprogramming. Nature 460: 1140-1144.
17. Kim YI, Park S, Jeoung DI, Lee H (2003) Point mutations affecting the oligomeric structure of Nm23-H1 abrogates its inhibitory activity on colonization and invasion of prostate cancer cells. Biochem Biophys Res Commun 307: 281-289.
18. Komarov PG, Komarova EA, Kondratov RV, Christov-Tselkov K, Coon JS, et al. (1999) A chemical inhibitor of p53 that protects mice from the side effects of cancer therapy. Science 285: 1733-1737.
19. Lin T, Chao C, Saito S, Mazur SJ, Murphy ME, et al. (2005) p53 induces differentiation of mouse embryonic stem cells by suppressing Nanog expression. Nat Cell Biol 7: 165-171.
20. Lowry WE, Richter L, Yachechko R, Pyle AD, Tchieu J, et al. (2008) Generation of human induced pluripotent stem cells from dermal fibroblasts. Proc Natl Acad Sci U S A 105: 2883-2888.
21. Lyssiotis CA, Foreman RK, Staerk J, Garcia M, Mathur D, et al. (2009) Reprogramming of murine fibroblasts to induced pluripotent stem cells with chemical complementation of Klf4. Proc Natl Acad Sci U S A 106: 8912-8917.
22. Mahanta S, Fessler SP, Park J, Bamdad C (2008) A minimal fragment of MUC1 mediates growth of cancer cells. PLoS One 3: e2054.
23. Maherali N, Sridharan R, Xie W, Utikal J, Eminli S, et al. (2007) Directly reprogrammed fibroblasts show global epigenetic remodeling and widespread tissue contribution. Cell Stem Cell 1: 55-70.
24. Maimets T, Neganova I, Armstrong L, Lako M (2008) Activation of p53 by nutlin leads to rapid differentiation of human embryonic stem cells. Oncogene 27: 5277-5287.
25. Nakagawa M, Koyanagi M, Tanabe K, Takahashi K, Ichisaka T, et al. (2008) Generation of induced pluripotent stem cells without Myc from mouse and human fibroblasts. Nat Biotechnol 26: 101-106.
26. Okita K, Ichisaka T, Yamanaka S (2007) Generation of germline-competent induced pluripotent stem cells. Nature 448: 313-317.
27. Okita K, Nakagawa M, Hyenjong H, Ichisaka T, Yamanaka S (2008) Generation of mouse induced pluripotent stem cells without viral vectors. Science 322: 949-953.
28. Park IH, Zhao R, West JA, Yabuuchi A, Huo H, et al. ( 2008) Reprogramming of human somatic cells to pluripotency with defined factors. Nature 451: 141-146.
29. Raina D, Ahmad R, Joshi MD, Yin L, Wu Z, et al. (2009) Direct targeting of the mucin 1 oncoprotein blocks survival and tumorigenicity of human breast carcinoma cells. Cancer Res 69: 5133-5141.
30. Smagghe BJ, Stewart AK, Carter MG, Shelton LM, Bernier KJ, et al. (2013) MUC1* ligand, NM23-H1, is a novel growth factor that maintains human stem cells in a more naive state. PLoS One 8: e58601.
31. Soldner F, Hockemeyer D, Beard C, Gao Q, Bell GW, et al. (2009) Parkinson's disease patient-derived induced pluripotent stem cells free of viral reprogramming factors. Cell 136: 964-977.
32. Sommer CA, Stadtfeld M, Murphy GJ, Hochedlinger K, Kotton DN, et al. (2009) Induced pluripotent stem cell generation using a single lentiviral stem cell cassette. Stem Cells 27: 543-549.
33. Stadtfeld M, Nagaya M, Utikal J, Weir G, Hochedlinger K (2008) Induced pluripotent stem cells generated without viral integration. Science 322: 945-949.
34. Strom E, Sathe S, Komarov PG, Chernova OB, Pavlovska I, et al. (2006) Small-molecule inhibitor of p53 binding to mitochondria protects mice from gamma radiation. Nat Chem Biol 2: 474-479.
35. Takahashi K, Tanabe K, Ohnuki M, Narita M, Ichisaka T, et al. (2007) Induction of pluripotent stem cells from adult human fibroblasts by defined factors. Cell 131: 861-872.
36. Takahashi K, Yamanaka S (2006) Induction of pluripotent stem cells from mouse embryonic and adult fibroblast cultures by defined factors. Cell 126: 663-676.
37. Thathiah A, Blobel CP, Carson DD (2003) Tumor necrosis factor-alpha converting enzyme/ADAM 17 mediates MUC1 shedding. J Biol Chem 278.
38. Vassilev LT, Vu BT, Graves B, Carvajal D, Podlaski F, et al. (2004) In vivo activation of the p53 pathway by small-molecule antagonists of MDM2. Science 303: 844-848.
39. Wei X, Xu H, Kufe D (2007) Human mucin 1 oncoprotein represses transcription of the p53 tumor suppressor gene. Cancer Res 67: 1853-1858.
40. Wernig M, Meissner A, Cassady JP, Jaenisch R (2008) c-Myc is dispensable for direct reprogramming of mouse fibroblasts. 2: 10-12.
41. Wernig M, Meissner A, Foreman R, Brambrink T, Ku M, et al. (2007) In vitro reprogramming of fibroblasts into a pluripotent ES-cell-like state. Nature 448: 318-324.
42. Woltjen K, Michael IP, Mohseni P, Desai R, Mileikovsky M, et al. (2009) piggyBac transposition reprograms fibroblasts to induced pluripotent stem cells. Nature 458: 766-770.
43. Yamanaka S (2007) Strategies and new developments in the generation of patient-specific pluripotent stem cells. Cell Stem Cell 1: 39-49.
44. Yu J, Hu K, Smuga-Otto K, Tian S, Stewart R, et al. (2009) Human induced pluripotent stem cells free of vector and transgene sequences. Science 324: 797-801.
45. Yu J, Vodyanik MA, Smuga-Otto K, Antosiewicz-Bourget J, Frane JL, et al. (2007) Induced pluripotent stem cell lines derived from human somatic cells. Science 318: 1917-1920.
46. Zhou H, Wu S, Joo JY, Zhu S, Han DW, et al. (2009 ) Generation of induced pluripotent stem cells using recombinant proteins. Cell Stem Cell 4: 381-384.
47. Zou X, Cong F, Coutts M, Cattoretti G, Goff SP, et al. (2000) p53 deficiency increases transformation by v-Abl and rescues the ability of a C-terminally truncated v-Abl mutant to induce pre-B lymphoma in vivo. Mol Cell Biol 20: 628-633.

## Claims

1. An *in vitro* or *ex vivo* method for generating less mature cells from starting cells in the absence of bFGF comprising inducing the starting cells to revert to a less mature state, comprising contacting the starting cells with a NME1 dimer or NME7 protein or gene encoding NME7 protein;
wherein the starting cells are chosen from the group comprising pluripotent stem cells in the primed state, multipotent stem cells and terminally differentiated cells, wherein the multipotent stem cell is a hematopoietic cell, a bone marrow cell or a neuronal cell;
wherein the less mature state is a pluripotent state; and wherein the less mature state is **characterized by** an increase in expression of:
(i) at least one of OCT4, SOX2, KLF4, KLF2, NANOG, LIN28, MUC1, NME1 and NME7; or
(ii) at least one of OCT4, SOX2, NANOG, KLF4, KLF2, Tra-1-60, Tra-1-81, SSEA4 and REX-1; or
(iii) at least one of MUC1/MUC1*, OCT4, SSEA4, Tra-1-60, REX-1, NANOG and KLF4:
as compared to the corresponding starting cells.

2. The method according to claim 1, further comprising:
(a) transfecting the starting cell with a nucleic acid that encodes Oct4, Sox2, Klf4, c-Myc, Lin28, or Nanog; or
(b) contacting the starting cells with Oct4, Sox2, Klf4, c-Myc, Lin28, or Nanog.

3. The method according to claim 1 or claim 2, wherein the terminally differentiated cell is a fibroblast, a dermablast, a blood cell, or a neuronal cell.

4. The method according to any one of claims 1 to 3, wherein the cells are mammalian.

5. The method according to any one of claims 1 to 4 wherein the cells are human.

6. The method according to any one of claims 1 to 5, wherein the pluripotent stem cell is an embryonic stem (ES) cell or induced pluripotent stem (iPS) cell.

7. The method according to any one of claims 1 to 6, wherein the generated cells are then differentiated *in vitro.*

## Patentansprüche

1. In-vitro- oder Ex-vivo-Verfahren zum Erzeugen von weniger reifen Zellen aus Ausgangszellen in Abwesenheit von bFGF, umfassend das Herbeiführen einer Rückkehr zu einem weniger reifen Zustand bei den Ausgangszellen, umfassend das Kontaktieren der Ausgangszellen mit einem NME1-Dimer oder NME7-Protein oder einem Gen, das für NME7-Protein kodiert;
wobei die Ausgangszellen ausgewählt sind aus der Gruppe umfassend pluripotente Stammzellen im geprimten Zustand, multipotente Stammzellen und terminal differenzierte Zellen, wobei die multipotente Stammzelle eine hämatopoetische Zelle, eine Knochenmarkszelle oder eine Nervenzelle ist;
wobei der weniger reife Zustand ein pluripotenter Zustand ist; und wobei der weniger reife Zustand **gekennzeichnet ist durch** einen Anstieg der Expression von:
(i) zumindest einem aus OCT4, SOX2, KLF4, KLF2, NANOG, LIN28, MUC1, NME1 und NME7; oder
(ii) zumindest einem aus OCT4, SOX2, NANOG, KLF4, KLF2, Tra-1-60, Tra-1-81, SSEA4 und REX-1; oder
(iii) zumindest einem aus MUC1/MUC1*, OCT4, SSEA4, Tra-1-60, REX-1, NANOG und KLF4;
im Vergleich zu den entsprechenden Ausgangszellen.

2. Verfahren nach Anspruch 1, ferner umfassend:
(a) Transfizieren der Ausgangszelle mit einer Nucleinsäure, die für Oct4, Sox2, Klf4, c-Myc, Lin28 oder Nanog kodiert; oder
(b) Kontaktieren der Ausgangszellen mit Oct4, Sox2, Klf4, c-Myc, Lin28 oder Nanog.

3. Verfahren nach Anspruch 1 oder 2, wobei die terminal differenzierte Zelle ein Fibroblast, ein Dermablast, eine Blutzelle oder eine Nervenzelle ist.

4. Verfahren nach einem der Ansprüche 1 bis 3, wobei die Zellen Säugetierzellen sind.

5. Verfahren nach einem der Ansprüche 1 bis 4, wobei die Zellen menschlich sind.

6. Verfahren nach einem der Ansprüche 1 bis 5, wobei die pluripotente Stammzelle eine embryonale Stammzelle (ES-Zelle) oder eine induzierte pluripotente Stammzelle (iPS-Zelle) ist.

7. Verfahren nach einem der Ansprüche 1 bis 6, wobei die erzeugten Zellen anschließend in vitro differenziert werden.

## Revendications

1. Procédé *in vitro* ou *ex vivo* pour générer des cellules moins matures à partir de cellules de départ en l'absence de bFGF, comprenant l'étape consistant à induire des cellules de départ à revenir à un état moins mature, comprenant l'étape consistant à mettre en contact des cellules de départ avec un dimère NME1 ou une protéine NME7 ou un gène codant pour la protéine NME7 ;
dans lequel les cellules de départ sont choisies dans le groupe comprenant des cellules souches pluripotentes à l'état amorcé, des cellules souches multipotentes et des cellules à différenciation terminale, dans lequel la cellule souche multipotente est une cellule hématopoïétique, une cellule de moelle osseuse ou une cellule neuronale ;
dans lequel l'état moins mature est un état pluripotent ; et
dans lequel l'état moins mature est **caractérisé par** une augmentation de l'expression de :
(i) au moins un parmi OCT4, SOX2, KLF4, KLF2, NANOG, LIN28, MUC1, NME1 et NME7 ; ou
(ii) au moins un parmi OCT4, SOX2, NANOG, KLF4, KLF2, Tra-1-60, Tra-1-81, SSEA4 et REX-1 ; ou
(iii) au moins un parmi MUC1/MUC1*, OCT4, SSEA4, Tra-1-60, REX-1, NANOG et KLF4 ;
par comparaison aux cellules de départ correspondantes.

2. Procédé selon la revendication 1, comprenant en outre les étapes consistant à :
(a) transfecter la cellule de départ avec un acide nucléique qui code pour Oct4, Sox2, Klf4, c-Myc, Lin28 ou Nanog ; ou
(b) mettre en contact les cellules de départ avec Oct4, Sox2, Klf4, c-Myc, Lin28 ou Nanog.

3. Procédé selon la revendication 1 ou la revendication 2, dans lequel la cellule à différenciation terminale est un fibroblaste, un dermablaste, une cellule sanguine ou une cellule neuronale.

4. Procédé selon l'une quelconque des revendications 1 à 3, dans lequel les cellules sont des cellules de mammifère.

5. Procédé selon l'une quelconque des revendications 1 à 4, dans lequel les cellules sont des cellules d'être humain.

6. Procédé selon l'une quelconque des revendications 1 à 5, dans lequel la cellule souche pluripotente est une cellule souche embryonnaire (ES) ou une cellule souche pluripotente induite (iPS).

7. Procédé selon l'une quelconque des revendications 1 à 6, dans lequel les cellules générées sont ensuite différenciées *in vitro.*
